# EUROPÄISCHE PATENTSCHRIFT

(11) **EP 0 373 335 B1**
(45) Veröffentlichungstag und Bekanntmachung des Hinweises auf die Patenterteilung: **10.12.1997**
(21) Anmeldenummer: 89120173.3
(22) Anmeldetag: 31.10.1989
(51) Int. Cl.: C07K 14/00, C12N 15/15, C12N 15/70, C12N 1/21, C12P 21/02

(54) **Neue Serinprotease-Inhibitor-Proteine, diese enthaltende Arzneimittel, DNA-Sequenzen die für diese Proteine codieren und Verfahren zur Herstellung dieser Proteine, Arzneimittel und DNA-Sequenzen**
Serine protease inhibitor proteins, medicaments containing them, DNA sequences coding for these proteins and methods for producing these proteins, medicaments and DNA sequences
Protéines inhibitrices des protéases du type sérine, médicaments les contenant, séquence d'ADN codant pour ces protéines et les procédés de production de ces protéines, médicament et séquences

(30) Priorität: 13.12.1988 DE 3841873
(43) Veröffentlichungstag der Anmeldung: 20.06.1990
(73) Patentinhaber: Grünenthal GmbH, D-52078 Aachen (DE)
(72) Erfinder: Heinzel-Wieland, Regina, Dr., D-5100 Aachen (DE); Ammann, Joachim, Dr., D-5100 Aachen (DE); Steffens, Gerd Josef, Prof. Dr., D-5100 Aachen (DE); Flohé, Leopold, Prof. Dr., D-5106 Roetgen (DE)

(56) Entgegenhaltungen:
- EP-A- 0 232 523
- WO-A-86/03497
- WO-A-89/06239
- EUROPEAN JOURNAL OF BIOCHEMISTRY, Band 160, Nr. 1, September 1986; R. HEINZEL et al., Seiten 61-67
- PROCEEDINGS OF THE NATL. ACADEMY OF SCIENCES USA, Band 83, September 1986, Washington, DC (US); R.C. THOMPSON et al., Seiten 6692-6696
- NATURE, Band 312, 01 November 1984; S. ROSENBERG et al., Seiten 77-80

## Beschreibung

Neue Serinprotease-Inhibitor-Proteine, diese enthaltende Arzneimittel, DNA-Sequenzen, die für diese Proteine codieren und Verfahren zur Herstellung dieser Proteine, Arzneimittel und DNA-Sequenzen.

Ein Gegenstand der vorliegenden Erfindung ist die Schaffung von neuen Serinprotease-Inhibitor-Proteinen, die insbesondere ein ausgeprägte Hemmwirkung gegenüber menschlicher Leukocytenelastase besitzen und im Gegensatz zu ähnlich wirkenden Naturprodukten gegen eine oxidative Inaktivierung resistent und daher für den therapeutischen Einsatz besser als die bekannten Produkte geeignet sind. Diese neuen Serinprotease-Inhibitor-Proteine sind mit Hilfe der recombinanten DNA-Technologie zugänglich. Weiterhin gehören zum Gegenstand der Erfindung daher diese Inhibitor-Proteine enthaltende Arzneimittel, DNA-Sequenzen, die für diese neuen Proteine codieren, und die Herstellung dieser Proteine, Arzneimittel und DNA-Sequenzen.

In lebenden Zellen und Organismen wird die Aktivität von Enzymen in erster Linie durch die Neusynthese und die chemische Modifikation von Enzymen reguliert. Dabei findet für eine schnelle Anpassung einer Zelle oder eines Organismus an eine veränderte Situation, die eine erhöhte Aktivität eines bestimmten Enzyms erfordert, jedoch nicht in jedem Fall eine erhöhte Neusynthese dieses Enzyms statt, häufig wird vielmehr ein latenter Enzymvorrat aktiviert. Als Aktivierungsmechanismen latenter Enzyme sind die Spaltung durch spezifische Peptidasen, die Phosphorylierung durch Proteinkinasen, die Ausschüttung aus Vesikeln und die Änderung der Proteinkonformation durch allosterische Liganden bekannt.

Ein Überschießen der genannten Aktivierungsreaktionen und die Langzeitwirkung der aktivierten Enzyme kann durch den gezielten Abbau oder die spezifische Hemmung dieser Enzyme vermieden werden. Beispielsweise wird die biologische Aktivität aktivierter Proteasen häufig durch spezifische Protease-Inhibitoren blockiert.

In den letzten Jahren wurde die klinische und pathogenetische Relevanz verschiedener Protease-Inhibitoren erkannt. So spielen endogene Serinproteasen eine bedeutende physiologische Rolle zum Beispiel beim Proteinabbau im Verdauungstrakt, beim intrazellulären Proteinab- und -umbau, bei der Aufrechterhaltung des hämostatischen Gleichgewichtes, bei der Infektabwehr und der Gewebeneubildung nach Verletzungen. In den meisten Fällen wird die Aktivität der Serinproteasen permanent oder vorübergehend durch verschiedene endogene Inhibitoren unterdrückt, wodurch der proteolytische Angriff auf biologisches Gewebe auf spezielle Situationen beschränkt bleibt, in denen proteolytische Vorgänge definierte biologische Funktionen erfüllen. In pathologischen Situationen kann jedoch dieses Gleichgewicht zwischen Proteasen und ihren Inhibitoren erheblich gestört werden. Unphysiologisch hohe Serinproteaseaktivitäten können dann die ursprüngliche Gewebsschädigung verstärken. Als Beispiele für derartige Situationen seien erwähnt: Systemische Komplement-Aktivierung infolge von extrakorporaler Zirkulation, von Antigen-Antikörperreaktionen oder von Septikämie; intravasale Gerinnung oder extensive Fibrinolyse im septischen Schock; Abbau von Bindegewebe bei der Tumorinvasion, bei Infektion, Entzündung und Schock; proteolytischer Abbau körpereigener Proteine durch Serinproteasen des Pankreas, die während einer Pankreasentzündung aus ihrer natürlichen Umgebung austreten.

Klinische Krankheitsbilder, die mit einem gestörten Gleichgewicht zwischen Serinproteasen und Serinprotease-Inhibitoren einhergehen, umfassen dementsprechend Gerinnungsstörungen, invasives Wachstum von Tumoren, Emphysem, Arthritis, Nierenentzündung (z. B. Glomerulonephritis) und insbesondere fulminant verlaufende Entzündungen wie beim polytraumatischen oder septischen Schock, die jeweils mit oder ohne Atemnotsyndrom verlaufen können, sowie bei der Pankreatitis.

Aus der Vielzahl von Serinproteasen hat in letzter Zeit die aus menschlichen polymorphkernigen Leukocyten freigesetzte Elastase Interesse im Hinblick auf therapeutische Interventionen gefunden. Diese wird von weißen Blutzellen ausgeschüttet, wenn diese z. B. durch opsonierte Fremdkörper oder lösliche Stimuli wie zum Beispiel formylierte bakterielle Peptide oder Anaphylaktoxine aktiviert werden. (Diese menschliche Leukocytenelastase wird nachstehend als "HLE" bezeichnet.) Als wesentlichste physiologische Rolle der HLE wird die Verdauung phagocytierter Mikroorganismen oder proteinhaltiger Zelltrümmer angesehen, jedoch baut sie auch Elastin und Kollagen ab, also wesentliche Bestandteile des elastischen Bindegewebes bzw. der gesamten extracellulären Bindegewebssubstanz.

Unter physiologischen Bedingungen kann aktivierte HLE kaum je gemessen werden, da sie durch α₂-Makroglobulin, α₁-Protease-Inhibitor (α₁PI oder α₁-Antitrypsin) oder Anti-Leukoprotease (ALP oder auch zum Beispiel als "HUSI-I" bezeichnet) gehemmt wird. Dabei scheint α₂-Makroglobulin von untergeordneter Bedeutung bei der Regulierung der HLE-Aktivität zu sein, da seine Assoziationsgeschwindigkeits-Konstante (kₐₛₛ) zu gering ist. Im Gegensatz dazu hemmen α₁PI und ALP wirksam die freigesetzte HLE, jedoch werden sie gerade unter den Bedingungen der Leukocytenaktivierung leicht durch gleichzeitig freigesetzte Sauerstoffradikale oxidativ inaktiviert .

Die Aktivierung von Leukocyten, die insbesondere bei Entzündungskrankheiten im Vordergrund steht, führt nämlich gleichermaßen zur Freisetzung von Elastase und Superoxidradikalen. Superoxidradikale dismutieren zu molekularem Sauerstoff und Wasserstoffperoxid. Aus dem Gemisch von Wasserstoffperoxid und Superoxidradikalen entstehen dann in Gegenwart von (allgegenwärtigen) Spuren von Übergangsmetallionen Hydroxylradikale, welche die Serinprotease-Inhibitoren α₁PI und ALP schnell inaktivieren. Diese Inaktivierung kann ferner durch Wasserstoffperoxid in Gegenwart von Halogenidionen und des Leukocytenenzyms Myeloperoxidase hervorgerufen werden (Flohé et al. in: Handbook of Inflammation, Vol. 5, Seiten 255 - 281, Elsevier 1985). Die Inaktivierung der Protease-Inhibitoren scheint eine Voraussetzung für die Gewebszerstörungen zu sein, die bei chronischen und insbesondere bei akuten Entzündungen beobachtet werden.

Im Falle des α₁PI wird die Inaktivierung auf die Oxidation eines speziellen Methioninrestes zurückgeführt, der auf der Proteinschleife exponiert ist, die an die Aktivitätstasche der HLE bindet. Durch Ersatz dieses Methionins gegen Valin mit Hilfe der rekombinanten DNA-Technologie wurde ein aktives α₁PI-Analogon erhalten, das gegen oxidative Inaktivierung resistent war (Rosenberg et al., Nature 312, 77 - 80, 1984). Andererseits ist bekannt, daß der Austausch von Methionin in Position 358 des α₁PI gegen Arginen zur Änderung der Funktion des Proteins führt, welches dann kann Elastase-Inhibitor mehr ist, sondern als Thrombin-Inhibitor wirkt (Owen et al., New Eng. J. Med. 309, 694 - 698, 1983).

Für die Therapie von Krankheiten, die auf einem gestörten Gleichgewicht zwischen Serinproteasen und Serinprotease-Inhibitoren beruhen, wurden z. B. in der DE 36 00 571 A1 Proteine mit der dort angegebenen Aminosäuresequenz vorgeschlagen, bei denen es sich um Inhibitoren vom Typ HUSI-I (Human Seminalplasma Inhibitor), also vom Typ ALP, handelt. HUSI-I ist ein säurestabiler Proteinaseinhibitor, der Proteasen aus den lysosomalen Granula der Granulozyten inhibiert, beispielsweise also auch die Elastase. Gegen andere intra- und extrazelluläre Proteasen zeigt HUSI-I nur eine abgeschwächte Hemmaktivität. Sein Molekulargewicht beträgt etwa 11.000.

Die in der DE 36 00 571 A1 erhaltenen Inhibitor-Moleküle wurden einerseits durch die Hemmung des Enzyms Chymotrypsin hinsichtlich ihrer biologischen Aktivität, andererseits immunologisch als HUSI-Typ-I-Inhibitoren charakterisiert.

Unter der Bezeichnung "Proteine mit der biologischen Aktivität der HUSI-Typ-I-Inhibitoren" werden in der DE 36 00 571 A1 Proteine und Fusionsproteine, die die biologische Aktivität der HUSI-Typ-I-Inhibitoren aufweisen, also beispielsweise über immunologische Eigenschaften des natürlichen Proteins verfügen und/oder die spezifischen inhibitorischen Eigenschaften des natürlichen Proteins haben, verstanden. Auch Proteine und Fusionsproteine, die nur Teilbereiche der Aminosequenzen von HUSI-I umfassen bzw. enthalten, werden in der DE 36 00 571 A1 als "Proteine mit der biologischen Aktivität der HUSI-Typ-I-Inhibitoren" bezeichnet. Solche Teilbereiche der Aminosäuresequenz von Proteinen werden auch "Domänen" genannt.

ALP (bzw. HUSI-I) ist ein seit langem bekanntes und untersuchtes Protein (vgl. z. B. Schiessler et al. in "Neutral proteases of human polymorphonuclear leukocytes" - Havemann et al. eds. - Urban & Schwarzenberg, Baltimore, Munich 1978, Seiten 195 - 207), das 107 Aminosäurereste enthält und aufgrund von Homologieüberlegungen aus zwei Inhibitordomänen besteht. Der ersten (N-terminalen) Domäne wird die Hemmung Trypsin-ähnlicher Enzyme zugeschrieben, während die zweite (C-terminale) Domäne nach Röntgen-Kristallographischen Untersuchungen an Chymotrypsin bindet, und es wird vermutet, daß diese Domäne auch die die Elastase hemmende Wirkung beinhaltet (Grütter et al., Embo J. 7, 345 bis 351, 1988).

Es ist bereits vorgeschlagen, aber noch nicht realisiert worden (vgl. WO 86/03497, insbesondere Seiten 11 - 13), in der Aminosäurekette dieses bekannten 107 Aminosäuren enthaltenden Serinprotease-Inhibitorproteins einen oder mehrere Aminosäurereste gegen andere auszutauschen. So soll der Austausch von Arginin (Position 20) gegen Lysin, von Leucin (Position 72 oder 74) gegen Lysin oder Arginin und/oder von Methionin (Position 73) gegen Lysin oder Arginin die Hemmwirkung gegenüber Trypsin-ähnlichen Serinproteasen erhöhen. Andererseits wurde dort zur Aktivitätsteigerung gegen Chymotrypsin-ähnliche Serinproteasen, einschließlich Cathepsin G, der Austausch von einer oder mehreren der Aminosäuren in Position 20 (Arginin), Positionen 72 oder 74 (Leucin) und/oder Position 73 (Methionin) gegen Phenylalanin, Tyrosin oder Trypthophan in Betracht gezogen. Schließlich wurde noch vorgeschlagen, die Inhibitorwirkung gegenüber Pankreas-Elastase-ähnlichen Serinproteasen dadurch zu verbessern, daß man eine oder mehrere der Aminosäuren in Position 20 (Arginin), in Positionen 72 oder 74 (Leucin) und/oder in Position 73 (Methionin) gegen Alanin austauscht.

Andererseits wurde in der WO 86/03497 aber auch darauf hingewiesen, daß z. B. durch Austausch von Arginin in Position 20 gegen Glycin die Hemmwirkung gegenüber Trypsin, durch den Austausch von Methionin in Position 73 bzw. den von Leucin in Position 72 und/oder 74 gegen Glycin die Hemmwirkung gegenüber Leukocytenelastase aufgehoben wird.

In der WO 86/03497 wird ferner der sich den Befunden von Rosenberg et al. (loc. cit.) anschließende Vorschlag gemacht, zur Verbesserung der Stabilität des Protease-Inhibitors gegen oxidative Inaktivierung, aber auch zur Steigerung der Hemmwirkung gegen Leukocyten-Elastase und Cathepsin G Methionin gegen Valin auszutauschen (wobei allerdings offen blieb, ob alle oder welche der vier Methionin-Gruppen in dem Protein zur Erreichung dieser Effekte auszutauschen sind oder ob es sich z. B. um eine zusätzliche Abwandlung in Ergänzung zum bereits oben genannten Austausch von Methionin in Position 73 durch Phenylalanin, Tyrosin oder Tryptophan handeln soll).

Für alle diese vorstehend referierten Abwandlungsvorschläge des Proteinase-Inhibitors gemäß der WO 86/03497 sowie für die dazu genannten Wirkungs- bzw. Eigenschaftsänderungen des Proteins fehlen zwar experimentelle oder sonstige konkrete Belege, man kann aber daraus entnehmen, daß dem Fachmann bekannt war, daß eine Änderung der Aminosäuren in den Positionen 20 und/ oder 72 - 74, insbesondere 73, voraussichtlich zum Verlust oder zur Änderung der Inhibitorwirkung gegenüber bestimmten Serinproteasen führt bzw. führen kann und daß völlig unvorhersehbar ist, welche Wirkungsänderung aus der Einführung anderer als der genannten Aminosäuren z. B. in Positionen 72 - 74, insbesondere 73, resultieren mag. Diese Ungewißheit wird noch unterstützt durch die weiter oben referierten Befunde von Owen et al. hinsichtlich der Wirkungsänderung des α₁-Antitrypsins durch Austausch eines Methionin-Restes gegen einen Arginin-Rest. Dabei ist noch zu beachten, daß die oxidative Inaktivierung von ALP nach Befunden von Kramps et al., Biol. Chem. Hoppe-Seyler 369, Suppl., 83 - 88, 1988, zum Verlust sowohl der tryptischen als auch der elastolytischen Hemmwirkung führt, also - da in der 1. Domäne kein Methionin enthalten ist - nicht (bzw. nicht nur) auf der Methionin-Oxidation beruhen kann. Es war daher nicht vorhersehbar, ob ein (völliger oder teilweiser) Methioninaustausch in der 2. Domäne der ALP zu aktiven, oxidationsresistenten ALP-Mutanten führen würde.

Wie schon ausgeführt, ist bekannt, daß ALP (bzw. Protease-Inhibitoren vom HUSI-I-Typ usw.) insbesondere auch (auf bisher nicht bekanntem Wege) durch Oxidation schnell inaktiviert werden. Im Hinblick auf den therapeutischen Einsatz derartiger Leukocyten-Elastase-Inhibitoren ist es daher in hohem Maße erwünscht, über oxidationsbeständigere Serinprotease-Inhibitoren verfügen zu können.

Ein Gegenstand der vorliegenden Erfindung ist daher die Schaffung neuer Mutanten des Serinprotease-Inhibitors ALP, für die weitestgehend die Strucktur des authentischen menschlichen Inhibitors beibehalten wurde und die insbesondere die Hemmwirkungen gegenüber menschlicher Leukocyten-Elastase (HLE) sowie weitere günstige Eigenschaften aufweisen, die jedoch gegen oxidative Inaktivierung durch aktivierte Leukocyten unempfindlicher als natürliche ALP oder gar völlig resistent sind.

Diese neuen erfindungsgemäßen Serinprotease-Inhibitor-Proteine enthalten Aminosäuresequenzen der folgenden Formel I oder solche Teilbereiche davon, welche zumindest die Sequenz der Positionen 53 - 101 umfassen:

18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu-X1-Asp-Gly-Gln-Cys-Lys-Arg-Asp-Leu--
107
Lys-Cys-Cys-X2-Gly-X3-Cys-Gly-Lys-Ser-Cys-Val-Ser-Pro-Val-Lys-Ala

Darin steht W für Tyrosin oder Glutaminsäure, X₁, X₂ und X₃ sind gleich oder verschieden und jeder dieser Reste bedeutet Methionin oder Leucin, wobei X₃ außerdem auch Phenylalanin bedeuten kann, Y steht für Prolin oder Arginin und Z bedeutet Leucin oder, wenn W für Tyrosin und X₁, X₂ und X₃ für Leucin stehen, auch Isoleucin.

Vorzugsweise steht in dieser Formel I W für Tyrosin.

Eine bevorzugte Gruppe dieser neuen Serinprotease-Inhibitor-Proteine der Formel (I) entspricht der Formel (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn--Y--Pro-Asn-Phe-Cys-Glu-X1-Asp-Gly-Gln-Cys-Lys-Arg-Asp-Leu--
107
Lys-Cys-Cys-X2-Gly-X[?]-Cys-Gly-Lys-Ser-Cys-Val-Ser-Pro-Val-Lys-Ala
 oder Teilbereichen davon, welche zumindest die Sequenz der Positionen 53 - 101 umfassen, worin X₁, X₂, Y und Z die gleiche Bedeutung wie oben haben und X₃' für Methionin oder Leucin steht.

Ganz generell, d. h. unabhängig von der Bedeutung von W und Y sind besonders bevorzugt diejenigen Produkte der Formeln I oder Ia bzw. deren Teilbereiche, in denen X₁, X₂, X₃ und Z für Leucin stehen.

Weiterhin werden diejenigen Produkte der Formel I oder Ia bzw. deren Teilbereiche, in denen Y für Prolin steht, bevorzugt.

Die Aminosäuresequenz der erfinderungsgemäßen neuen Serinprotease-Inhibitoren umfaßt bevorzugt die Aminosäurepositionen 1 bis 107 und die Teilsequenzen 48 - 107, 49 - 107 sowie 50 - 107 aus dem Formelbild I bzw. Ia. Zum Gegenstand der Erfindung gehören aber auch Fusionsproteine dieser neuen Serinprotease-Inhibitoren, insbesondere auch solche mit N-terminalen Signalsequenzen oder z. B. β-Galactosidase. In den Proteinen der Formeln I bzw. Ia können im N-terminalen Teilbereich bis zu Asparaginsäure (Position 49) auch weitere Aminosäuren ausgetauscht sein, beispielsweise in der aus der WO 86/03497 für den Austausch von Arginin in Position 20 bekannten Weise.

Die erfindungsgemäßen neuen Serinprotease-Inhibitoren zeichnen sich, insbesondere wenn Y für Prolin und Z für Leucin stehen, durch eine ausgeprägte Inhibitorwirkung gegen Leukocyten-Elastase, aber auch z. B. gegen Chymotrypsin sowie - in geringerem Maße - gegen Cathepsin G, Chymase und Trypsin bei gesteigerter Oxidationsstabilität aus.

Bedeuten in den erfindungsgemäßen Serinprotease-Inhibitoren X₁, X₂ und X₃ Leucin und steht entweder Y für Arginin oder Z für Isoleucin, oder sind X₁, X₂ und Z Leucin, während X₃ Phenylalanin darstellt, so ändert sich die Substratspezifität des betreffenden Produktes, da dann zwar nach wie vor die Leukocyten-Elastase ausgezeichnet gehemmt wird, die Inhibitorwirkung gegenüber Chymotrypsin und z. B. Cathepsin G jedoch nicht mehr gegeben ist. Diese Produkte, insbesondere das mit X₁ - X₃ = Leucin und Z = Isoleucin sowie das mit X₁, X₂ und Z = Leucin und X₃ = Phenylalanin, stellen also selektive Inhibitoren für die Leukocyten-Elastase dar (insbesondere wenn nur eine Domäne mit einem der Sequenzbereiche umfassend die Positionen 53 - 101 bis Positionen 48 - 107 vorliegt oder z. B. im Protein der Formel I bzw. Ia durch Austausch von Arginin in Position 20 gegen Glycin die Hemmwirkung gegenüber Trypsin aufgehoben wurde), die außerdem besonders oxidationsresistent sind. Bevorzugte erfindungsgemäße Produkte sind dementsprechend auch diejenigen Serinprotease-Inhibitoren der Formel I bzw. Ia (oder Teilbereiche dieser Sequenzen), in denen X₁ und X₂ für Leucin stehen, während X₃ Leucin und Z Isoleucin oder X₃ Phenylalanin und Z Leucin bedeuten. Von dieser Verbindungsgruppe werden diejenigen bevorzugt, in denen W für Tyrosin und Y für Prolin steht.

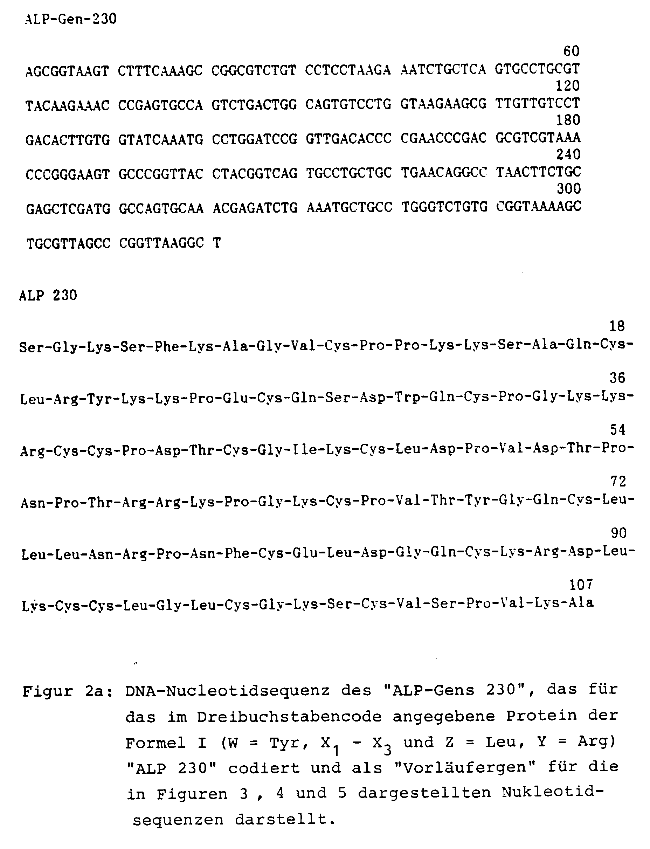

Ein weiterer Gegenstand der Erfindung ist die Schaffung von DNA-Sequenzen, die für ein die Aminosäuresequenzen der Formel I oder Ia oder zumindest deren Teilbereich der Positionen 53 - 101 enthaltendes Serinprotease-Inhibitor-Protein codieren. In einer bevorzugten Ausführungsform codiert die erfindungsgemäße DNA-Sequenz für ein 107 Aminosäuren enthaltendes Protein der Formeln I bzw. Ia. Besonders bevorzugte erfindungsgemäße Ausführungsformen sind die DNA-Sequenzen, die für die Proteine mit den in Figuren 2a, 2b und 3 bis 8 dargestellten Aminosäuresequenzen codieren.

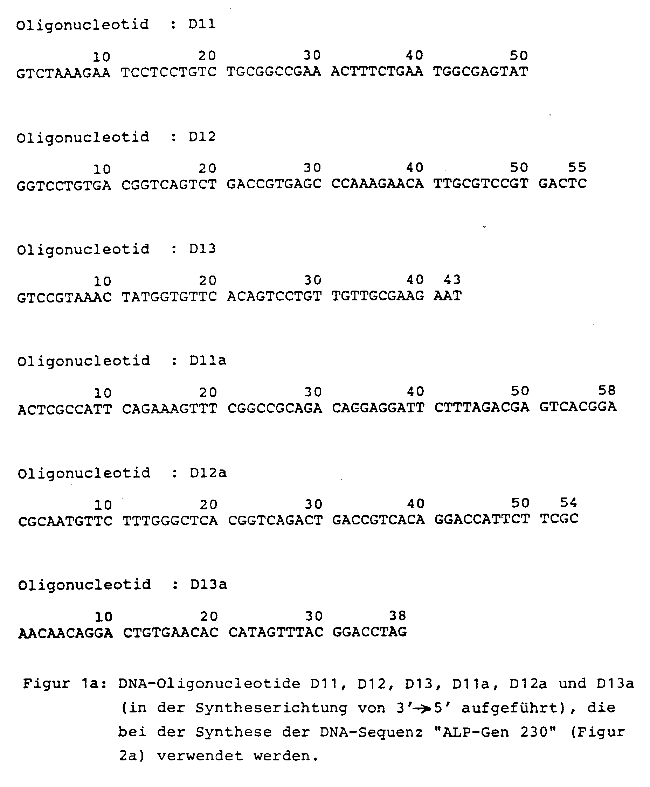

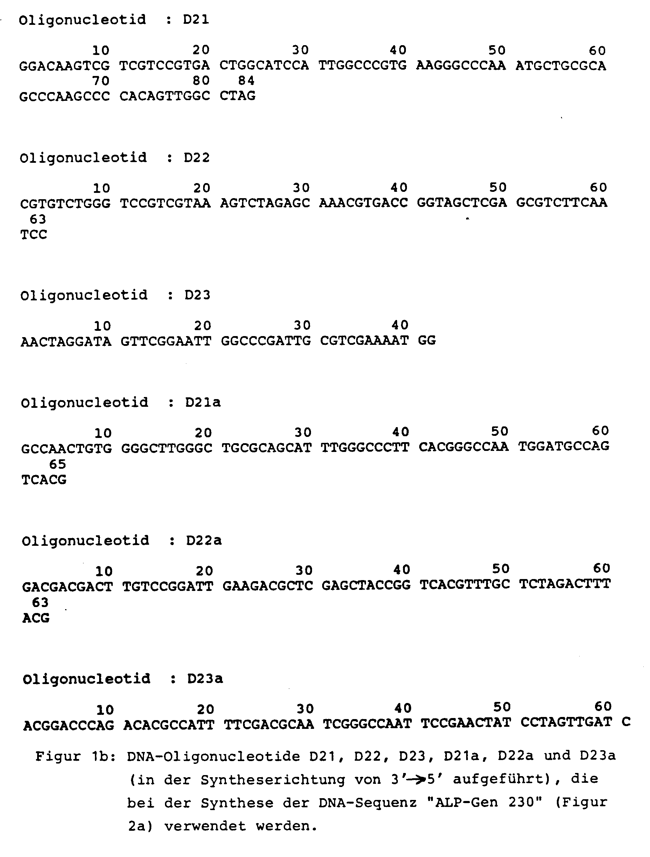

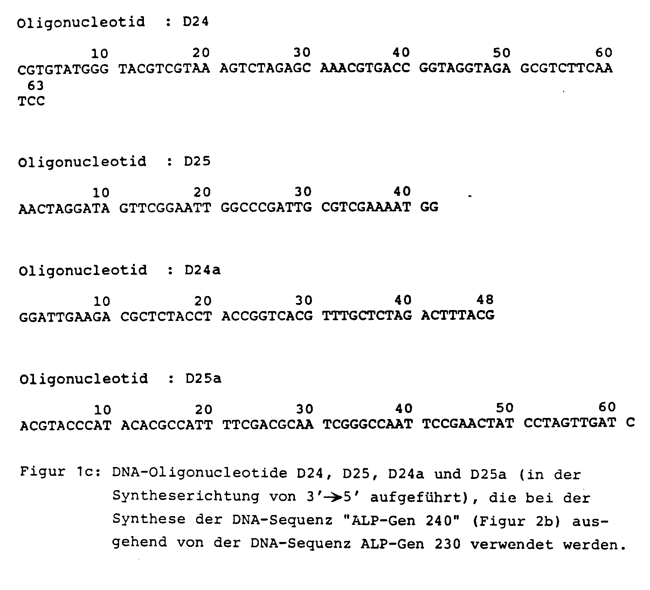

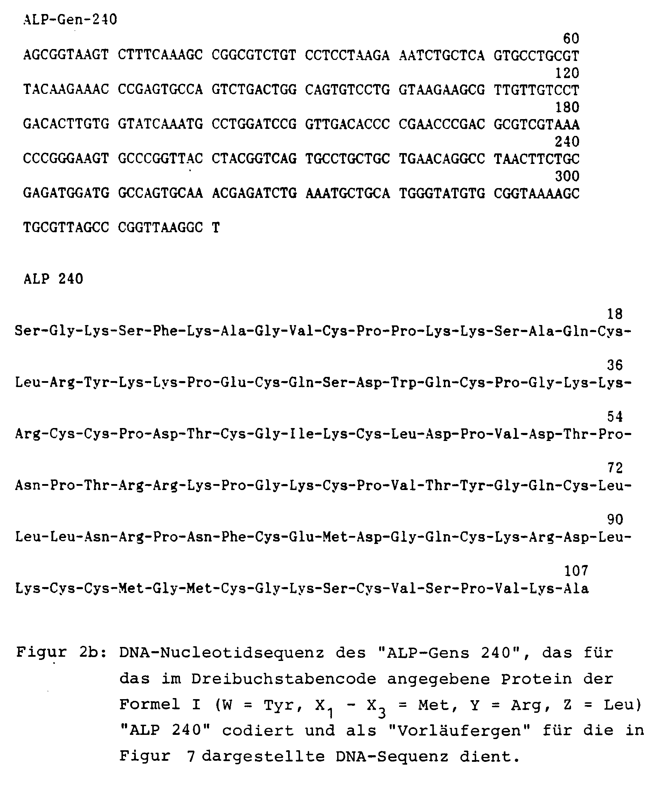

Unter Berücksichtigung der Codonnutzung durch den für die Expression einzusetzenden Wirt, vorzugsweise E.coli, und der Zweckmäßigkeit der Einführung von singulär vorkommenden Restriktionsschnittstellen werden diese DNA-Sequenzen auf der Basis der angestrebten Aminosäuresequenzen gemäß Formel I bzw. Ia anhand des genetischen Codes entworfen und synthetisiert. Für diese Synthese stellt man zweckmäßig zunächst mit im Synthesizer erhaltenen DNA-Oligonucleotiden (siehe z. B. Figur 1) durch Klonierung in einem geeigneten Vektor, vorzugsweise ptacSDT (DSM 5018), Gene her, die bereits als solche für bestimmte Proteine der Formel I codieren können, die aber auch als Vorläufergene für weitere DNA-Sequenzen dienen können, die für andere Proteine der Formel I codieren. Beispiele für solche Gene, die direkt für Proteine der Formel I codieren, aber auch als Vorläufergene eingesetzt werden können, sind die in Figuren 2a und 2b dargestellen Gene ALP 230 und ALP 240. Zur Konstruktion von für andere Proteine der Formel I codierenden DNA-Sequenzen aus einem ein Vorläufergen enthaltendem Plasmid wird dieses durch Restriktionsendonucleasen, vorzugsweise Bst EII und Stu I, gespalten und dann dephosphoryliert. Durch das Klonieren von für die Aminosäuren der Positionen 66 - 76 in der Sequenz gemäß Formel I codierenden Oligonucleotiden zwischen diesen beiden Spaltstellen werden die für Verbindungen der Formel I unterschiedlicher Aminosäuresequenz codierenden Gene erhalten. Die dabei (paarweise, also M1 und M2, M3 und M4 bzw. M5 und M6) eingesetzten Oligonucleotide entsprechen den nachstehenden Formeln:
3′ - GCC CAA GTC GTC GTC CGT GAC TGG CAT CCA TTG - 5′ (M1)
3′ - G ATG CCA GTC ACG GAC GAC GAC TTG GGC - 5′ (M2)
3′ - GCC CAA GTC GTC CTA CGT GAC TGG CAT CCA TTG - 5′ (M3)
3′ - G ATG CCA GTC ACG TAG GAC GAC TTG GGC - 5′ (M4)
3′ - GCC CAA GTC GTC GTC CGT GAC TGG AAG CCA TTG - 5′ (M5)
und
3′ - GCTT CCA GTC ACG GAC GAC GAC TTG GGC - 5′ (M6)

Soll in einer Verbindung der Formel I jedoch X₃ für Phenylalanin stehen, so wird von einem mit Hilfe der Oligonucleotide M1 - M6 konstruierten Plasmid ausgegangen, in das dann solche Oligonucleotide kloniert werden, die an Position 96 des Proteins der Formel I die Expression von Phenylalanin bewirken. Solche Oligonucleotide sind z. B., wenn im Protein der Formel I X₁ und X₂ für Leucin und X₃ für Phenylalanin stehen, wobei W, Y und Z die gleiche Bedeutung wie oben haben, die Oligonucleotide der folgenden Formeln:
3′ - A GAA CGC AAT CGG GCC AAT TCC GAA CTA TCC TAG TTG ATC - 5′ (E1)
3′ - TC GAG CTA CCG GTC ACG TTT GCT CTA GAC TTT ACG ACG GAC CCA AAG ACG CCA TTT - 5′ (E2)
und
3′ - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5′ (E3)

Zum Einbau dieser Oligonucleotide (nach deren Phosphorylierung und Hybridisierung) in das jeweilige Ausgangsplasmid wird letzteres vorzugsweise mit den Restriktionsendonucleasen Sac I und Spe I gespalten, in üblicher Weise dephosphoryliert und dann das so erhaltene Fragment mit den hybridisierten Oligonucleotiden E1 - E3 ligiert.

Die so erhaltenen Plasmide werden vorzugsweise anschließend so umkonstruiert, daß die Expressionsvektoren die entsprechenden Gene zweifach (getrennt durch 2 Stoppcodons, eine 14 - 17, vorzugsweise 16 Nucleotide enthaltende Sequenz, eine Shine-Dalgarno-Sequenz sowie eine weitere Sequenz mit 6 - 9, vorzugsweise 7 Nucleotiden) hinter dem tac-Promoter und gefolgt von einer Terminatorsequenz tragen. Eine von Jorgensen et al., J. Bact. 138, 705 - 714 (1979) beschriebene Terminatorsequenz (der tetA/orfL-Terminator) ist besonders geeignet.

Ein weiterer Gegenstand der Erfindung ist ein Verfahren zur Herstellung der neuen Serinprotease-Inhibitoren, die die Aminosäuresequenzen der Formel I oder zumindest deren Teilbereich der Positionen 53 - 101 enthalten, durch Transformation geeigneter Wirtsotganismen mit den wie (oben bzw. nachstehend und in den weiter unter folgenden Beispielen) beschrieben erhaltenen Vektoren und der Expression mit anschließender Isolierung, Refolding-Stufe und Reinigung des Proteins sowie gegebenenfalls anschließender Variation der Kettenlänge durch partielle Hydrolyse zur Abspaltung von Sequenzteilen, die N-terminal vor Position 48, insbesondere vor Position 50 oder C-terminal hinter Position 101 liegen.

Geeignete Wirtsorganismen sind Stämme der Art E.coli, Bacillus subtilis, Staphylococcus carnosus oder anderer gram-positiver oder gram-negativer Bakterien, Saccharomyces cerevisiae, Streptomyceten, andere mikroskopisch kleine Pilze wie Aspergillus oryzae, Aspergillus niger usw. sowie tierische oder menschliche Zellen. Bevorzugte Wirtsorganismen sind Stämme der Art E.coli, insbesondere der Untergruppe K12, wie z. B. E.coli K12 JM 101 (ATCC 33876), E.coli K12 JM 103 (ATCC 39403), E.coli K12 JM 105 (DSM 4162) oder E.coli K12 DH1 (ATCC 33849).

Geeignete Vektoren für die Transformation der genannten Wirtsorganismen und die Expression der hierin beschriebenen Proteine sind Plasmide wie z. B. pBR 322 und davon abgeleitete Plasmide, oder auch Virusgenome wie beispielsweise das Genom des Lambda-Phagen oder das des Phagen M13. Handelsübliche geeignete Expressionsvektoren sind z. B. pUC 9, pUC 12, pUC 13, pUC 18, pUC 19, pPL-Lambda, pKK 233-2, pKK 223-3, pYEJ 001, pTZ 18R, pTZ 19R, pUB 110, pWH 418, pDH 32, pJDB 207, pAN 7-1, p35R₂, pSVL, pKSV und andere.

Die Expressionseinleitung in E.coli-Expressionssystemen, die den tac-Promoter enthalten, kann z. B. durch Glukoseentzug oder vorzugsweise durch Zugabe von Isopropyl-β-D-thiogalactopyranosid bewirkt werden. Das entstandene Protein wird isoliert, chromatographisch vorgereinigt und dann einem Refolding-Verfahren in Gegenwart z. B. von Harnstoff oder Guanidin-Hydrochlorid sowie in Gegenwart eines Thiole und Disulfide enthaltenden Puffergemisches unterworfen. Schließlich wird das gewünschte Produkt chromatographisch gereinigt.

Aufgrund des Vorhandenseins einer säurelabilen Asparaginsäure-Prolin-Bindung in den Positionen 49 - 50 der Proteine gemäß Formel I bzw. Ia oder ihrer wenigstens den Sequenzteil 49 - 101 enthaltenden Teilbereiche bzw. N-terminalen Fusionsproteine dieser Produkte ist es möglich, alle vor Position 50 liegenden Sequenzteile der Proteins bzw. Fusionsproteins abzuspalten und so die C-terminalen Domänen der Produkte der Formel I bzw. Ia umfassend die Sequenzbereiche 50 - 101 bis 50 - 107 herzustellen. Dazu wird das Ausgangsprotein bzw. Fusionsprotein (mit Sequenzen vor Position 49) beispielsweise für 24 - 36 Stunden bei Raumtemperatur mit 70 %iger Ameisensäure oder vergleichbar sauren Reagenzien behandelt und dann die intakte C-terminale Domäne durch Chromatographie isoliert und anschließend gereinigt.

Sollen in dem angestrebten Produkt der Formel I oder Ia bzw. deren Teilbereichen die Gruppen X₁ und X₂ für Leucin und X₃ für Leucin oder Phenylalanin stehen, so kann man in an sich bekannter Weise auch so vorgehen, daß man an die für das Produkt codierende DNA-Sequenz 5′-terminal das für Methionin codierende Codon ATG anfügt. Daraus resultiert bei der Expression eines Fusionsproteins die zusätzliche Aminosäure Met⁻¹ vor dem N-Terminus des gewünschten Proteins, das dann in üblicher Weise z. B. durch Brom-Cyan-Spaltung zugänglich ist.

Andererseits können vom C-terminalen Ende der erhaltenen Proteine alle oder nur eine bis fünf der Aminosäuren von Positionen 107 - 102 (beginnend bei Alanin in Position 107) enzymatisch z. B. durch Einwirkung von Carboxypeptidasen, abgespalten werden.

Die so erhaltenen Inhibitoren unterschiedlicher Kettenlänge können z. B. mit Hilfe eines ELISA-Testes quantitativ bestimmt werden, wobei ein polyklonaler Antikörper gegen authentische menschliche ALP verwendet werden kann.

Die im folgenden benutzten Abkürzungen haben die Bedeutungen:
- ALP: (natürliche) Antileukoprotease
- bp: Basenpaare
- Chloramin T: Natriumsalz des N-Chlor-p-toluolsulfonamids
- DTT: Dithiothreitol
- EDTA: Ethylendiamintetraessigsäure
- HEPES: 2-[4-(2-Hydroxyäthyl)-1-piperazino]-ethansulfonsäure
- HLE: menschliche Leukocytenelastase
- IPTG: Isopropyl-β-D-thiogalactopyranosid
- PMA: Phorbolmyristylacetat
- SD-Sequenz: Shine-Dalgarno-Sequenz
- Tris: Trishydroxymethylaminomethan
- Triton^{(R)}-X-100: Octylphenoxydecaethoxyethanol (auch Octoxynol-10)

Die Hemmwirkung von erfindungsgemäßen Proteinen im Vergleich zur Hemmwirkung von natürlicher ALP gegenüber humaner Leukocyten-Elastase (HLE) wird wie folgt ermittelt:

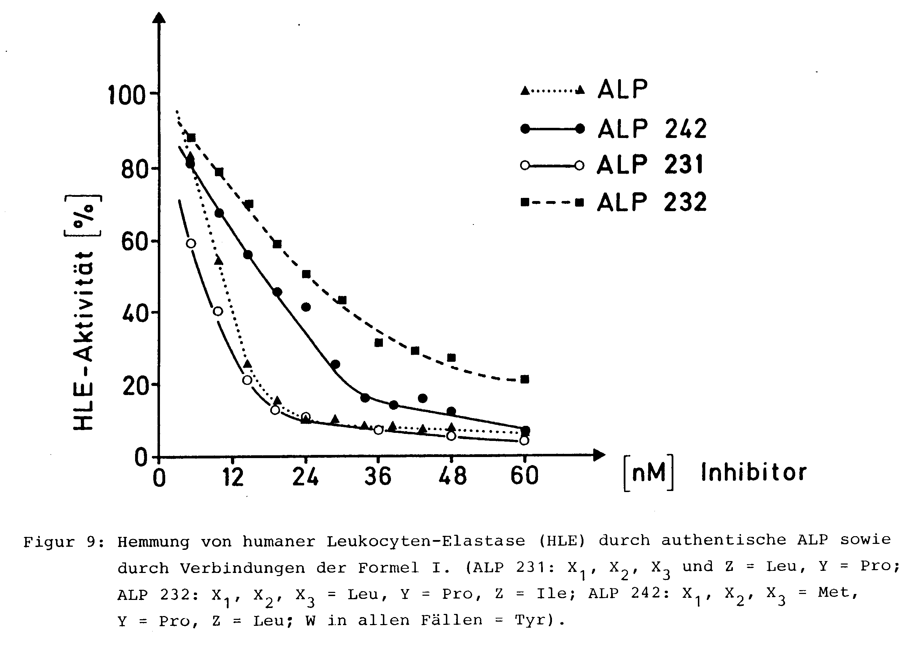

Die Aktivitätsbestimmung von HLE wird bei 25° C mit dem chromogenen Substrat Methoxysuccinyl-L-Alanyl-L-Alanyl-L-Prolyl-L-Valyl-p-Nitroanilid (= "HLE-Substrat", Protogen) durchgeführt (Geiger et al., J. Clin. Chem. Clin. Biochem. 23, 821, 1985). Die Testbedingungen zur Bestimmung der HLE-Aktivität sind: 0,1 M HEPES, pH 7,5, 30 nM HLE (Stammlösung: 0,83 µM, 2,5 mM HCl) und 0,3 mM HLE-Substrat. Die Reaktion wird durch Zugabe von HLE-Substrat zu dem 0,1 M HEPES und 30 nM HLE enthaltenden Reaktionsansatz gestartet und nach 10minütiger Inkubation bei 25° C durch Einstellen auf 0,6 M Essigsäure abgestoppt. Ein Referenzansatz wird ohne HLE parallel inkubiert. Die Änderung der Absorption/10 min. wird bei 405 nm bestimmt. Zur Bestimmung der Hemmaktivität von Verbindungen der Formel I bzw. von ALP werden steigende Mengen der Inhibitoren eingesetzt, mit konstanter HLE-Konzentration (30 nM) 10 min. bei 25° C vorinkubiert und die Reaktion, wie oben beschrieben, gestartet, abgestoppt und gemessen. Die Hemmung von HLE in Gegenwart von ALP bzw. unterschiedlicher Verbindungen der Formel I wird als % HLE-Aktivität versus die Inhibitorkonzentration aufgetragen (Fig. 9).

Weiterhin wird auch die Hemmaktivität von erfindungsgemäßen Proteinen sowie - als Kontrolle - natürlicher ALP gegenüber Chymotrypsin aus Rinderpankreas wie folgt gemessen:

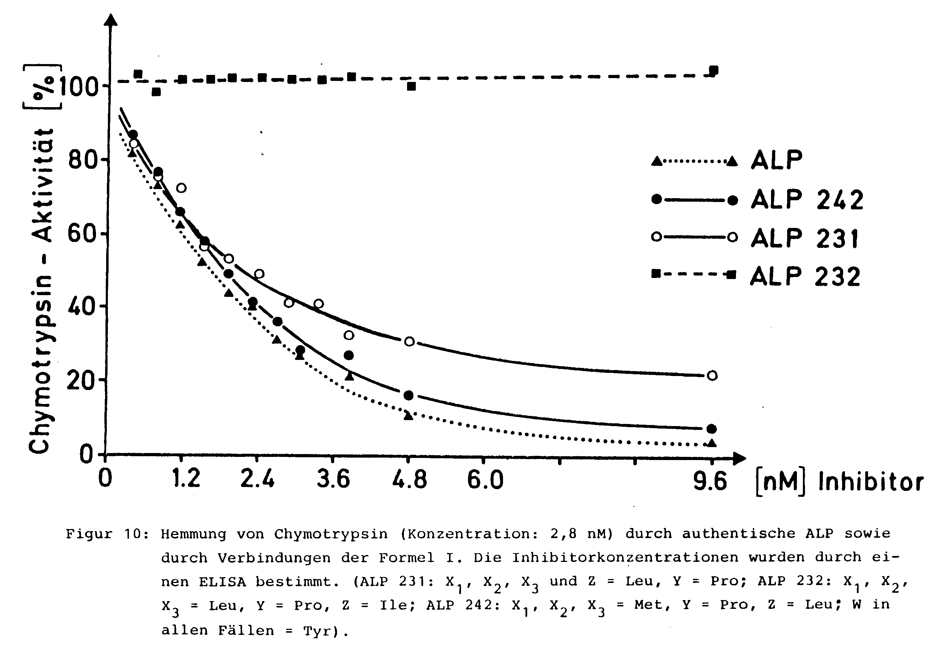

Die Aktivitätsbestimmung von Chymotrypsin (Boehringer Mannheim) wird mit dem chromogenen Substrat Succinyl-L-Alanyl-L-Alanyl-L-Prolyl-L-Phenylalanyl-p-Nitroanilid (= "Chymotrypsin-Substrat", Protogen) durchgeführt (Geiger in: Methods of Enzymatic Analysis, Vol. 5, Seiten 99 - 109, Verlag Chemie, 1984). Die Testbedingungen sind 0,1 M Tris-HCl, pH 7,8, 20 mM CaCl₂, 0,8 mM Triton-X-100, 2,8 nM Chymotrypsin (Stammlösung: 100 nM Chymotrypsin, 2,5 mM HCl, 0,8 mM Triton-X-100) und 0,15 mM Chymotrypsin-Substrat. Die Reaktion wird durch Einstellen eines Reaktionsansatzes, der 2,8 nM Chymotrypsin in dem genannten Puffer enthält, auf 0,15 mM Chymotrypsin-Substrat gestartet und nach 15minütiger Inkubation bei 25° C durch Einstellen auf 0,6 M Essigsäure gestoppt. Parallel dazu wird ein Referenzansatz ohne Chymotrypsin inkubiert. Die Änderung der Absorption/15 min. wird bei 405 nm gemessen. In entsprechend aufgebauten Testansätzen werden steigende Konzentrationen der zu testenden Inhibitoren (ALP und Verbindungen der Formel I) bei der Chymotrypsin-Konzentration von 2,8 nM 10 min. bei 25° C vorinkubiert und die Messung, wie oben beschrieben, durchgeführt. Die Hemmung von Chymotrypsin wird als % Chymotrypsin-Aktivität versus die Inhibitorkonzentration aufgetragen (Fig. 10).

Die Aktivitätsbestimmung von Cathepsin G (Calbiochem GmbH) wird ebenfalls mit dem "Chymotrypsin-Substrat" (Protogen) durchgeführt. Dafür sind die Testbedingungen: 0,1 M Tris-HCl, pH 8,0, 0,2 M NaCl, 10 mM MgCl₂, 0,8 mM Triton-X-100, 100 nM Cathepsin G (Stammlösung: 4 µM Cathepsin G, 50 mM Natriumacetat, pH 4,0, 0,7 M NaCl, 16 mM Triton-X-100) und 0,4 mM Chymotrypsin-Substrat. Reaktionsansätze, die steigende Mengen des jeweils zu testenden Inhibitors und 100 nM Cathepsin G enthalten, werden 20 Minuten bei 25° C inkubiert, dann zum Starten der Reaktion mit Chymotrypsin-Substrat versetzt und nach 20minütiger Inkubation bei 25° C zum Abstoppen der Reaktion auf 0,6 M Essigsäure eingestellt. Die Änderung der Absorption/20 min. wird bei 405 nm bestimmt. Ein Referenzansatz ohne Cathepsin G, ein weiterer ohne Inhibitor werden parallel dazu inkubiert.

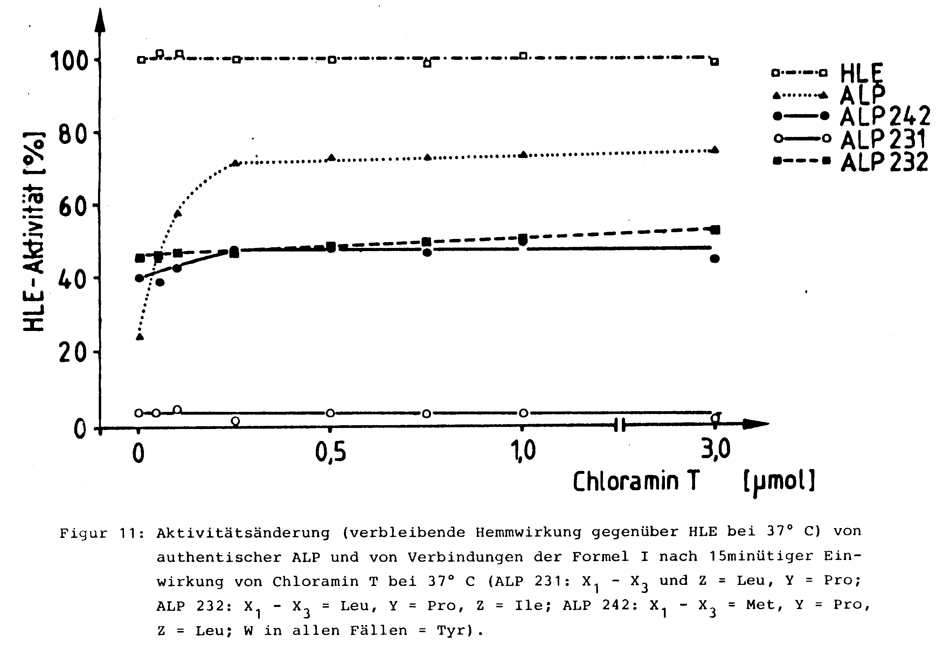

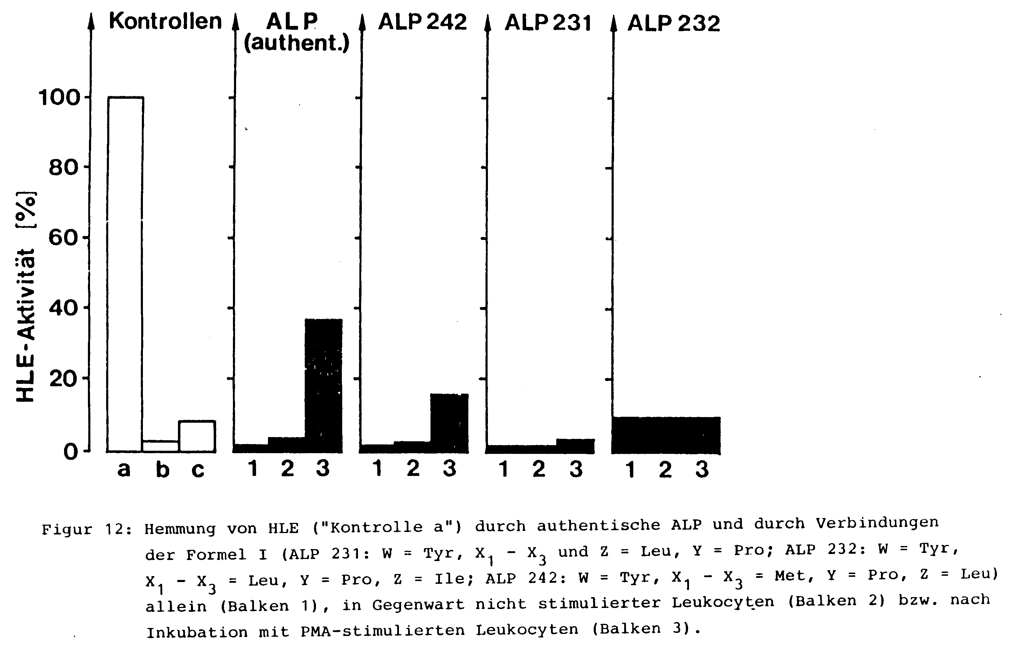

Weiterhin wird die Resistenz von erfindungsgemäßen Proteinen im Vergleich zu der von ALP gegen eine oxidative Inaktivierung überprüft, und zwar einerseits durch Einwirkung von Chloramin T, andererseits durch Inkubation mit aktivierten Leukocyten. Die Versuchsdurchführungen sind wie folgt:
a) Einwirkung von Chloramin T
   Konstante Mengen der zu testenden Inhibitoren werden mit steigenden Konzentrationen an Chloramin T als chemischem Oxidans inkubiert, bevor die HLE-inhibierende Kapazität bestimmt wird. Die Behandlung der Inhibitoren mit Chloramin T und die HLE-Messung erfolgt bei 37° C in 0,7 ml Puffer der Zusammensetzung 50 mM Tris-HCl, pH 8,5, 100 mM NaCl, 0,8 mM Triton-X-100. 0,1 µg (8,5 pmol) ALP bzw. Verbindung der Formel I werden mit 0,05 bis 3 µmol Chloramin T 15 min inkubiert. Zur Bestimmung der HLE-Hemmaktivität werden 0,2 µg (8,3 pmol) HLE zugegeben, 5 min inkubiert und danach die Reaktion mit "HLE-Substrat" (Protogen; siehe oben) gestartet. Nach 5 min wird die Reaktion durch Einstellen auf 0,6 M Essigsäure gestoppt. Die Änderung der Absorption wird bei 405 nm gemessen. Als Kontrollen dienen Reaktionsansätze, die a) ohne Inhibitor, HLE und Chloramin T (Referenz), b) ohne Inhibitor und Chloramin T (100 % HLE-Aktivität) und c) ohne Chloramin T (Inhibitoraktivität ohne oxidative Beeinflussung) parallel inkubiert werden. In einem weiteren Kontrollexperiment konnte gezeigt werden, daß 0,05 bis 3 µmol Chloramin T keinen Einfluß auf die HLE-Aktivität haben. Die Auswirkungen der Behandlung von ALP und Verbindungen der Formel I mit Chloramin T sind in Fig. 11 dargestellt.
b) Inkubation mit aktivierten Leukocyten
   Bei dieser Versuchsdurchführung, welche die natürlichen Verhältnisse besser wiederspiegelt, werden frisch isolierte Leukocyten in vitro in Gegenwart von ALP bzw. Inhibitoren der Formel I durch Phorbolmyristylacetat (PMA) zu einer Superoxidradikalbildung angeregt. Die Ausgangsaktivität und die Hemmaktivität nach Inaktivierung werden im HLE-Test bestimmt. Je Testansatz werden 0,5 x 10⁶ isolierte humane Leukocyten mit konstanter Menge an Inhibitoren (0,3 µg) in "HBSS-"Puffer (8 g/l NaCl, 6 g/l HEPES, 0,4 g/l KCl, 0,35 g/l NaHCO₃, 0,06 g/l Na₂HPO₄, 0,06 g/l KH₂PO₄, 1 g/l Glukose, pH 7,4) 10 min bei 37° C inkubiert. Die Zellen werden durch Zugabe von PMA (30 ng/ml) stimuliert, 30 min bei 37° C inkubiert und durch Zentrifugation (2500 g für 5 min. bei 4° C) sedimentiert. Der zellfreie Überstand wird zu Inhibitionsmessungen in den HLE-Test mit 0,4 µg (16,6 pmol) HLE eingesetzt (vgl. oben). In Parallelexperimenten werden gleiche Inhibitormengen mit nicht stimulierten Leukocyten bzw. ohne Leukocyten getestet. Weitere Kontrollen werden ohne Inhibitoren durchgeführt (s. Fig. 12).

Bei diesen und weiteren Testen zeigte sich, daß die natürliche ALP, wie erwartet, HLE, Chymotrypsin, Cathepsin G und Trypsin hemmt, jedoch weitgehend inaktiviert wurde, wenn sie der Einwirkung von Chloramin T oder von aktivierten Leukocyten ausgesetzt wurde. Überraschenderweise führte z. B. der Austausch des Methioninrestes in Position 73 der natürlichen ALP gegen Leucin, also die Bildung des Proteins der Formel I, worin W Tyrosin ist, X₁, X₂ und X₃ Methionin sind, Y für Prolin steht und Z Leucin bedeutet ("ALP 242") nur zu einer unbedeutenden Änderung der Hemmwirkung gegenüber HLE (Figur 9) und zu keiner Änderung der Hemmwirkung gegenüber Chymotrypsin (Figur 10). Wurde diese Verbindung der Formel I (ALP 242) der Einwirkung von Choramin T oder aktivierten Leukocyten ausgesetzt, so wurde sie nennenswert weniger inaktiviert als authentische ALP (Figuren 11 und 12). Dies belegt eine ausgeprägtere Biostabilität des Produktes in solchen Situationen, in denen es therapeutisch zum Einsatz gelangen kann.

Die Testergebnisse einiger der erfindungsgemäßen Serinprotease-Inhibitoren sind in Figuren 9 - 12 ausführlich wiedergegeben. In der folgenden Tabelle werden einige dieser und weiterer Ergebnisse für einige 107 Aminosäuren enthaltende Proteins der Formel I zusammengestellt:

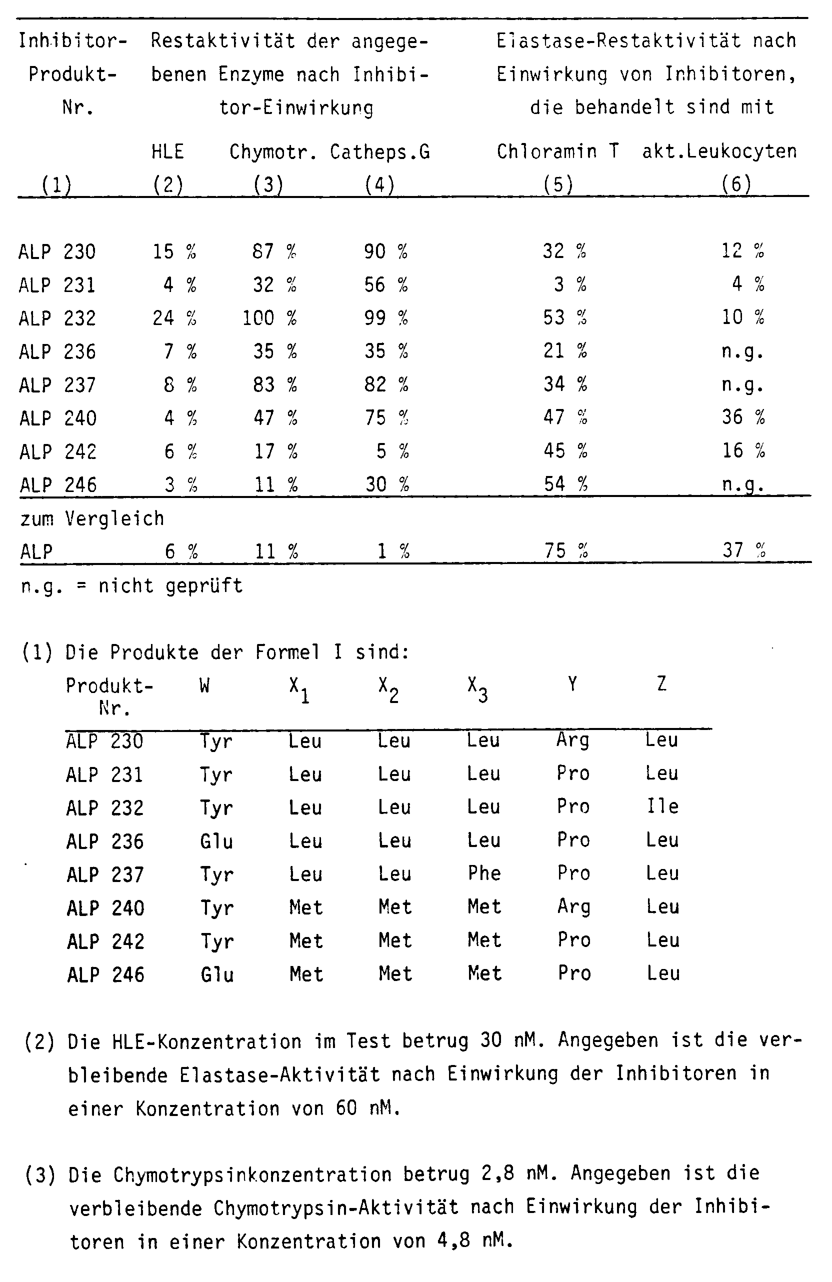

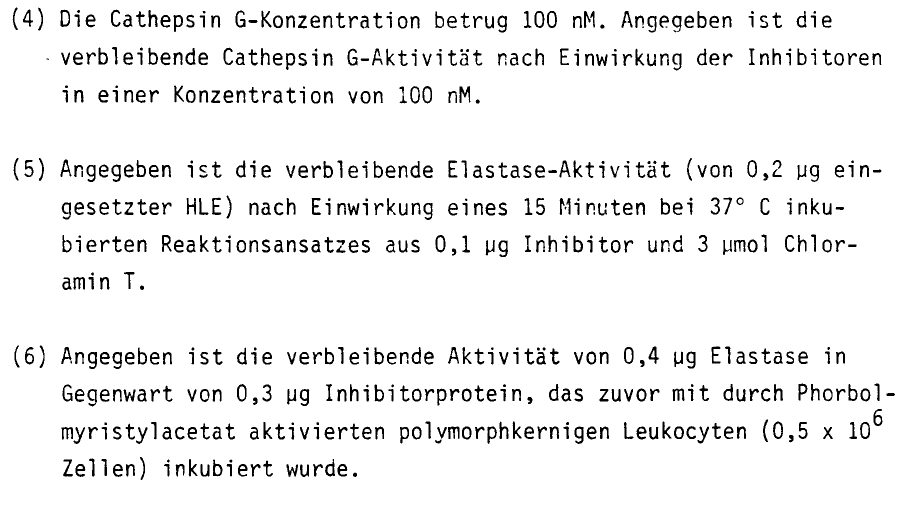

Die Tabelle zeigt die gute Hemmwirkung der neuen Serinprotease-Inhibitoren gegenüber menschlicher Leukocyten-Elastase, verbunden mit guter Hemmwirkung gegenüber Chymotrypsin, sofern Z für Leucin steht und X₃ von Phenylalanin verschieden ist. Aus der Tabelle ist auch ersichtlich, daß, wenn zumindest einer der Reste X₁, X₂, X₃ oder Z für Leucin steht, sich die Verbindungen der Formel I durch eine ausgeprägte Stabilität gegenüber oxidativer Inaktivierung, sei es durch chemische Einflüsse (Chloramin T), sei es durch Enzymeinwirkung auszeichnen.

Besonders überraschend ist, daß die Verbindungen, in denen X₁, X₂, X₃ und Z für Leucin stehen, insbesondere aber die Verbindung ALP 231 (W = Tyr; Y = Pro), in gleicher Weise hochaktiv gegen HLE und Chymotrypsin sind wie ALP und daß diese Verbindungen durch eine 30minütige Inkubation mit 0,5 x 10⁶ Leukocyten, die mit dem stärksten Stimulator für Sauerstoffradikalbildung, dem PMA, behandelt wurden, in keiner Weise oder allenfalls in unbeachtlichem Maße inaktiviert werden. Diese Kombination von hoher Hemmaktivität und Widerstandsfähigkeit gegenüber einer Inaktivierung durch Oxidantien macht insbesondere das Produkt ALP 231 zu einer wertvollen Verbindung zur Therapie u. a. der oben genannten Krankheiten.

Wie sich aus der Tabelle weiter ergibt, zeigt auch die Verbindung ALP 232, die sich von ALP 231 nur darin unterscheidet, daß hier Z einen Isoleucin-Rest darstellt, wertvolle Eigenschaften. Wie ALP 231 besitzt die Verbindung ALP 232 eine ausgeprägte Resistenz gegenüber einer oxidativen Inaktivierung durch aktivierte Leukocyten, jedoch stellt sie einen Inhibitor mit geänderter Substratspezifität dar. Sie hemmt nach wie vor HLE, aber sie ist unwirksam gegenüber Chymotrypsin und Cathepsin G. Auch wenn in der ALP 231 lediglich X₃ von Leucin zu Phenylalanin verändert wird, erhält man einen selektiven hoch wirksamen HLE-Inhibitor, die ALP 237, mit verbesserter Oxidationsstabilität. Da die Elastase als diejenige Protease betrachtet wird, die überwiegend für die Gewebezerstörung unter pathologischen Bedingungen verantwortlich ist, stellen solche selektivieren Inhibitoren, zumal unter Berücksichtigung ihrer ausgeprägten Biostabilität, einen weiteren therapeutischen Fortschritt dar.

Die erfindungsgemäßen neuen Serinprotease-Inhibitoren stellen daher wertvolle Wirkstoffe für prophylaktische und therapeutische Anwendungen dar. Beispielsweise eignen sie sich zur Therapie chronischer und akuter entzündlicher Prozesse sowie zur Verhinderung der Ausbildung von Emphysemen und Lungenödemen, aber auch zur Vorbeugung gegen eine Tumorinvasion. Weiterhin eignen sie sich zur Therapie und Prophylaxe von Schockzuständen und z. B. zur Behandlung von postoperativen Blutungen aufgrund von Hyperfibrinolyse. Auch gewebedegenerative Erkrankungen wie rheumatoide Arthritis oder Osteoarthritis können mit den Verbindungen der Formel I bzw. Ia oder deren als Serinprotease-Inhibitoren wirksamen Teilbereichen therapiert werden.

Die zur Therapie der genannten Erkrankungen einzusetzenden Dosen der erfindungsgemäßen Serinprotease-Inhibitorproteine richten sich - abgesehen von der Applikationsart bzw. -form - insbesondere nach dem bei Therapiebeginn bestehenden Krankheitsstadium und sind vom Arzt individuell zu ermitteln. Als Anhaltspunkt kann davon ausgegangen werden, daß der in Betracht zu ziehende Dosierungsbereich für die parenterale Applikation bei Mengen von 100 mg bis 5 g pro Einzeldosis, vorzugsweise 100 mg bis 3 g des Serinprotease-Inhibitorproteins liegt. Dies steht auch im Einklang mit z. B. von Egbring et al., Blood 49 (1977) 219 - 231 erhobenen Befunden, aus denen sich ableiten läßt, daß beim erwachsenen Sepsis-Patienten im Laufe eines Tages eine Menge von wenigstens 1g Leukocytenelastase freigesetzt wird, zur Therapie also durch das in etwa 6- bis 10-fachem molaren Überschuß zu verabreichende erfindungsgemäße Protein inhibiert werden muß.

Dementsprechend sind Arzneimittel mit einem wirksamen Gehalt an einem die Aminosäuresequenz der Formeln I bzw. Ia oder Teilbereichen davon (zumindest die Sequenz der Positionen 53 - 101) enthaltendem Protein und üblichen Trägerstoffen und/oder Verdünnungsmitteln und/oder Hilfsstoffen ebenfalls Gegenstand der vorliegenden Erfindung. Zur Therapie können die erfindungsgemäßen Proteine in Form steriler isotonischer Lösungen durch intramuskuläre, intravenöse oder subkutane Injektionen in das Entzündungsgebiet oder gegebenenfalls durch Infusion verabfolgt werden. Es kommen aber auch Zubereitungen der neuen Proteine in Spray-oder Inhalationsform in Betracht, die zur Behandlung von Erkrankungen der Atemwege durch direktes Aufbringen der Wirkstoffe auf die betroffenen Teile der Bronchien und Lunge besonders geeignet sind.

Die Figuren zeigen:
- Figur 1a - c:: Sequenzen von 16 Oligonucleotiden, die bei der Synthese der DNA-Sequenzen "ALP-Gen 230" und "ALP-Gen 240" (vgl. Figur 2a und 2b) verwendet wurden.

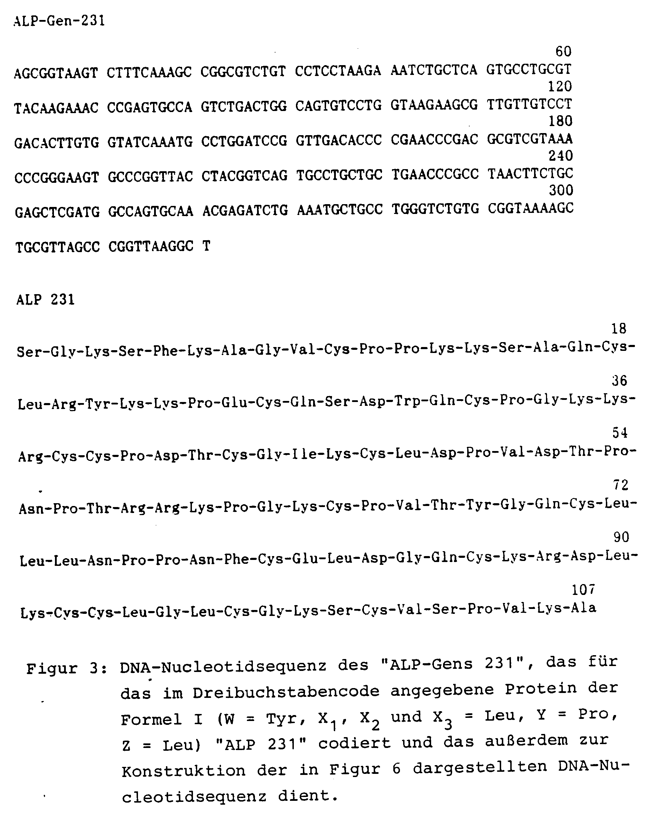

- Figur 2a:: Die DNA-Nucleotidsequenz des "ALP-Gens 230", das für das mit der Aminosäuresequenz dargestellte Protein "ALP 230", d. h. die Verbindung der Formel I, in der X₁, X₂, X₃ und Z Leucin, W Tyrosin und Y Arginin sind, codiert und das als "Vorläufergen" für die in Figuren 3, 4 und 5 dargestellten DNA-Nucleotidsequenzen dient.

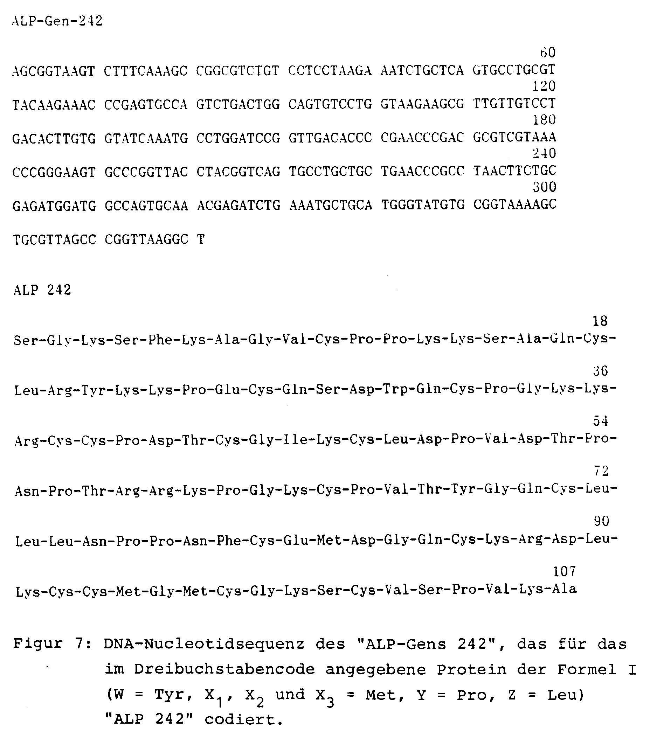

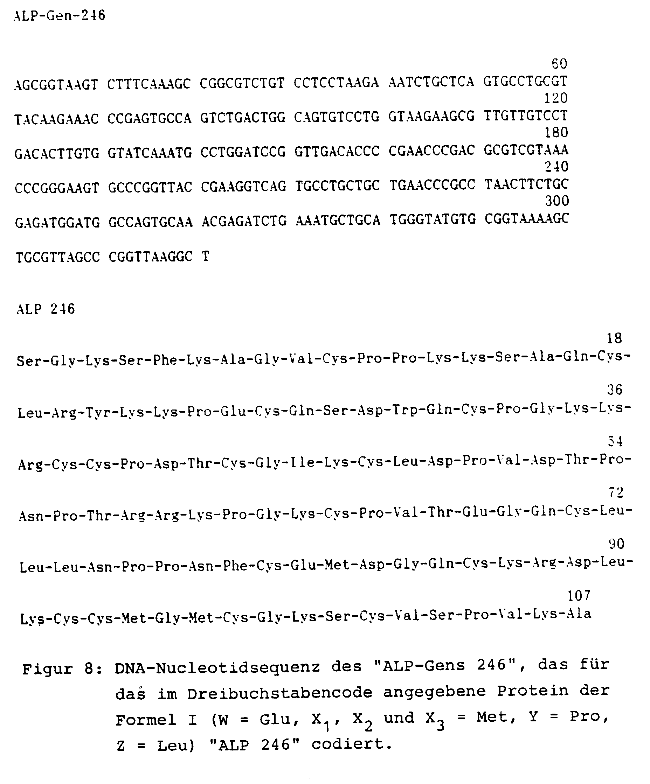

- Figur 2b:: Die DNA-Nucleotidsequenz des "ALP-Gens 240", das für das mit der Aminosäuresequenz dargestellte Protein "ALP 240", d. h. die Verbindung der Formel I, in der W Tyrosin, X₁, X₂ und X₃ Methionin, Y Arginin und Z Leucin bedeuten, codiert und das als "Vorläufer-Gen" für die in Figuren 7 und 8 dargestellten DNA-Nucleotidsequenzen dient.

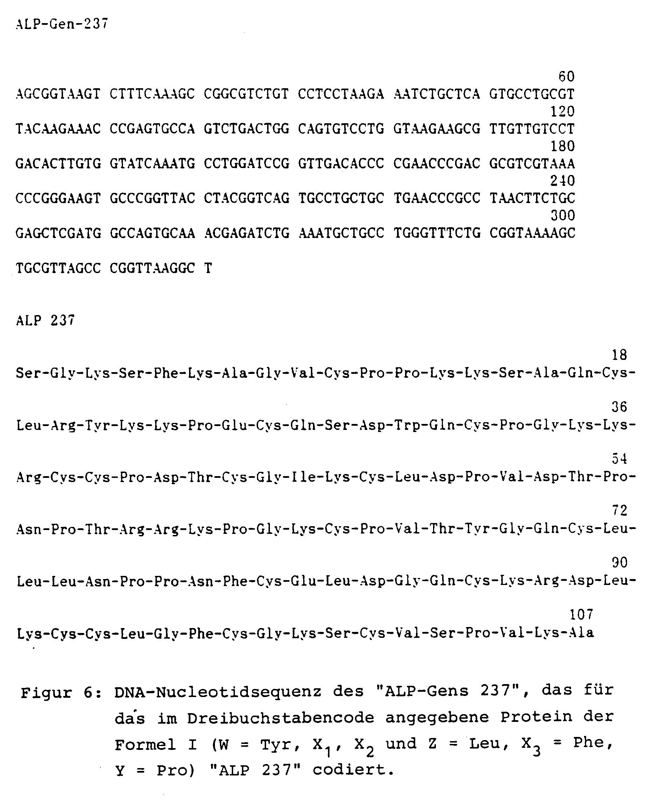

- Figur 3:: Die DNA-Nucleotidsequenz des ALP-Gens 231, das für das mit der Aminosäuresequenz dargestellte Protein "ALP 231", d. h. die Verbindung der Formel I, in der X₁, X₂, X₃ und Z Leucin, W Tyrosin und Y Prolin bedeuten, codiert und das außerdem zur Konstruktion der in Figur 6 dargestellten DNA-Nucleotidsequenz dient.

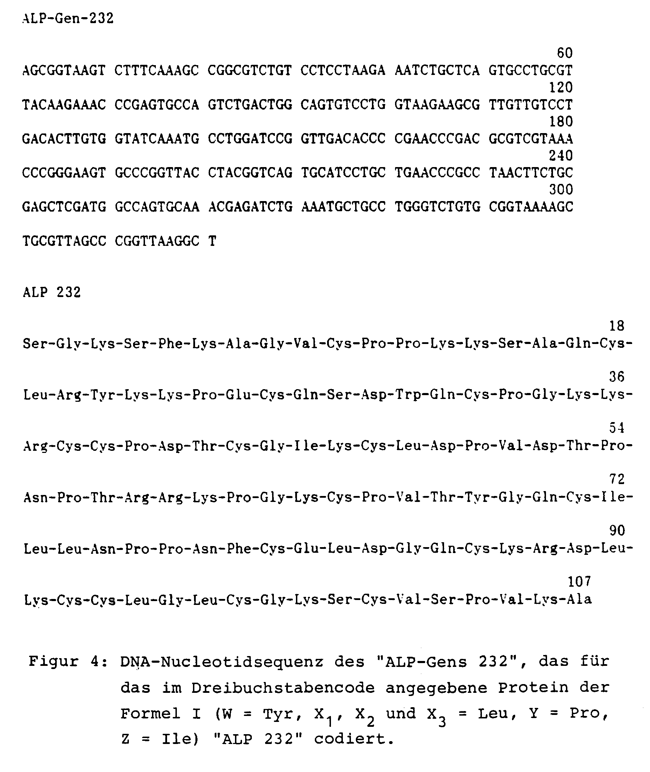

- Figur 4:: Die DNA-Nucleotidsequenz des ALP-Gens 232, das für das mit der Aminosäuresequenz dargestellte Protein "ALP 232", d. h. die Verbindung der Formel I, in der W Tyrosin, X₁, X₂ und X₃ Leucin, Y Prolin und Z Isoleucin darstellen, codiert.

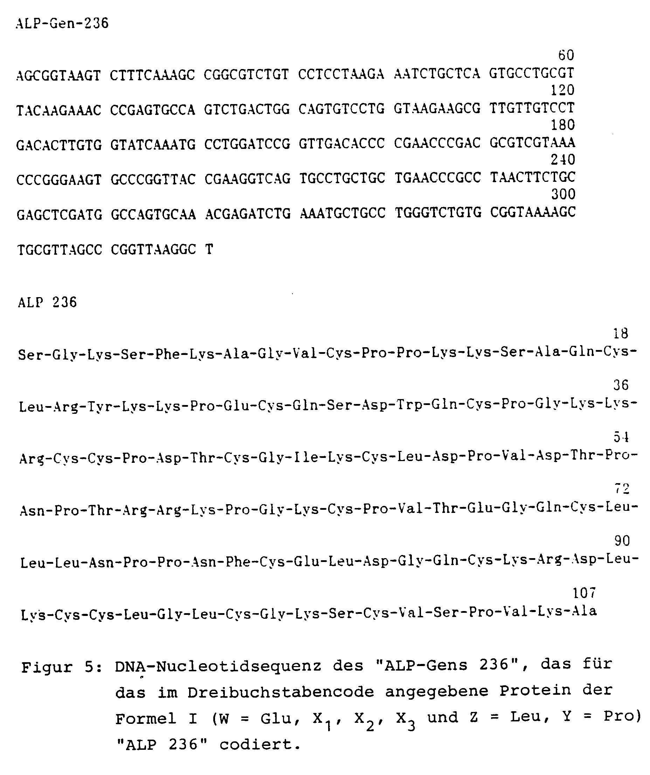

- Figur 5:: Die DNA-Nucleotidsequenz des ALP-Gens 236, das für das mit der Aminosäuresequenz dargestellte Protein "ALP 236", d. h. die Verbindung der Formel I, in der W Glutaminsäure ist, X₁, X₂, X₃ und Z Leucin sind und Y Prolin bedeutet, codiert.
- Figur 6:: Die DNA-Nucleotidsequenz des ALP-Gens 237, das für das mit der Aminosäuresequenz dargestellte Protein "ALP 237", d. h. die Verbindung der Formel I, in der W Tyrosin, X₁, X₂ und Z Leucin und X₃ Phenylalanin sind und Y Prolin bedeutet, codiert.
- Figur 7:: Die DNA-Nucleotidsequenz des ALP-Gens 242, das für das mit der Aminosäuresequenz dargestellte Protein "ALP 242", d. h. die Verbindung der Formel I, in der W Tyrosin, X₁, X₂ und X₃ Methionin, Y Prolin und Z Leucin sind, codiert.
- Figur 8:: Die DNA-Nucleotidsequenz des ALP-Gens 246, das für das mit der Aminosäuresequenz dargestellte Protein "ALP 246", d. h. die Verbindung der Formel I, in der W Glutaminsäure, X₁, X₂ und X₃ Methionin, Y Prolin und Z Leucin sind, codiert.
- Figur 9:: Hemmung von HLE durch authentische ALP sowie durch Verbindungen der Formel I. (Testdurchführung wie auf Seite 14 beschrieben.)
- Figur 10:: Hemmung von Chymotrypsin durch authentische ALP sowie durch Verbindungen der Formel I. (Testdurchführung wie auf Seite 15 beschrieben.)
- Figur 11:: Aktivitätsänderung von authentischer ALP und von Verbindungen der Formel I nach 15minütiger Einwirkung von Chloramin T bei 37° C. Dargestellt ist die nach Einwirkung der Chloramin T/Inhibitoransätze (0,1 µg Inhibitor, steigende Konzentrationen an Chloramin T; Versuchsdurchführung wie auf Seite 16 beschrieben) auf 0,2 µg HLE bei 37° C verbleibende HLE-Hemmaktivität.
- Figur 12:: Oxidative Beeinflussung der HLE-Hemmaktivität von authentischer ALP und von Verbindungen der Formel I durch aktivierte polymorphkernige Leukocyten. (Versuchsdurchführung wie auf Seiten 16 - 17 beschrieben). Bei den Kontrollexperimenten wurde die Aktivität von 0,4 µg HLE pro Test als 100 % HLE-Aktivität ("Kontrolle a") gesetzt. Der Balken "Kontrolle b" stellt die relative HLE-Aktivität im Überstand nicht durch PMA stimulierter Leukocyten dar, und als "Kontrolle c" ist die relative HLE-Aktivität von mit PMA stimulierten Leukocyten dargestellt. Für die Teste mit Wirkstoffen ist durch die Balken die jeweils verbleibende relative HLE-Aktivität dargestellt, und zwar in Gegenwart (1) des nichtinaktivierten Inhibitors, (2) des mit nicht stimulierten Leukocyten inkubierten Inhibitors und (3) des mit PMA-stimulierten Leukocyten inkubierten Inhibitors.

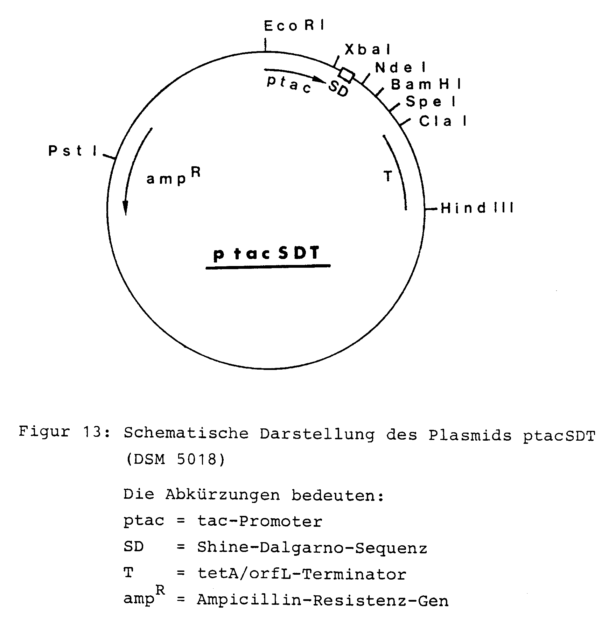

- Figur 13:: Schematische Darstellung des Plasmids ptacSDT (DSM 5018)

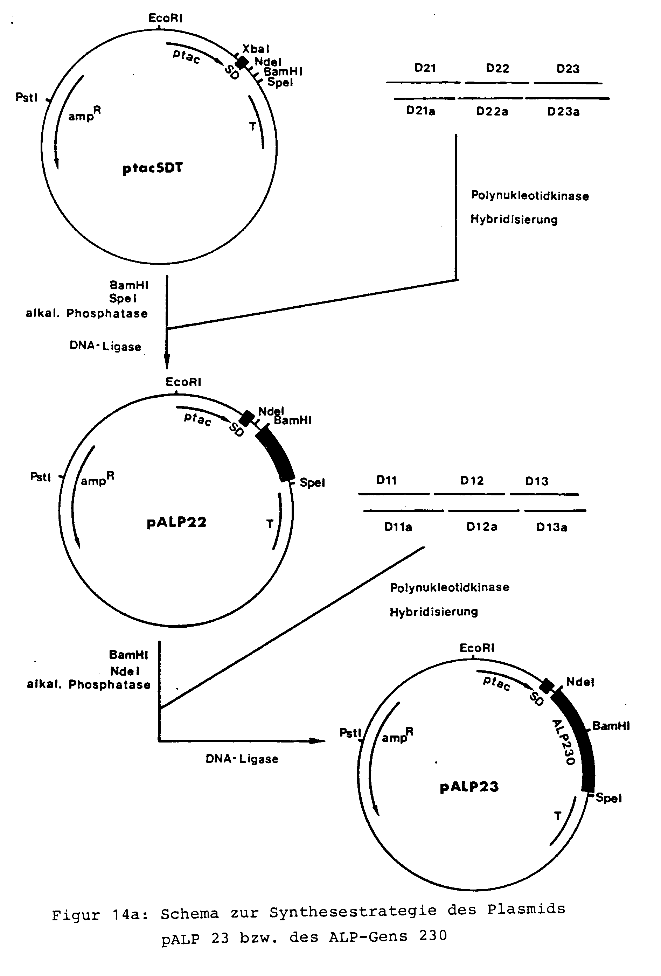

- Figur 14a:: Schema zur Synthesestrategie des Plasmids pALP 23 bzw. des ALP-Gens 230. Rechts oben sind die Oligonucleotide D21, D22, D23, D21a, D22a, und D23a (vgl. Figur 1b) gezeigt, die nach Phosphorylierung und Hybridisierung mit dem durch BamHI und SpeI gespaltenen und mit alkalischer Phosphatase dephosphorylierten Plasmid ptacSDT ligiert werden. Das erhaltene Zwischenprodukt pALP 22 enthält u. a. das durch den schwarzen Balken dargestellte 189 bp BamHI-SpeI-DNA-Fragment. Nach Spaltung mit NdeI und BamHI und Dephosphorylierung wurde es mit den 5′-terminal phosphorylierten und miteinander hybridisierten Oligonucleotiden D11, D12, D13, D11a, D12a und D13a (vgl. Figur 1a) ligiert. Das so gewonnene Plasmid pALP 23 enthält das ALP-Gen 230 (dargestellt als schwarzer Balken; vgl. Figur 2a).

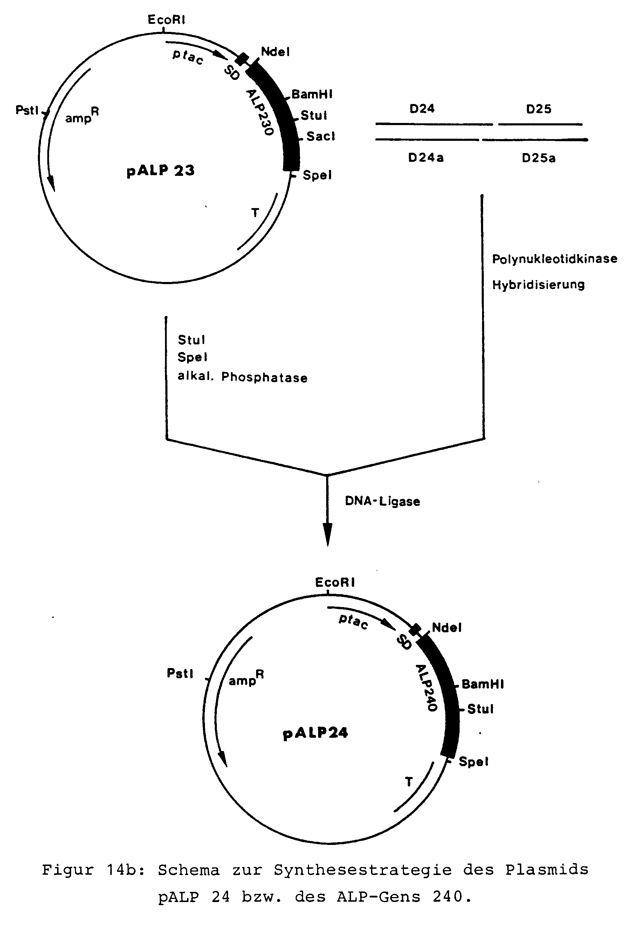

- Figur 14b:: Schema zur Synthesestrategie des Plasmids pALP 24 bzw. des ALP-Gens 240. Die rechts oben gezeigten Oligonucleotide D24, D25, D24a und D25a (vgl. Figur 1c) wurden 5′-terminal phosphoryliert, miteinander hybridisiert und mit dem durch StuI und SpeI gespaltenen, dann dephosphorylierten Fragment des Plasmids pALP 23 ligiert. Das erhaltene Plasmid pALP 24 enthält das als schwarzen Balken dargestellte ALP-Gen 240 (vgl. Figur 2b).

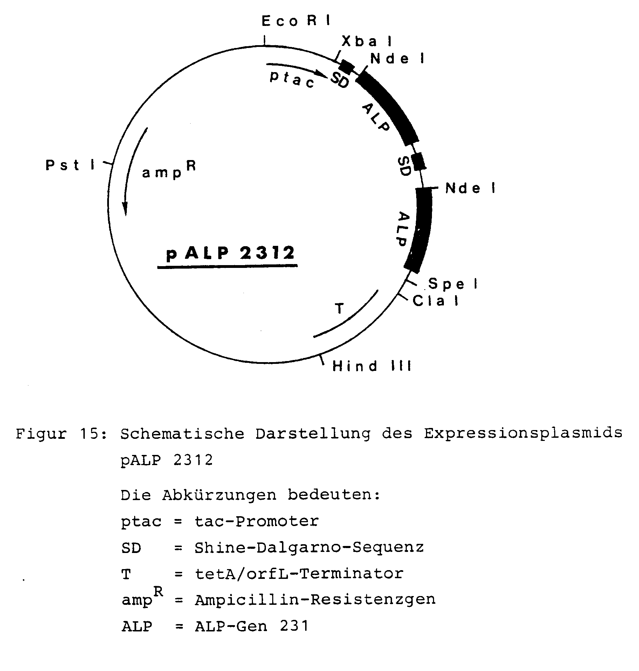

- Figur 15:: Schematische Darstellung des Plasmids pALP 2312. Dieses Plasmid enthält zweifach das ALP-Gen 231 hinter dem tac-Promoter und einer ersten Shine-Dalgarno Sequenz im Abstand von 7 Nucleotiden. Nach den beiden Stop-Codons des ersten Gens folgt im Abstand von 16 Nucleotiden die zweite Shine-Dalgarno-Sequenz, gefolgt von dem zweiten Gen in einem Abstand von wiederum 7 Nucleotiden. Dieses zweite Gen ist schließlich von dem tetA/orf L-Terminator flankiert.

Die auf Seiten 10 und 11 bzw. in Figuren 1a - 1c formelmäßig angegebenen Oligodesoxyribonucleotide wurden in 1µMol-Maßstab nach der Festphasenmethode von Adams et al., J. Am. Chem. Soc. 105, 661 (1983) mit Hilfe eines DNA-Synthesizers (Modell Biosearch 8600 der Firma New Brunswick Scientific Co.) hergestellt. Als monomere Synthone fanden die handelsüblichen β-Cyanoethyl-geschützten Diisopropylamino-Phosphoramidite der jeweils benötigten Desoxyribonucleoside Anwendung. Nach Abspaltung vom Träger wurden die endständig noch tritylgeschützten Stränge unter sterilen Bedingungen durch Gefiltration entsalzt und vorgereinigt und dann in zwei Läufen einer ersten Trennung durch "reversed phase"-HPLC unterworfen. Das jeweilige Hauptprodukt wurde detrityliert und ergab nach zwei weiteren "reversed phase"-HPLC-Reinigungsschritten das gewünschte Oligonucleotid in hoher Reinheit, wie die gelelektrophoretische Analyse (PAGE) zeigte.

Die in den Ausführungsbeispielen verwendeten Restriktionsenzyme sind handelsüblich (vgl. z. B. Nachr. Chem. Techn. Lab. 35, 939, 1987). Für andere Enzyme wird jeweils eine Bezugsquelle angegeben.

In den Ausführungsbeispielen wurde zur Konstruktion von Expressionsvektoren für Verbindungen der Formel I das Plasmid ptacSDT (DSM 5018) verwendet, das alle regulativen Elemente für eine effiziente Expressionsausbeute enthält. Es handelt sich dabei um ein pBR322-Derivat, dessen NdeI-Restriktionsschnittstelle (ehemalige Position 2296) durch Spaltung mit NdeI, "fill in" mit DNA-Polymerase I (Klenow-Fragment; Boehringer Mannheim) und Religation mit T4-DNA-Ligase (Bethesda Research Labs. "BRL") deletiert wurde. Des weiteren wurde das EcoRV-NruI-Fragment (ehemalig Pos. 189 bis 973) durch Spaltung mit beiden Enzymen und anschließende Religation mit T4-DNA-Ligase deletiert. Das Ausgangsexpressionsplasmid ptacSDT trägt ferner einen tac-Promoter (Amann et al., Gene 25, 167 - 178, 1983; de Boer et al., Proc. Natl. Acad. Sci. USA 80, 21 - 25, 1983), eine Shine-Dalgarno-Sequenz, den tetA/orfL-Terminator (Jorgensen et al., loc. cit.), sowie das Ampicillin-Resistenz-Gen. Die regulativen Elemente sind von singulär vorkommenden Restriktionsschnittstellen flankiert, um einzelne Elemente austauschen zu können. Eine schematische Darstellung dieses Plasmids ptacSDT ist in Figur 13 wiedergegeben.

Die nach Expression der erfindungsgemäß erhaltenen Plasmide in entsprechenden Wirtsorganismen gebildeten Proteine der Formel I bzw. Ia werden in der im Beispiel 7b für das Inhibitorprotein ALP 231 beschriebenen Weise aus der Wirtszellmasse isoliert, dem Refolding-Verfahren unterworfen und dann gereinigt. Zweckmäßig wird zur Feinreinigung vor der abschließenden Dialyse noch eine zusätzliche "reversed phase" - HPLC an Nucleosil C 18 (Fa. Macherey und Nagel) mit einem Gradienten von Acetonitril/Trifluoressigsäure in Wasser/Trifluoressigsäure durchgeführt. Als Bedingungen für diese HPLC-Reinigung kommen z. B. in Betracht:
Säulengröße: 0,72 x 25 cm
Temperatur: 25° C
Fließrate: 1,6 ml/min
Puffer A: 0,05 % Trifluoressigsäure in Wasser
Puffer B: 0,025 % Trifluoressigsäure in Acetonitril
Gradient:

| | |
|---|---|
| 0. - 56. Minute | 42 % Puffer B (Rest: Puffer A) |
| 56. - 70. Minute | 60 % Puffer B |
| 70. - 75. Minute | 0 % Puffer B |

Detektion bei 214 nm

Die Das Inhibitorprotein enthaltende Fraktion wird im Vakuum eingeengt und dann vor Weiterbehandlung z. B. in 20 mM Natriumacetat-Puffer, pH 5,4, aufgenommen.

Man erhält so das jeweilige Inhibitorprotein mit einer Reinheit von mehr als 90 %.

Die folgenden Beispiele dienen zur weiteren Erläuterung der Erfindung, welche durch die Angaben der Beispiele in keiner Weise beschränkt wird.

### Beispiel 1

Entsprechend den in der Beschreibung geschilderten Strategien wird zunächst das Plasmid pALP 23 in dem Plasmid ptacSDT (DSM 5018) konstruiert bzw. das ALP-Gen 230 kloniert.
a) Dazu wird gemäß dem Strategieschema aus Figur 14a zunächst das Plasmid ptacSDT mit den Restriktionsendonucleasen BamHI und SpeI gespalten und mit alkalischer Phosphatase aus Kälberdarm (Boehringer Mannheim) dephosphoryliert. Die Oligonucleotide D21, D22, D23 und D21a, D22a, D23a werden 5′-terminal mit T4-Polynucleotidkinase (Boehringer Mannheim) phosphoryliert, miteinander hybridisiert und mit dem vorbereiteten Fragment ptacSDT/Bam HI x Spe I ligiert. Dabei werden die Oligonucleotide und das Fragment in einem molaren Verhältnis von 10 : 1 eingesetzt. Die Ligierungsprodukte werden zur Transformation von kompetenten E.coli-JM 103-Zellen eingesetzt. Nach Selektion auf Ampicillin-Nährböden werden die Klone mit richtig rekombinierten Plasmiden, die ein 189 bp-Bam HI- SpeI-DNA-Fragment enthalten, durch Restriktionsanalyse der DNA identifiziert.
b) Das so gewonnene Plasmidzwischenprodukt, welches die Bezeichnung "pALP 22" erhielt, wird nun mit den Restriktionsendonucleasen Nde I und Bam HI gespalten und mit alkalischer Phosphatase behandelt. Die Oligonucleotide D11, D12, D13 und D11a, D12a, D13a werden 5′-terminal mit T4-Polynucleotidkinase phosphoryliert, miteinander hybridisiert und zur Ligierung mit dem Fragment pALP 22/Bam HI x Nde I und T4-DNA-Ligase eingesetzt. Nach Transformation von kompetenten E.coli-JM 103-Zellen und Selektion auf Ampicillin-Nährböden werden die richtig rekombinierten Plasmide - sie enthalten ein 148 bp Nde I- Bam HI- bzw. ein 337 bp Nde I- Spe I-DNA-Fragment - durch Restriktionsanalyse identifiziert. Das so erhaltene Plasmid wird mit "pALP 23" bezeichnet. Die Verifizierung der neu klonierten DNA-Sequenz ALP 230 - vgl. Figur 2a - erfolgte nach Subklonierung des 189 bp-Bam HI-Spe I-DNA-Fragments bzw. des 167bp-XbaI-Bam HI-DNA-Fragments aus Plasmid pALP 23 in M13 mp18- und M13mp19/Xba I x Bam HI durch Dideoxysequenzierung (Sanger et al., Proc. Natl. Acad. Sci. USA 74, 5463, 1977) mit Standardprimern (Primer M13 "sequencing" der Fa. Boehringer Mannheim).

### Beispiel 2

Entsprechend dem Strategieschema aus Figur 14b wird zur Konstruktion des Plasmids pALP 24 vorgegangen. Dazu wird das in Beispiel 1b erhaltene Plasmid pALP 23 mit den Restriktionsendonucleasen Stu I und Spe I gespalten und dann dephosphoryliert. Die Oligonucleotide D24, D25, D24a und D25a werden 5′-terminal mit T4-Polynucleotidkinase phosphoryliert, miteinander hybridisiert und mit dem Fragment pALP 23/Stu I x Spe I in einem molaren Verhältnis von 10 : 1 zur Ligierung eingesetzt. Die Ligierungsprodukte werden zur Transformation von kompetenten E.coli-JM 103-Zellen eingesetzt. Nach Selektion auf Ampicillin-Nährböden werden richtig rekombinierte pALP 24-Plasmide identifiziert, indem ihr 189bp-Bam HI-Spe I-Fragment wie in Beispiel 1b beschrieben, in M13mp18 und M13mp19 subkloniert und sequenziert wird. Die so synthetisierte DNA-Sequenz "ALP 240", also das ALP 240-Gen, ist in Figur 2b formelmäßig angegeben.

### Beispiel 3

a) Unter Verwendung des Vektors pALP 23 wird das für das Protein "ALP 231" (Verbindung der Formel I in der W Tyrosin ist, X₁, X₂, X₃ und Z Leucin sind und Y Prolin bedeutet) codierende ALP 231-Gen (vgl. Figur 3) synthetisiert. Dazu wird der Expressionsvektor pALP 23 mit den Restriktionsendonucleasen BstE II und Stu I gespalten und dann dephosphoryliert. Die analog Beispiel 1 durchgeführte Klonierung der Oligonucleotide M1 und M2 (vgl. Seite 10) in das Fragment pALP 23/Bst E II x Stu I führte zu dem Plasmid pALP 231, das das ALP 231-Gen zwischen der Nde I- und der Spe I-Spaltstelle des Ausgangsplasmids ptacSDT trägt.
b) Die Klonierung der Oligonucleotide M3 und M4 (vgl. Seite 10) in das nach Beispiel 3a hergestellte Fragment pALP 23/Bst E II x Stu I führt zu dem Plasmid pALP 232 bzw. zum ALP 232-Gen, das für das Protein "ALP 232", also die Verbindung der Formel I in der (vgl. Figur 4) W für Tyrosin, X₁, X₂ und X₃ für Leucin, Y für Prolin und Z für Isoleucin stehen, codiert.
c) Verfährt man wie in Beispiel 3b, kloniert aber in das genannte Fragment die Oligonucleotide M5 und M6 (vgl. Seite 11), so erhält man das Plasmid pALP 236 bzw. das ALP 236-Gen, das für das Inhibitorprotein "ALP 236", also die Verbindung der Formel I in der (vgl. Figur 5) W Glutaminsäure, X₁, X₂, X₃ und Z Leucin und Y Prolin bedeuten, codiert.

### Beispiel 4

Das für das Protein "ALP 237", also die Verbindung der Formel I, in der W Tyrosin bedeutet, X₁, X₂ und Z Leucin sind, X₃ Phenylalanin und Y Prolin darstellen (vgl. Figur 6), codierende ALP 237-Gen wird unter Verwendung des in Beispiel 3a gewonnenen Plasmids pALP 231 wie folgt konstruiert: Das Expressionsplasmid pALP 231 wird mit den Restriktionsendonucleasen Sac I und Spe I gespalten und dann dephosphoryliert. Analog zu dem in Beispiel 1 beschriebenen Vorgehen werden die Oligonucleotide E1, E2 und E3 (vgl. Seite 11) phosphoryliert, hybridisiert und dann mit dem vorbereiteten Fragment pALP 231/Sac I x Spe I ligiert. Das Klonierungsprodukt pALP 237 enthält zwischen den Spaltstellen Nde I und Spe I das ALP 237-Gen. Dessen Sequenz wurde durch DNA-Sequenzierung verifiziert.

### Beispiel 5

a) Man verfährt wie in Beispiel 3a, verwendet aber statt des Vektors pALP 23 (bzw. dessen Fragment pALP 23/Bst E II x Stu I) den in Beispiel 2 erhaltenen Vektor pALP 24 (bzw. dessen dephosphoryliertes Spaltprodukt pALP 24/ Bst E II x Stu I) und kloniert in diesen die Oligonucleotide M1 und M2. Man erhält so das Plasmid pALP 242 bzw. das für das Protein "ALP 242" (also die Verbindung der Formel I, in der W Tyrosin, X₁, X₂ und X₃ Methionin, Y Prolin und Z Leucin bedeuten) codierende ALP 242-Gen (vgl. Figur 7).
b) Geht man analog Beispiel 5a vor, kloniert aber in das Fragment pALP 24/Bst EII x Stu I die Oligonucleotide M5 und M6 (vgl. Seite 11), so erhält man das Plasmid pALP 246 bzw. das ALP 246-Gen, welches für das Inhibitorprotein ALP 246 codiert, also für diejenige Verbindung der Formel I, in der W Glutaminsäure, X₁, X₂ und X₃ Methionin, Y Prolin und Z Leucin bedeuten (vgl. Figur 8).

### Beispiel 6

In einer bevorzugten Ausführungsform der Erfindung werden solche Expressionsplasmide konstruiert und zur Expression der Verbindungen der Formel I mit erhöhter Ausbeute eingesetzt, die die entsprechenden Gene zweifach hinter dem tac-Promoter tragen.
a) Zur Konstruktion des Plasmids pALP 2312, das schematisch in Figur 15 dargestellt ist, wird vom Expressionsplasmid pALP 231 (erhalten in Beispiel 3a) ausgegangen, welches mit den Restriktionsendonucleasen Pst I und Spe I gespalten wird. Durch präparative Gelelektrophorese und anschließende Gelelution wird das Fragment, welches den Anfang des Ampicillin-Resistenzgens, den tac-Promoter, die Shine-Dalgarno-Sequenz und das ALP 231-Gen trägt, isoliert.
   In einem zweiten Ansatz wird das Plasmid pALP 231 mit den Restriktionsendonucleasen Pst I und Xba I gespalten, mit alkalischer Phosphatase aus Kälberdarm 5′-terminal dephosphoryliert und zusammen mit dem vorher isolierten Pst I-Spe I-Fragment und dem Enzym T4-DNA-Ligase ligiert. Die Transformation wird mit kompetenten E.coli-JM 103-Zellen durchgeführt. Die Selektion erfolgt auf ampicillinhaltigem gem Nährboden. Durch Restriktionsanalyse werden Klone identifiziert, die zwei ALP-231-Gene hintereinander enthalten. Dabei tragen jeweils 5′-terminal beide Gene die gleiche Shine-Dalgarno-Sequenz im Abstand von 7 Nucleotiden. Nach den beiden Stop-Codons des ersten Gens folgt im Abstand von 16 Nucleotiden die zweite Shine-Dalgarno-Sequenz gefolgt von dem zweiten Gen in einem Abstand von wiederum 7 Nucleotiden. Dieses neue Expressionsplasmid erhält die Bezeichnung pALP 2312.
b) Man verfährt ausgehend vom Plasmid pALP 232 (erhalten gemäß Beispiel 3b) in der in Beispiel 6a geschilderten Weise und erhält so das Expressionsplasmid pALP 2322, das zweifach das ALP 232-Gen enthält.
c) Analog Beispielen 6a und 6b wird ausgehend von den Plasmiden pALP 23 (Beispiel 1b), pALP 24 (Beispiel 2), pALP 236 (Beispiel 3c), pALP 237 (Beispiel 4), bzw. pALP 242 (Beispiel 5a) bzw. pALP 246 (Beispiel 5b) vorgegangen, wobei man die Expressionsplasmide pALP 2302, pALP 2402, pALP 2362, pALP 2372, PALP 2422 bzw. pALP 2462 erhält, die zweifach die Gene ALP 230, ALP 240, ALP 236, ALP 237, ALP 242 bzw. ALP 246 enthalten.

### Beispiel 7

a) Kompetente Zellen des Stammes E.coli K12 JM 103 (ATCC 39 403) werden mit dem Plasmid pALP 2312, erhalten in Beispiel 6a, transformiert. Der so gewonnene Stamm E.coli K12 JM 103/pALP 2312 wird in 1 l SM-Medium (5 g/l Ammoniumsulfat, 6 g/l Di-kaliumhydrogenphosphat, 3 g/l Natriumdihydrogenphosphat, 10 g/l Hefeextrakt, 0,25 g/l Magnesiumsulfat, 10 g/l Glukose) unter Ampicillinselektionsdruck (100 mg/l) bei 22° C in einem Biostat-M-Fermenter (Fa. Braun, Melsungen) kultiviert. Bei einer optischen Dichte von 5, gemessen bei 578 nm, wird durch Zugabe von 75 mg/l Isopropylthio-β-D-galactopyranosid die Expressionsphase eingeleitet. Während der Expressionsphase wird das Medium durch viermalige Zugabe (im Abstand von jeweils einer Stunde) von je 5 g Hefeextrakt supplementiert. 5 Stunden nach Induktionsbeginn werden die E.coli-Zellen durch Zentrifugieren geerntet. Die Expressionsausbeute beträgt ca. 3 % Inhibitorprotein, bezogen auf das E.coli-Gesamtprotein.
b) Zur Isolierung, Rückfaltung ("Refolding") und Reinigung des Inhibitorproteins ALP 231 werden die Zellsedimente (ca. 25 g) in 350 ml eines 50 mM Tris-HCl (pH 7,5), 5 mM EDTA und 5 mM DTT enthaltenden Gemisches suspendiert und durch zwei Umläufe in einem Hochdruckhomogenisator aufgeschlossen. Zur Abtrennung der unlöslichen Bestandteile wird für 20 min. bei 15 000 g und 4° C zentrifugiert. Ca. 80 - 90 % des Proteins ALP 231 befinden sich in der löslichen Fraktion (Überstand). Zur Einleitung der Rückfaltung in die enzymatisch aktive Form wird der Überstand auf 20 mM DTT eingestellt, 1 Stunde bei 4° C inkubiert und an 50 ml Bettvolumen SP-Sephadex-C25 (Pharmacia Fine Chemicals, Inc.), welches mit einem 50 mM Tris-HCl (pH 7,5), 5 mM EDTA, 20 mM DTT enthaltenden Gemisch equilibriert worden ist, chromatographiert. Das Säulenmaterial wird mit drei Säulenvolumina 50 mM Tris-HCl (pH 7,5), 5 mM EDTA, 5 mM DTT, zwei Säulenvolumina 50 mM Tris-HCl (pH 7,5), 1 mM EDTA, 5 mM DTT, 130 mM NaCl gewaschen, und das Protein ALP 231 wird dann mit zwei Säulenvolumina eines 50 mM Tris-HCl (pH 7,5), 1 mM EDTA, 5 mM DTT, 300 mM NaCl enthaltenden Gemisches eluiert. Die ALP 231-Lösung wird auf 3,5 M Guanidinium-Hydrochlorid eingestellt, 1 Stunde bei 22° C inkubiert, 1 : 7 mit 100 mM Tris-HCl (pH 9) verdünnt, auf 3 mM Cystein eingestellt und 6 Stunden bei 22° C inkubiert. Zur erneuten Chromatographie an SP-Sephadex-C25 wird der Reaktionsansatz 1 : 4 mit 50 mM Tris-HCl (pH 7,5) verdünnt, mit HCl auf pH 7,5 titriert und an 10 ml Säulenmaterial (equilibriert mit 50 mM Tris-HCL (pH 7,5) und 125 mM NaCl) chromatographiert. Das Säulenmaterial wird mit fünf Säulenvolumina 50 mM Tris-HCl (pH 7,5), 125 mM NaCl gewaschen und mit zwei Säulenvolumina eines 50 mM Tris-HCl (pH 7,5) und 500 mM NaCl enthaltenden Gemisches eluiert. Die Proteinfraktion wird - gegebenenfalls nach Feinreinigung durch reversed-phase-HPLC (vgl. Seiten 25 - 26) gegen 50 mM Tris-HCl (pH 7,5) über Nacht bei 4° C dialysiert.
   Die Konzentrationsbestimmung von ALP 231 wird mit Hilfe eines doppelten "Sandwich-ELISAs" durchgeführt (Kramps et al., Am. Rev. Respir. Dis. 129, 959 - 963, 1984).
   Alternativ ist ein quantitative Bestimmung des ALP 231 (wie auch die weiterer Proteine der Formel I) aber auch z. B. mit Hilfe einer quantitativen Aminosäureanalyse möglich.
   Die Homogenität und Identität der erhalten Verbindung der Formel I "ALP 231" wird durch N-terminale Sequenzanalyse sowie durch die Ermittlung der Aminosäurezusammensetzung bestätigt. Die Hemmaktivität des Produktes gegenüber HLE und Chymotrypsin sowie seine gesteigerte Oxidationsstabilität ergeben sich z. B. aus den Figuren 9 - 12.

### Beispiel 8

Zur Abspaltung der C-terminalen ALP-231-Domäne wird das in Beispiel 7b isolierte, 107 Aminosäuren enthaltende Protein der Formel I, worin W Tyrosin, X₁, X₂, X₃ und Z Leucin und Y Prolin bedeuten, in 70 %iger Ameisensäure gelöst. Das Vorhandensein einer säurelabilen Asparaginsäure-Prolin-Bindung (vgl. Formel I, Positionen 49 - 50) führt nach einer Reaktionszeit von 24 - 36 Stunden bei Raumtemperatur zi einer wenigstens 20 %igen Abspaltung der C-terminalen ALP-231-Domäne. Diese intakte Domäne kann nach dieser Säurebehandlung durch HPLC abgetrennt und gereinigt werden. Ihre Aminosäuresequenz ist identisch mit den Positionen 50 - 107 des gesamten ALP-231-Proteins:
Pro-Val-Asp-Thr-Pro-Asn-Pro-Thr-Arg-Arg-Lys-Pro-Gly-Lys-Cys-Pro-Val-Thr-Tyr-Gly-Gln-Cys-Leu-Leu-Leu-Asn-Pro-Pro-Asn-Phe-Cys-Glu-Leu-Asp-Gly-Gln-Cys-Lys-Arg-Asp-Leu-Lys-Cys-Cys-Leu-Gly-Leu-Cys-Gly-Lys-Ser-Cys-Val-Ser-Pro-Val-Lys-Ala

In der folgenden Tabelle sind die oben genannten hinterlegten Mikroorganismen zusammengefaßt:

| Mikroorganismus | Hinterlegungs- | |
|---|---|---|
| | stelle | nummer |
| pBR 322 | ATCC | 31 344 |
| ptacSDT (in E.coli K12 JM 103) | DSM | 5 018 |
| E.coli K12 JM 101 | ATCC | 33 876 |
| E.coli K12 JM 103 | ATCC | 39 403 |
| E.coli K12 JM 105 | DSM | 4 162 |
| E.coli K12 DH 1 | ATCC | 33 849 |

## Patentansprüche (Patentansprüche für folgende(n) Vertragsstaat(en): AT, BE, CH, DE, FR, GB, IT, LI, LU, NL, SE)

1. Serinprotease-Inhibitor-Proteine der Formel (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 oder Teilbereichen davon, weiche zumindest die Sequenz der Positionen 53 - 101 umfassen,
worin W für Tyrosin oder Glutaminsäure steht, X₁ und X₂ gleich oder verschieden sind und jeder dieser Reste Methionin oder Leucin bedeutet, X₃ Methionin, Leucin oder Phenylalanin darstellt, Y für Prolin oder Arginin steht und Z Leucin oder, wenn W für Tyrosin und X₁, X₂ und X₃ für Leucin stehen, auch Isoleucin bedeutet,
und Fusionsproteine dieser neuen Serinprotease-Inhibitorproteine.

2. Serinprotease-Inhibitor-Proteine der Formel I oder ein zumindest die Sequenz der Positionen 53 - 101 umfassender Teilbereich davon, worin W Tyrosin bedeutet.

3. Serinprotease-Inhibitor-Proteine gemäß Ansprüchen 1 und 2 der Formel (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2--Gly--X'3--Cys-Gly-Lys-Ser-Cys-Val-Ser-Pro-Val-Lys-Ala
 oder Teilbereichen davon, welche zumindest die Sequenz der Positionen 53 - 101 umfassen,
worin X₁, X₂, Y und Z die gleiche Bedeutung wie in Anspruch 1 haben und X₃' für Methionin oder Leucin steht,
und Fusionsproteine dieser neuen Serinprotease-Inhibitorproteinp.

4. Serinprotease-Inhibitorproteine gemäß Ansprüchen 1 bis 3, die Teilbereiche der Formeln I bzw. Ia darstellen und die Sequenzbereiche der Positionen 53 - 101 bis die der Positionen 48 - 107 umfassen.

5. Serinprotease-Inhibitor-Proteine gemäß Ansprüchen 1 bis 3, welche die Aminosäurepositionen 1 bis 107 oder die Teilsequenzen 48 - 107, 49 - 107 oder 50 - 107 aus Formeln I oder Ia umfassen.

6. Serinprotease-Inhibitor-Proteine gemäß Ansprüchen 1, 4 und 5 der Formel 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 worin W, X₁, X₂, X₃, Y und Z die gleiche Bedeutung wie in Anspruch 1 haben.

7. Serinprotease-Inhibitorproteine gemäß Ansprüchen 1 - 6, in denen X₁, X₂, X₃ und Z für Leucin stehen.

8. Serinprotease-Inhibitor-Protein mit der Aminosäuresequenz gemäß Figur 3.

9. Serinprotease-Inhibitor-Protein mit der Aminosäuresequenz gemäß Figur 4.

10. Serinprotease-Inhibitor-Protein mit der Aminosäuresequenz gemäß Figur 5.

11. Serinprotease-Inhibitor-Protein mit der Aminosäuresequenz gemäß Figur 6.

12. Serinprotease-Inhibitor-Protein mit der Aminosäuresequenz gemäß Figur 7.

13. Serinprotease-Inhibitor-Protein mit der Aminosäuresequenz gemäß Figur 8.

14. Serinprotease-Inhibitor-Protein mit der Aminosäuresequenz

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. DNA-Sequenzen, dadurch gekennzeichnet, daß sie für Serinprotease-Inhibitor-Proteine gemäß Ansprüchen 1 - 14 codieren.

16. DNA-Sequenz gemäß Anspruch 15, dadurch gekennzeichnet, daß sie eine der in Figuren 2a, 2b oder 3 bis 8 dargestellten Nucleotidsequenzen aufweist.

17. Rekomoinanter Vektor, dadurch gekennzeichnet, daß er eine codierende DNA-Sequenz nach einem der Ansprüche 15 oder 16 aufweist.

18. Rekombinanter Vektor gemäß Anspruch 17, dadurch gekennzeichnet, daß er ein Derivat des Plasmids ptacSDT (DSM 5018) ist.

19. Als rekomoinante Vektoren gemäß Ansprüchen 17 und 18 die Plasmide pALP 23, erhältlich gemäß Fig. 14a, und pALP 24, erhältlich gemäß Fig. 14b.

20. Als rekombinante Vektoren gemäß Ansprüchen 17 bis 19 die Plasmide pALP 231, erhältlich aus pALP 23 (Fig. 14a) durch Spaltung und Dephosphorylierung von pALP 23 mit den Restriktionsendonukleasen BstE II und Stu I und Klonierung der Oligonukleotide M1 und M2 in das Fragment pALP 23/Bst E II x Stu I, pALP 232, erhältlich aus pALP 23 (Fig. 14a) durch Spaltung und Dephosphorylierung von pALP 23 mit den Restriktionsendonukleasen BstE II und Stu I und Klonierung der Oligonukleotide M3 und M4 in das Fragment pALP 23/Bst E II x Stu I, pALP 236, erhältlich aus pALP 23 (Fig. 14a) durch Spaltung und Dephosphorylierung von pALP 23 mit den Restriktionsendonukleasen BstE II und Stu I und Klonierung der Oligonukleotide M5 und M6 in das Fragment pALP 23/Bst E II x Stu I, pALP 237, erhältlich aus pALP 231 durch Spaltung und Dephosphorylierung von pALP 231 mit den Restriktionsendonukleasen Sac I und Spe I und Klonierung der Oligonukleotide E1, E2 und E3 in das Fragment pALP 231/Sac I x Spe I, pALP 242, erhältlich aus pALP 24 (Fig. 14b) durch Spaltung und Dephosphorylierung von pALP 24 mit den Restriktionsnukleasen BstE II und Stu I und Klonierung der Oligonukleotide M1 und M2 in das Fragment pALP 24/Bst E II x Stu I und pALP 246, erhältlich aus pALP 24 (Fig. 14b) durch Spaltung und Dephosphorylierung von pALP 24 mit den Restriktionsendonukleasen BstE II und Stu I und Klonierung der Oligonukleotide M5 und M6 in das Fragment pALP 24/BstE II x Stu I.

21. Als rekombinante Vektoren gemäß Ansprüchen 17 bis 20 die Plasmide pALP 231, pALP 232 und pALP 242.

22. Rekombinanter Vektor gemäß Anspruch 17 oder 18, dadurch gekennzeichnet, daß er eine codierende DNA-Sequenz nach einem der Ansprüche 15 oder 16 zweifach aufweist.

23. Rekombinanter Vektor gemäß Anspruch 22, dadurch gekennzeichnet, daß die beiden codierenden DNA-Sequenzen (nach einem der Ansprüche 15 oder 16) jeweils 5'-terminal die gleiche Shine-Dalgarno-Sequenz im Abstand von etwa 7 Nucleotiden tragen, wobei nach den beiden Stop-Codons der ersten codierenden DNA-Sequenz im Abstand von etwa 16 Nucleotiden die zweite Shine-Dalgarno-Sequenz folgt, der sich im Abstand von etwa 7 Nucleotiden die zweite codierende DNA-Sequenz anschließt.

24. Als rekombinante Vektoren gemäß Ansprüchen 17 bis 23 die Plasmide pALP 2302 gemäß Fig. 15 mit 2 ALP 230-Genen mit der in Fig. 2a angegebenen Sequenz anstelle von 2 ALP 231-Genen, pALP 2312 gemäß Fig. 15, erhältlich aus pALP 23 (Fig. 14a), pALP 2322 gemäß Fig. 15 mit 2 ALP 232-Genen anstelle von 2 ALP 231-Genen, pALP 2362 gemäß Fig. 15 mit 2 ALP 236-Genen anstellen von 2 ALP 231-Genen, pALP 2372 gemäß Fig. 15 mit 2 ALP 237-Genen anstelle von 2 ALP 231-Genen, pALP 2402 gemäß Fig. 15 mit 2 ALP 240-Genen mit der Sequenz gemäß Fig. 2b anstelle von 2 ALP 231-Genen, pALP 2422 gemäß Fig. 15 mit 2 ALP 242-Genen anstelle von 2 ALP 231-Genen und pALP 2462 gemäß Fig. 15 mit 2 ALP 246-Genen anstelle von 2 ALP 231-Genen.

25. Als rekombinante Vektoren gemäß Ansprüchen 17 bis 24 die Plasmide pALP 2302, pALP 2312, pALP 2322, pALP 2402 und pALP 2422.

26. Als rekombinanter Vektor gemäß Ansprüchen 17 bis 25 das Plasmid pALP 2312 entsprechend Figur 15, erhältlich aus pALP 23 (Fig. 14a).

27. Wirtsorganismus, dadurch gekennzeichnet, daß er mit einem der rekombinanten Vektoren nach Ansprüchen 17 bis 26 transformiert ist.

28. Wirtsorganismus gemäß Anspruch 27, dadurch gekennzeichnet, daß er ein Bakterium, vorzugsweise ein Stamm der Art E.coli oder B.subtilis, ein mikroskopisch kleiner Pilz, vorzugsweise der Arten Aspergillus oryzae, Aspergillus niger oder Saccharomyces cerevisiae oder eine tierische oder menschliche Zelle ist.

29. Wirtsorganismus gemäß Ansprüchen 27 und 28, dadurch gekennzeichnet, daß er ein Stamm der Art E.coli, insbesondere der Untergruppe K12 ist.

30. Wirtsorganismus gemäß Ansprüchen 27 bis 29, der aus der die folgenden Stämme umfassenden Gruppe ausgewählt ist: E.coli K12 JM 101 (ATCC 33876), E.coli K12 JM 103 (ATCC 39403), E.coli K12 JM 105 (DSM 4162) und E.coli K12 DH1 (ATCC 33849).

31. Verfahren zur Synthese der DNA-Sequenzen gemäß Ansprüchen 15 und 16, dadurch gekennzeichnet, daß man ausgehend von dem Plasmid ptacSDT (DSM 5018) in an sich bekannter Weise unter Benutzung der Oligonucleotide D21 bis D23a gemäß Figur 1b und in einer weiteren Stufe unter Benutzung der Oligonucleotide D11 bis D13a gemäß Figur 1a das Plasmid pALP23, enthaltend die in Figur 2a dargestellte DNA-Sequenz, gemäß Fig. 14a konstruiert, dieses gegebenenfalls unter Mitverwendung der Oligonucleotide D24 - D25a gemäß Figur 1c zur Konstruktion des Plasmids pALP 24 gemäß Fig. 14b, enthaltend die in Figur 2b dargestellte DNA-Sequenz, einsetzt, gegebenenfalls in die so erhaltenen Plasmide pALP 23 oder pALP 24 dann paarweise solche Oligonucleotide kloniert, daß die resultierenden Plasmide für Proteine der Formel I, in denen X₃ Methionin oder Leucin ist und W, X₁, X₂, Y und Z die gleiche Bedeutung wie oben haben, codierende DNA-Sequenzen enthalten und diese gegebenenfalls mit Hilfe weiterer Oligonucleotide so modifiziert, daß sie für ein Protein der Formel I, in dem X₃ Phenylalanin ist und W, X₁, X₂, Y und Z die gleiche Bedeutung wie oben haben, codierende DNA-Sequenzen aufweisen.

32. Verfahren gemäß Anspruch 31, dadurch gekennzeichnet, daß man in das Plasmid pALP 23 (erhältlich gemäß Fig. 14a) oder in das Plasmid pALP 24 (erhältlich gemäß Fig. 14b) paarweise die Oligonucleotide M1 und M2, M3 und M4 oder M5 und M6 der Formeln
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
und
3' - GCTT CCA GTC ACG GAC GAC GAC TTG GGC - 5' (M6)
kloniert und so DNA-Sequenzen herstellt, die für Verbindungen der Formel I codieren, in denen X₃ für Methionin oder Leucin steht und W, X₁, X₂, Y und Z die gleiche Bedeutung wie oben haben.

33. Verfahren gemäß Ansprüchen 31 und 32, dadurch gekennzeichnet, daß man zur Synthese einer DNA-Sequenz, die für ein Serinprotease-Inhibitorprotein der Formel I codiert, worin X₃ Phenylalanin bedeutet, in eine gemäß Anspruch 32 erhaltene DNA-Sequenz solche Oligonucleotide kloniert, die an Position 96 der Formel I die Expression von Phenylalanin bewirken.

34. Verfahren gemäß Ansprüchen 31 bis 33, dadurch gekennzeichnet, daß man zur Herstellung einer DNA-Sequenz, die für eine Verbindung der Formel I codiert, in der X₁ und X₂ für Leucin und X₃ für Phenylalanin stehen und W, Y und Z die gleiche Bedeutung wie oben haben, in das Plasmid pALP 23 (erhältlich gemäß Fig. 14a) paarweise die Oligonucleotide M1 und M2, M3 und M4 oder M5 und M6 kloniert, das erhaltene Plasmid mit den Restriktionsendonucleasen Sac I und Spe I spaltet, anschließend in üblicher Weise dephosphoryliert und in das so erhaltene Fragment das Hybridisierungsprodukt der (phosphorylierten) Oligonucleotide E1, E2 und E3 der Formeln
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
und
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
ligiert.

35. Verfahren zur Synthese der in Figur 6 dargestellten, für das Serinprotease-Inhibitorprotein ALP 237 codierenden DNA-Sequenz, dadurch gekennzeichnet, daß das durch Klonieren der Oligonucleotide M1 und M2 in das Plasmid pALP 23 (konstruiert gemäß Fig. 14a) erhaltene Plasmid pALP 231 mit den Restriktionsendonucleasen Sac I und Spe I gespalten und dann in üblicher Weise dephosphoryliert wird und man in das so erhaltene Fragment pALP 231/Sac I x Spe I das Hybridisierungsprodukt der (phosphorylierten) Oligonucleotide E1, E2 und E3 ligiert.

36. Verfahren zur Herstellung eines rekombinanten Vektors zur Expression einer Verbindung der Formeln I oder Ia, dadurch gekennzeichnet, daß eine nach den Ansprüchen 31 - 35 erhaltene DNA-Sequenz in ein Vektor-Molekül so eingesetzt wird, daß sie funktionell mit einer Expressions-Kontroll-Sequenz, vorzugsweise beginnend mit einem tac-Promoter und endend mit einer Terminatorsequenz, verbunden ist.

37. Verfahren zur Herstellung eines rekombinanten Vektors zur Expression einer Verbindung der Formeln I oder Ia, dadurch gekennzeichnet, daß der gemäß Anspruch 36 erhaltene Expressionsvektor in an sich bekannter Weise in Teilmengen mit unterschiedlichen Paaren von Restriktionsendonucleasen gespalten wird, eines der erhaltenen Fragmente gegebenenfalls dephosphoryliert wird und dann diejenigen Fragmente ligiert werden, die zu einem Expressionsvektor führen, der die für die Verbindung der Formel I bzw. Ia codierende DNA-Sequenz hinter dem tac-Promoter zweifach, getrennt durch zwei Stoppcodons, eine etwa 16 Nucleotide enthaltende Sequenz, eine Shine-Dalgarno-Sequenz und eine weitere Sequenz mit etwa 7 Nucleotiden, trägt.

38. Verfahren gemäß Anspruch 37 zur Herstellung eines rekombinanten Vektors zur Expression einer Verbindung der Formel I bzw. Ia, dadurch gekennzeichnet, daß eine Teilmenge des gemäß Anspruch 36 erhaltenen Expressionsvektors mit den Restriktionsendonucleasen Pst I und Spe I die andere Teilmenge dieses Vektors mit den Restriktionsendonucleasen Pst I und Xba I gespalten wird und die dabei erhaltenen Fragmente, die die für die Verbindung der Formel I bzw. Ia codierende DNA-Sequenz enthalten, gegebenenfalls nach Dephosphorylierung, ligiert werden.

39. Verfahren zur Herstellung eines Serinprotease-Inhibitorproteins gemäß Ansprüchen 1 bis 14, dadurch gekennzeichnet, daß man einen Wirtsorganismus nach einem der Ansprüche 27 bis 30 in einem üblichen Nährmedium züchtet, gegebenenfalls die Expression des gewünschten Proteins induziert und dann das Expressionsprodukt aus der Kultur isoliert, einem Refolding-Verfahren durch Einwirkung reduzierender und darauffolgend oxidierender Mittel unterwirft und schließlich das erhaltene biologisch aktive Serinprotease-Inhibitorprotein der Formel I in an sich bekannter Weise isoliert und reinigt.

40. Verfahren zur Herstellung eines Serinprotease-Inhibitorproteins gemäß Ansprüchen 1 - 7 und 14, umfassend Teilbereiche der Aminosäuresequenzen nach Formeln I oder Ia, dadurch gekennzeichnet, daß man das beim Vorgehen nach Anspruch 39 gebildete Expressionsprodukt aus der Kultur isoliert und dann einer kontrollierten sauren Hydrolyse zur Spaltung der Asparaginsäure-Prolin-Bindung in den Positionen 49 - 50 und/ oder einer kontrollierten enzymatischen Abspaltung aller oder nur einer bis fünf der Aminosäuren von Positionen 107 - 102 unterwirft und das so erhaltene Hydrolyseprodukt aus dem Hydrolysat gewinnt.

41. Verfahren zur Herstellung eines Serinprotease-Inhibitorproteins gemäß Ansprüchen 1 bis 5, umfassend zumindest die Teilbereiche der Positionen 53 - 101 der Aminosäuresequenzen nach Formeln I oder Ia, in denen X₁ und X₂ für Leucin und X₃ für Leucin oder Phenylalanin stehen, dadurch gekennzeichnet, daß man an die für das Inhibitorprotein codierende DNA-Sequenz 5'-terminal das Codon ATG anfügt und in dem bei der Expression gebildeten, vor dem N-Terminus des herzustellenden Proteins die zusätzliche Aminosäure Methionin aufweisenden Produkt dann in üblicher Weise, vorzugsweise mit Brom-cyan, die Bindung zwischen dem Methionin und der ersten (N-terminalen) Aminosäure des gewünschten Inhibitorproteins spaltet.

42. Arzneimittel, gekennzeichnet durch einen Gehalt an einem der Serinprotease-Inhibitor-Proteine nach Ansprüchen 1 bis 14 und üblichen Trägerstoffen und/oder Verdünnungsmitteln und/oder Hilfsstoffen.

43. Verwendung eines Serinprotease-Inhibitor-Proteins nach einem der Ansprüche 1 bis 14 zur Herstellung eines Arzneimittels zur Behandlung und/oder Prophylaxe von Gerinnungsstörungen, invasivem Wachstum von Tumoren, Emphysemen, Arthritis, Nierenentzündung und insbesondere excessiven Entzündungen beim polytraumatischen oder septischen Schock oder bei der Pankreatitis und weiterer Krankheitsbildern, die mit einem gestörten Gleichgewicht zwischen Serinproteasen und Serinprotease-Inhibitoren einhergehen.

## Patentansprüche (Patentansprüche für folgende(n) Vertragsstaat(en): ES)

1. Verfahren zur Synthese von DNA-Sequenzen, die für neue Serinprotease-Inhibitorproteine der Formel (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 oder Teilbereichen davon, welche zumindest die Sequenz der Positionen 53 - 101 umfassen,
worin W für Tyrosin oder Glutaminsäure steht, X₁ und X₂ gleich oder verschieden sind und jeder dieser Reste Methionin oder Leucin bedeutet, X₃ Methionin, Leucin oder Phenylalanin darstellt, Y für Prolin oder Arginin steht und Z Leucin oder, wenn W für Tyrosin und X₁, X₂ und X₃ für Leucin stehen, auch Isoleucin bedeutet,
oder für Fusionsproteine dieser neuen Serinprotease-Inhibitorproteine codieren, dadurch gekennzeichnet, daß man ausgehend von dem Plasmid ptacSDT (DSM 5018) in an sich bekannter Weise unter Benutzung der Oligonucleotide D21 bis D23a gemäß Figur 1b und in einer weiteren Stufe unter Benutzung der Oligonucleotide D11 bis D13a gemäß Figur 1a das Plasmid pALP 23, enthaltend die in Figur 2a dargestellte DNA-Sequenz, gemäß Fig. 14a konstruiert, dieses gegenenfalls unter Mitverwendung der Oligonuleotide D24 - D25a gemäß Figur 1c zur Konstruktion des Plasmids pALP 24 gemäß Fig. 14b, enthaltend die in Figur 2b dargestellte DNA-Sequenz, einsetzt, gegebenenfalls in die so erhaltenen Plasmide pALP 23 oder pALP 24 dann paarweise solche Oligonucleotide kloniert, daß die resultierenden Plasmide für Proteine der Formel I, in denen X₃ Methionin oder Leucin ist und W, X₁, X₂, Y und Z die gleiche Bedeutung wie oben haben, codierende DNA-Sequenzen enthalten und diese gegebenenfalls mit Hilfe weiterer Oligonucleotide so modifiziert, daß sie für ein Protein der Formel I, in dem X₃ Phenylalanin ist und W, X₁, X₂, Y und Z die gleiche Bedeutung wie oben haben, codierende DNA-Sequenzen aufweisen.

2. Verfahren gemäß Anspruch 1, dadurch gekennzeichnet, daß man in das Plasmid pALP 23 (erhältlich gemäß Fig. 14a) oder in das Plasmid pALP 24 (erhältlich gemäß Fig. 14b) paarweise die Oligonucleotide M1 und M2, M3 und M4 oder M5 und M6 der Formeln
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
und
3' - GCTT CCA GTC ACG GAC GAC GAC TTG GGC - 5' (M6)
kloniert und so DNA-Sequenzen herstellt, die für Verbindungen der Formel I codieren, in denen X₃ für Methionin oder Leucin steht und W, X₁, X₂, Y und Z die gleiche Bedeutung wie oben haben.

3. Verfahren gemäß Ansprüchen 1 und 2, dadurch gekennzeichnet, daß man zur Synthese einer DNA-Sequenz, die für ein Serinprotease-Inhibitorprotein der Formel Ia: 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X'3--Cys-Gly-Lys-Ser-Cys-Val-Ser-Pro-Val-Lys-Ala
 oder Teilbereichen davon, welche zumindest die Sequenz der Positionen 53 - 101 umfassen,
worin X₁, X₂, Y und Z die gleiche Bedeutung wie in Anspruch 1 haben und X₃' für Methionin oder Leucin steht,
codiert, in das Plasmid pALP 23 (erhältlich gemäß Fig. 14a) oder in das Plasmid pALP 24 (erhältlich gemäß Fig. 14b) paarweise die Oligonucleotide M1 und M2 oder M3 und M4 kloniert.

4. Verfahren gemäß Anspruch 1, dadurch gekennzeichnet, daß man zur Herstellung einer DNA-Sequenz, die für eine Verbindung der Formel I codiert, in der X₃ Phenylalanin bedeutet, in eine gemäß Ansprüchen 2 oder 3 erhaltene DNA-Sequenz solche Oligonucleotide kloniert, die an Position 96 der Formel I die Expression von Phenylalanin bewirken.

5. Verfahren gemäß Ansprüchen 1, 2 und 4, dadurch gekennzeichnet, daß man zur Herstellung einer DNA-Sequenz, die für eine Verbindung der Formel I codiert, in der X₁ und X₂ für Leucin und X₃ für Phenylalanin stehen und W, Y und Z die gleiche Bedeutung wie oben haben, in das Plasmid pALP 23 (erhältlich gemäß Fig. 14a) paarweise die Oligonucleotide M1 und M2, M3 und M4 oder M5 und M6 kloniert, das erhaltene Plasmid mit den Restriktionsendonucleasen Sac I und Spe I spaltet, anschließend in üblicher Weise dephosphoryliert und in das so erhaltene Fragment das Hybridisierungsprodukt der (phosphorylierten) Oligonucleotide E1, E2 und E3 der Formeln
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
und
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
ligiert.

6. Verfahren zur Synthese der in Figur 6 dargestellten, für das Serinprotease-Inhibitor-Protein ALP 237 codierenden DNA-Sequenz, dadurch gekennzeichnet, daß das durch Klonieren der Oligonucleotide M1 und M2 in das Plasmid pALP 23 (konstruiert gemäß Fig. 14a) erhaltene Plasmid pALP 231 mit den Restriktionsendonucleasen Sac I und Spe I gespalten und dann in üblicher Weise dephosphoryliert wird und man in das so erhaltene Fragment pALP 231/Sac I x Spe I das Hybridisierungsprodukt der (phosphorylierten) Oligonucleotide E1, E2 und E3 ligiert.

7. Verfahren zur Herstellung eines rekombinanten Vektors zur Expression einer Verbindung der Formeln I oder Ia, dadurch gekennzeichnet, daß eine nach den Ansprüchen 1 - 6 erhaltene DNA-Sequenz in ein Vektor-Molekül so eingesetzt wird, daß sie funktionell mit einer Expressions-Kontroll-Sequenz, vorzugsweise beginnend mit einem tac-Promoter und endend mit einer Terminatorsequenz, verbunden ist.

8. Verfahren zur Herstellung eines rekombinanten Vektors zur Expression einer Verbindung der Formeln I oder Ia, dadurch gekennzeichnet, daß der gemäß Anspruch 7 erhaltene Expressionsvektor in an sich bekannter Weise in Teilmengen mit unterschiedlichen Paaren von Restriktionsendonucleasen gespalten wird, eines der erhaltenen Fragmente gegebenenfalls dephosphoryliert wird und dann diejenigen Fragmente ligiert werden, die zu einem Expressionsvektor führen, der die für die Verbindung der Formel I bzw. Ia codierende DNA-Sequenz hinter dem tac-Promoter zweifach, getrennt durch zwei Stoppcodons, eine etwa 16 Nucleotide enthaltende Sequenz, eine Shine-Dalgarno-Sequenz und eine weitere Sequenz mit etwa 7 Nucleotiden, trägt.

9. Verfahren gemäß Anspruch 8 zur Herstellung eines rekombinanten Vektors zur Expression einer Verbindung der Formel I bzw. Ia, dadurch gekennzeichnet, daß eine Teilmenge des gemäß Anspruch 7 erhaltenen Expressionsvektors mit den Restriktionsendonucleasen Pst I und Spe I, die andere Teilmenge dieses Vektors mit den Restriktionsendonucleasen Pst I und Xba I gespalten wird und die dabei erhaltenen Fragmente, die die für die Verbindung der Formel I bzw. Ia codierende DNA-Sequenz enthalten, gegebenenfalls nach Dephosphorylierung, ligiert werden.

10. Verfahren zur Herstellung eines Serinprotease-Inhibitorproteins der Formel I oder eines Fusionsproteins davon, dadurch gekennzeichnet, daß ein Wirtsorganismus mit einem rekombinanten Vektor nach Ansprüchen 7 - 9 transformiert und dann in einem üblichen Nährmedium kultiviert wird, man gegebenenfalls die Expression des gewünschten Proteins induziert und dann das gebildete Expressionsprodukt aus der Kultur isoliert, einem Refolding-Verfahren durch Einwirkung reduzierender und darauffolgend oxydierender Mittel unterwirft und schließlich das erhaltene biologisch aktive Serinprotease-Inhibitorprotein der Formel I in an sich bekannter Weise isoliert und reinigt.

11. Verfahren gemäß Anspruch 10, dadurch gekennzeichnet, daß der Wirtsorganismus ein Bakterium, vorzugsweise ein Stamm der Art E.coli oder B.subtilis, ein mikroskopisch kleiner Pilz, vorzugsweise der Arten Aspergillus oryzae, Aspergillus niger oder Saccharomyces cerevisiae oder eine tierische oder menschliche Zelle ist.

12. Verfahren gemäß Ansprüchen 10 und 11, dadurch gekennzeichnet, daß der Wirtsorganismus ein Stamm der Art E.coli, insbesondere der Untergruppe K12 ist.

13. Verfahren gemäß Ansprüchen 10 bis 12, dadurch gekennzeichnet, daß der Wirtsorganismus aus der die folgenden Stämme umfassenden Gruppe ausgewählt ist: E.coli K12 JM 101 (ATCC 33876), E.coli K12 JM 103 (ATCC 39403), E.coli K12 JM 105 (DSM 4162) und E.coli K12 DH1 (ATCC 33849).

14. Verfahren gemäß Anspruch 10 zur Herstellung eines Serinprotease-Inhibitorproteins umfassend Teilbereiche der Aminosäuresequenzen nach Formeln I oder Ia, dadurch gekennzeichnet, daß man das beim Vorgehen nach Anspruch 10 gebildete Expressionsprodukt aus der Kultur isoliert und dieses dann einer kontrollierten sauren Hydrolyse zur Spaltung der Asparaginsäure-Prolin-Bindung in den Positionen 49 - 50 und/oder einer kontrollierten enzymatischen Abspaltung aller oder nur einer bis fünf der Aminosäuren von Positionen 107 - 102 unterwirft und das so erhaltene Hydrolyseprodukt aus dem Hydrolysat gewinnt.

15. Verfahren gemäß Anspruch 14, dadurch gekennzeichnet, daß man aus dem Serinprotease-Inhibitorprotein ALP 231 gemäß Figur 3 durch saure Hydrolyse, vorzugsweise mit 70 %iger Ameisensäure, die Aminosäuren der Positionen 1 - 49 abtrennt und so das neue Serinprotease-Inhibitorprotein mit der Aminosäuresequenz 
50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107
 erhält.

16. Verfahren zur Herstellung eines Serinprotease-inhibitorproteins umfassend zumindest die Teilbereiche der Positionen 53 - 101 der Aminosäuresequenzen nach Formeln I oder Ia, in denen X₁ und X₂ für Leucin und X₃ für Leucin oder Phenylalanin stehen, dadurch gekennzeichnet, daß man an die für das Inhibitorprotein codierende DNA-Sequenz 5'-terminal das Codon ATG anfügt und in dem bei der Expression gebildeten, vor dem N-Terminus des herzustellenden Proteins die zusätzliche Aminosäure Methionin aufweisenden Produkt dann in üblicher Weise, vorzugsweise mit Brom-cyan, die Bindung zwischen dem Methionin und der N-terminalen Aminosäure des gewünschten Inhibitorproteins spaltet.

17. Verfahren zur Herstellung eines Arzneimittels, das ein Serinprotease-Inhibitorprotein der Formel I oder Ia oder eines zumindest die Sequenz der Positionen 53 - 101 umfassenden Teilbereiches davon als Wirkstoff enthält, dadurch gekennzeichnet, daß der Wirkstoff in an sich bekannter Weise mit üblichen Trägerstoffen und/oder Verdünnungsmitteln und/oder Hilfsstoffen zu einer parenteral oder lokal anwendbaren Arzneimittelform verarbeitet wird.

## Patentansprüche (Patentansprüche für folgende(n) Vertragsstaat(en): GR)

1. Proteine der Formel (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 oder Teilbereichen davon, welche zumindest die Sequenz der Positionen 53 - 101 umfassen,
worin W für Tyrosin oder Glutaminsäure steht, X₁ und X₂ gleich oder verschieden sind und jeder dieser Reste Methionin oder Leucin bedeutet, X₃ Methionin, Leucin oder Phenylalanin darstellt, Y für Prolin oder Arginin steht und Z Leucin oder, wenn W für Tyrosin und X₁, X₂ und X₃ für Leucin stehen, auch Isoleucin bedeutet,
und Fusionsproteine dieser neuen Proteine.

2. Proteine der Formel I oder ein zumindest die Sequenz der Positionen 53 - 101 umfassender Teilbereich davon, worin W Tyrosin bedeutet.

3. Proteine gemäß Ansprüchen 1 und 2 der Formel (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2--Gly--X'3--Cys-Gly-Lys-Ser-Cys-Val-Ser-Pro-Val-Lys-Ala
 oder Teilbereichen davon, welche zumindest die Sequenz der Positionen 53 - 101 umfassen,
worin X₁, X₂, Y und Z die gleiche Bedeutung wie in Anspruch 1 haben und X₃' für Methionin oder Leucin steht,
und Fusionsproteine dieser neuen Proteine.

4. Proteine gemäß Ansprüchen 1 bis 3, die Teilbereiche der Formeln I bzw. Ia darstellen und die Sequenzbereiche der Positionen 53 - 101 bis die der Positionen 48 - 107 umfassen.

5. Proteine gemäß Ansprüchen 1 bis 3, welche die Aminosäurepositionen 1 bis 107 oder die Teilsequenzen 48 - 107, 49 - 107 oder 50 - 107 aus Formeln I oder Ia umfassen.

6. Proteine gemäß Ansprüchen 1, 4 und 5 der Formel 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 worin W, X₁, X₂, X₃, Y und Z die gleiche Bedeutung wie in Anspruch 1 haben.

7. Proteine gemäß Ansprüchen 1 - 6, in denen X₁, X₂, X₃ und Z für Leucin stehen.

8. Protein mit der Aminosäuresequenz gemäß Figur 3.

9. Protein mit der Aminosäuresequenz gemäß Figur 4.

10. Protein mit der Aminosäuresequenz gemäß Figur 5.

11. Protein mit der Aminosäuresequenz gemäß Figur 6.

12. Protein mit der Aminosäuresequenz gemäß Figur 7.

13. Protein mit der Aminosäuresequenz gemäß Figur 8.

14. Protein mit der Aminosäuresequenz

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. DNA-Sequenzen, dadurch gekennzeichnet, daß sie für Proteine gemäß Ansprüchen 1 - 14 codieren.

16. DNA-Sequenz gemäß Anspruch 15, dadurch gekennzeichnet, daß sie eine der in Figuren 2a, 2b oder 3 bis 8 dargestellten Nucleotidsequenzen aufweist.

17. Rekombinanter Vektor, dadurch gekennzeichnet, daß er eine codierende DNA-Sequenz nach einem der Ansprüche 15 oder 16 aufweist.

18. Rekombinanter Vektor gemäß Anspruch 17, dadurch gekennzeichnet, daß er ein Derivat des Pladmids ptacSDT DSM 5018) ist.

19. Als rekombinante Vektoren gemäß Ansprüchen 17 und 18 die Plasmide pALP 23, erhältlich gemäß Fig. 14a, und pALP 24, erhältlich gemäß Fig. 14b.

20. Als rekombinante Vektoren gemäß Ansprüchen 17 bis 19 die Plasmide pALP 231, erhältlich aus pALP 23 (Fig. 14a) durch Spaltung und Dephosphorylierung von pALP 23 mit den Restriktionsendonukleasen BstE II und Stu I und Klonierung der Oligonukleotide M1 und M2 in das Fragment pALP 23/Bst E II x Stu I, pALP 232, erhältlich aus pALP 23 (Fig. 14a) durch Spaltung und Dephosphorylierung von pALP 23 mit den Restriktionsendonukleasen BstE II und Stu I und Klonierung der Oligonukleotide M3 und M4 in das Fragment pALP 23/Bst E II x Stu I, pALP 236, erhältlich aus pALP 23 (Fig. 14a) durch Spaltung und Dephosphorylierung von pALP 23 mit den Restriktionsendonukleasen BstE II und Stu I und Klonierung der Oligonukleotide M5 und M6 in das Fragment pALP 23/Bst E II x Stu I, pALP 237, erhältlich aus pALP 231 durch Spaltung und Dephosphorylierung von pALP 231 mit den Restriktionsendonukleasen Sac I und Spe I und Klonierung der Oligonukleotide E1, E2 und E3 in das Fragment pALP 231/Sac I x Spe I, pALP 242, erhältlich aus pALP 24 (Fig. 14b) durch Spaltung und Dephosphorylierung von pALP 24 mit den Restriktionsnukleasen BstE II und Stu I und Klonierung der Oligonukleotide M1 und M2 in das Fragment pALP 24/Bst E II x Stu I und pALP 246, erhältlich aus pALP 24 (Fig. 14b) durch Spaltung und Dephosphorylierung von pALP 24 mit den Restriktionsendonukleasen BstE II und Stu I und Klonierung der Oligonukleotide M5 und M6 in das Fragment pALP 24/BstE II x Stu I.

21. Als rekombinante Vektoren gemäß Ansprüchen 17 bis 20 die Plasmide pALP 231, pALP 232 und pALP 242.

22. Rekombinanter Vektor gemäß Anspruch 17 oder 18, dadurch gekennzeichnet, daß er eine codierende DNA-Sequenz nach einem der Ansprüche 15 oder 16 zweifach aufweist.

23. Rekombinanter Vektor gemäß Anspruch 22, dadurch gekennzeichnet, daß die beiden codierenden DNA-Sequenzen (nach einem der Ansprüche 15 oder 16) jeweils 5'-terminal die gleiche Shine-Dalgarno-Sequenz im Abstand von etwa 7 Nucleotiden tragen, wobei nach den beiden Stop-Codons der ersten codierenden DNA-Sequenz im Abstand von etwa 16 Nucleotiden die zweite Shine-Dalgarno-Sequenz folgt, der sich im Abstand von etwa 7 Nucleotiden die zweite codierende DNA-Sequenz anschließt.

24. Als rekombinante Vektoren gemäß Ansprüchen 17 bis 23 die Plasmide pALP 2302 gemäß Fig. 15 mit 2 ALP 230-Genen mit der in Fig. 2a angegebenen Sequenz anstelle von 2 ALP 231-Genen, pALP 2312 gemäß Fig. 15, erhältlich aus pALP 23 (Fig. 14a), pALP 2322 gemäß Fig. 15 mit 2 ALP 232-Genen anstelle von 2 ALP 231-Genen, pALP 2362 gemäß Fig. 15 mit 2 ALP 236-Genen anstelle von 2 ALP 231-Genen, pALP 2372 gemäß Fig. 15 mit 2 ALP 237-Genen anstelle von 2 ALP 231-Genen, pALP 2402 gemäß Fig. 15 mit 2 ALP 240-Genen mit der Sequenz gemäß Fig. 2b anstelle von 2 ALP 231-Genen, pALP 2422 gemäß Fig. 15 mit 2 ALP 242-Genen anstelle von 2 ALP 231-Genen und pALP 2462 gemäß Fig. 15 mit 2 ALP 246-Genen anstelle von 2 ALP 231-Genen.

25. Als rekombinante Vektoren gemäß Ansprüchen 17 bis 24 die Plasmide pALP 2302, pALP 2312, pALP 2322, pALP 2402 und pALP 2422.

26. Als rekombinanter Vektor gemäß Ansprüchen 17 bis 25 das Plasmid pALP 2312 entsprechend Figur 15, erhältlich aus pALP 23 (Fig. 14a).

27. Wirtsorganismus, dadurch gekennzeichnet, daß er mit einem der rekombinanten Vektoren nach Ansprüchen 17 bis 26 transformiert ist.

28. Wirtsorganismus gemäß Anspruch 27, dadurch gekennzeichnet, daß er ein Bakterium, vorzugsweise ein Stamm der Art E.coli oder B.subtilis, ein mikroskopisch kleiner Pilz, vorzugsweise der Arten Aspergillus oryzae, Aspergillus niger oder Saccharomyces cerevisiae oder eine tierische oder menschliche Zelle ist.

29. Wirtsorganismus gemäß Ansprüchen 27 und 28, dadurch gekennzeichnet, daß er ein Stamm der Art E.coli, insbesondere der Untergruppe K12 ist.

30. Wirtsorganismus gemäß Ansprüchen 27 bis 29, der aus der die folgenden Stämme umfassenden Gruppe ausgewählt ist: E.coli K12 JM 101 (ATCC 33876), E.coli K12 JM 103 (ATCC 39403), E.coli K12 JM 105 (DSM 4162) und E.coli K12 DH1 (ATCC 33849).

31. Verfahren zur Synthese der DNA-Sequenzen gemäß Ansprüchen 15 und 16, dadurch gekennzeichnet, daß man ausgehend von dem Plasmid ptacSDT (DSM 5018) in an sich bekannter Weise unter Benutzung der Oligonucleotide D21 bis D23a gemäß Figur 1b und in einer weiteren Stufe unter Benutzung der Oligonucleotide D11 bis D13a gemäß Figur 1a das Plasmid pALP23 gemäß Fig. 14a, enthaltend die in Figur 2a dargestellte DNA-Sequenz, konstruiert, dieses gegebenenfalls unter Mitverwendung der Oligonucleotide D24 - D25a gemäß Figur 1c zur Konstruktion des Plasmids pALP 24 gemäß Fig. 14b, enthaltend die in Figur 2b dargestellte DNA-Sequenz, einsetzt, gegebenenfalls in die so erhaltenen Plasmide pALP 23 oder pALP 24 dann paarweise solche Oligonucleotide kloniert, daß die resultierenden Plasmide für Proteine der Formel I, in denen X₃ Methionin oder Leucin ist und W, X₁, X₂, Y und Z die gleiche Bedeutung wie oben haben, codierende DNA-Sequenzen enthalten und diese gegebenenfalls mit Hilfe weiterer Oligonucleotide so modifiziert, daß sie für ein Protein der Formel I, in dem X₃ Phenylalanin ist und W, X₁, X₂, Y und Z die gleiche Bedeutung wie oben haben, codierende DNA-Sequenzen aufweisen.

32. Verfahren gemäß Anspruch 31, dadurch gekennzeichnet, daß man in das Plasmid pALP 23 (erhältlich gemäß Fig. 14a) oder in das Plasmid pALP 24 (erhältlich gemäß Fig. 14b) paarweise die Oligonucleotide M1 und M2, M3 und M4 oder M5 und M6 der Formeln
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
und
3' - GCTT CCA GTC ACG GAC GAC GAC TTG GGC - 5' (M6)
kloniert und so DNA-Sequenzen herstellt, die für Proteine der Formel I codieren, in denen X₃ für Methionin oder Leucin steht und W, X₁, X₂, Y und Z die gleiche Bedeutung wie oben haben.

33. Verfahren gemäß Ansprüchen 31 und 32, dadurch gekennzeichnet, daß man zur Synthese einer DNA-Sequenz, die für ein Protein der Formel I codiert, worin X₃ Phenylalanin bedeutet, in eine gemäß Anspruch 32 erhaltene DNA-Sequenz solche Oligonucleotide kloniert, die an Position 96 der Formel I die Expression von Phenylalanin bewirken.

34. Verfahren gemäß Ansprüchen 31 bis 33, dadurch gekennzeichnet, daß man zur Herstellung einer DNA-Sequenz, die für ein Protein der Formel I codiert, in der X₁ und X₂ für Leucin und X₃ für Phenylalanin stehen und W, Y und Z die gleiche Bedeutung wie oben haben, in das Plasmid pALP 23 (erhältlich gemäß Fig. 14a) paarweise die Oligonucleotide M1 und M2, M3 und M4 oder M5 und M6 kloniert, das erhaltene Plasmid mit den Restriktionsendonucleasen Sac I und Spe I spaltet, anschließend in üblicher Weise dephosphoryliert und in das so erhaltene Fragment das Hybridisierungsprodukt der (phosphorylierten) Oligonucleotide E1, E2 und E3 der Formeln
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
und
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
ligiert.

35. Verfahren zur Synthese der in Figur 6 dargestellten, für das Protein ALP 237 codierenden DNA-Sequenz, dadurch gekennzeichnet, daß das durch Klonieren der Oligonucleotide M1 und M2 in das Plasmid pALP 23 (konstruiert gemäß Fig. 14a) erhaltene Plasmid pALP 231 mit den Restriktionsendonucleasen Sac I und Spe I gespalten und dann in üblicher Weise dephosphoryliert wird und man in das so erhaltene Fragment pALP 231/Sac I x Spe I das Hybridisierungsprodukt der (phosphorylierten) Oligonucleotide E1, E2 und E3 ligiert.

36. Verfahren zur Herstellung eines rekombinanten Vektors zur Expression eines Proteins der Formeln I oder Ia, dadurch gekennzeichnet, daß eine nach den Ansprüchen 31 - 35 erhaltene DNA-Sequenz in ein Vektor-Molekül so eingesetzt wird, daß sie funktionell mit einer Expressions-Kontroll-Sequenz, vorzugsweise beginnend mit einem tac-Promoter und endend mit einer Terminatorsequenz, verbunden ist.

37. Verfahren zur Herstellung eines rekombinanten Vektors zur Expression eines Proteins der Formeln I oder Ia, dadurch gekennzeichnet, daß der gemäß Anspruch 36 erhaltene Expressionsvektor in an sich bekannter Weise in Teilmengen mit unterschiedlichen Paaren von Restriktionsendonucleasen gespalten wird, eines der erhaltenen Fragmente gegebenenfalls dephosphoryliert wird und dann diejenigen Fragmente ligiert werden, die zu einem Expressionsvektor führen, der die für das Protein der Formel I bzw. Ia codierende DNA-Sequenz hinter dem tac-Promoter zweifach, getrennt durch zwei Stopp-codons, eine etwa 16 Nucleotide enthaltende Sequenz und eine Shine-Dalgarno-Sequenz und eine weitere Sequenz mit etwa 7 Nucleotiden, trägt.

38. Verfahren gemäß Anspruch 37 zur Herstellung eines rekombinanten Vektors zur Expression eines Proteins der Formel I bzw. Ia, dadurch gekennzeichnet, daß eine Teilmenge des gemäß Anspruch 36 erhaltenen Expressionsvektors mit den Restriktionsendonucleasen Pst I und Spe I die andere Teilmenge dieses Vektors mit den Restriktionsendonucleasen Pst I und Xba I gespalten wird und die dabei erhaltenen Fragmente, die die für das Protein der Formel I bzw. Ia codierende DNA-Sequenz enthalten, gegebenenfalls nach Dephosphorylierung, ligiert werden.

39. Verfahren zur Herstellung eines Proteins gemäß Ansprüchen 1 bis 14, dadurch gekennzeichnet, daß man einen Wirtsorganismus nach einem der Ansprüche 27 bis 30 in einem üblichen Nährmedium züchtet, gegebenenfalls die Expression des gewünschten Proteins induziert und dann das Expressionsprodukt aus der Kultur isoliert, einem Refolding-Verfahren durch Einwirkung reduzierender und darauffolgend oxidierender Mittel unterwirft und schließlich das erhaltene biologisch aktive Protein der Formel I in an sich bekannter Weise isoliert und reinigt.

40. Verfahren zur Herstellung eines Proteins Gemäß Ansprüchen 1 bis 7 und 14, umfassend Teilbereiche der Aminosäuresequenzen nach Formeln I oder Ia, dadurch gekennzeichnet, daß man das beim Vorgehen nach Anspruch 39 gebildete Expressionsprodukt aus der Kultur isoliert und dann einer kontrollierten sauren Hydrolyse zur Spaltung der Asparaginsäure-Prolin-Bindung in den Positionen 49 - 50 und/ oder einer kontrollierten enzymatischen Abspaltung aller oder nur einer bis fünf der Aminosäuren von Positionen 107 - 102 unterwirft und das so erhaltene Hydrolyseprodukt aus dem Hydrolysat gewinnt.

41. Verfahren zur Herstellung eines Proteins gemäß Ansprüchen 1 bis 5, umfassend zumindest die Teilbereiche der Positionen 53 - 101 der Aminosäuresequenzen nach Formeln I oder Ia, in denen X₁ und X₂ für Leucin und X₃ für Leucin oder Phenylalanin stehen, dadurch gekennzeichnet, daß man an die für das gewünschte Protein codierende DNA-Sequenz 5'-terminal das Codon ATG anfügt und in dem bei der Expression gebildeten, vor dem N-Terminus des herzustellenden Proteins die zusätzliche Aminosäure Methionin aufweisenden Produkt dann in üblicher Weise, vorzugsweise mit Brom-cyan, die Bindung zwischen dem Methionin und der ersten (N-terminalen) Aminosäure des gewünschten Proteins spaltet.

42. Verfahren zur Herstellung eines Arzneimittels, das ein Protein der Formeln I oder Ia oder eines zumindest die Sequenz der Positionen 53 - 101 umfassenden Teilbereiches davon als Wirkstoff enthält, dadurch gekennzeichnet, daß der Wirkstoff in an sich bekannter Weise mit üblichen Trägerstoffen und/oder Verdünnungsmitteln und/oder Hilfsstoffen zu einer parenteral oder lokal anwendbaren Arzneimittelform verarbeitet wird.

## Claims (Claims for the following Contracting State(s): AT, BE, CH, DE, FR, GB, IT, LI, LU, NL, SE)

1. Serine protease inhibitor proteins of formula (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 or partial regions thereof which comprise at least the sequence of positions 53 - 101,
wherein W represents tyrosine or glutamate, X₁ and X₂ are the same or different and each denotes methionine or leucine, X₃ constitutes methionine, leincine or phenylalanine, Y represents proline or arginine, and Z denotes leucine or, if W represents tyrosine and X₁, X₂ and X₃ represent leucine, also denotes isoleucine,
and fusion proteins of these new serine protease inhibitor proteins.

2. Serine protease inhibitor proteins of formula I or a partial region thereof which comprises at least the sequence of positions 53 - 101, wherein W represents tyrosine.

3. Serine protease inhibitor proteins according to claims 1 or 2 of formula (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2--Gly--X3′--Cys-Gly-Lys-Ser-Cys-Val-Ser-Pro-Val-Lys-Ala
 or partial regions thereof which comprise at least the sequence of positions 53 - 101,
wherein X₁, X₂, Y and Z have the same meaning as in claim 1 and X₃' represents methionine or leucine,
and fusion proteins of these new serine protease inhibitor proteins.

4. Serine protease inhibitor proteins according to claims 1 to 3, which constitute partial regions of formulae I or Ia and comprise the sequence regions of positions 53 - 101 to those of positions 48 - 107.

5. Serine protease inhibitor proteins according to claims 1 to 3, which comprise amino acid positions 1 to 107 or the partial sequences 48 - 107, 49 - 107 or 50 - 107 from formulae I or Ia.

6. Serine protease inhibitor proteins according to claims 1, 4 and 5 of formula 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 wherein W, X₁, X₂, X₃ and Z have the same meaning as in claim 1.

7. Serine protease inhibitor proteins according to claims 1 - 6, in which X₁, X₂, X₃ and Z represent leucine.

8. A serine protease inhibitor protein having the amino acid sequence according to Figure 3.

9. A serine protease inhibitor protein having the amino acid sequence according to Figure 4.

10. A serine protease inhibitor protein having the amino acid sequence according to Figure 5.

11. A serine protease inhibitor protein having the amino acid sequence according to Figure 6.

12. A serine protease inhibitor protein having the amino acid sequence according to Figure 7.

13. A serine protease inhibitor protein having the amino acid sequence according to Figure 8.

14. A serine protease inhibitor protein having the amino acid sequence

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. DNA sequences, characterised in that they code for serine protease inhibitor proteins according to claims 1 - 14.

16. A DNA sequence according to claim 15, characterised in that it comprises one of the nucleotide sequences illustrated in Figures 2a, 2b, or 3 to 8.

17. A recombinant vector, characterised in that comprises a coding DNA sequence according to either one of claims 15 or 16.

18. A recombinant vector according to claim 17, characterised in that it is a derivative of plasmid ptacSDT (DSM 5018).

19. Plasmids pALP 23, obtainable according to Figure 14a, and pALP 24, obtainable according to Figure 14b, as recombinant vectors according to claims 17 and 18.

20. Plasmids pALP 231 as recombinant vectors according to claims 17 to 19, which plasmids are obtainable from pALP 23 (Figure 14a) by the cleavage and dephosphorylation of pALP 23 with restriction endonucleases BstE II and Stu I and cloning the oligonucleotides M1 and M2 into the fragment pALP 23/Bst E II x Stu I, pALP 232, which is obtainable from pALP 23 (Figure 14a) by the cleavage and dephosphorylation of pALP 23 with restriction endonucleases BstE II and Stu I and cloning the oligonucleotides M3 and M4 into the fragment pALP 23/Bst E II x Stu I, pALP 236, which is obtainable from pALP 23 (Figure 14a) by the cleavage and dephosphorylation of pALP 23 with restriction endonucleases BstE II and Stu I and cloning the oligonucleotides M5 and M6 into the fragment pALP 23/Bst E II x Stu I, pALP 237, which is obtainable from pALP 231 by the cleavage and dephosphorylation of pALP 231 with restriction endonucleases Sac I and Spe I and cloning the oligonucleotides E1, E2 and E3 into the fragment pALP 231/Sac I x Spe I, pALP 242, which is obtainable from pALP 24 (Figure 14b) by the cleavage and dephosphorylation of pALP 24 with restriction nucleases BstE II and Stu I and cloning the oligonucleotides M1 and M2 into the fragment pALP 24/Bst E II x Stu I and pALP 246, which is obtainable from pALP 24 (Figure 14b) by the cleavage and dephosphorylation of pALP 24 with restriction endonucleases BstE II and Stu I and cloning the oligonucleotides M5 and M6 into the fragment pALP 24/BstE II x Stu I.

21. Plasmids pALP 231, pALP 232 and pALP 242 as recombinant vectors according to claims 17 to 20.

22. A recombinant vector according to claim 17 or 18, characterised in that in comprises a coding DNA sequence, according to either one of claims 15 or 16, twice.

23. A recombinant vector according to claim 22, characterised in that the two coding DNA sequences (according to either one of claims 15 or 16) comprise, at the 5' end in each case, the same Shine-Dalgarno sequence at a spacing of about 7 nucleotides, wherein, after the two stop codons of the first coding DNA sequence, there follows, at a spacing of about 16 nucleotides, the second Shine-Dalgarno sequence, which is followed by the second coding DNA sequence at a spacing of about 7 nucleotides.

24. The following plasmids as recombinant vectors according to claims 17 to 23: plasmids pALP 2302 according to Figure 15 and having 2 ALP 230 genes with the sequence given in Figure 2a instead of 2 ALP 231 genes, pALP 2312 according to Figure 15, which is obtainable from pALP 23 (Figure 14a), pALP 2322 according to Figure 15 having 2 ALP 232 genes instead of 2 ALP 231 genes, pALP 2362 according to Figure 15 having 2 ALP 236 genes instead of 2 ALP 231 genes, pALP 2372 according to Figure 15 having 2 ALP 237 genes instead of 2 ALP 231 genes, pALP 2402 according to Figure 15 having 2 ALP 240 genes with the sequence according to Figure 2b instead of 2 ALP 231 genes, pALP 2422 according to Figure 15 having 2 ALP 242 genes instead of 2 ALP 231 genes, and pALP 2462 according to Figure 15 having 2 ALP 246 genes instead of 2 ALP 231 genes.

25. Plasmids pALP 2302, pALP 2312, pALP 2322, pALP 2402 and pALP 2422 as recombinant vectors according to claims 17 to 24.

26. Plasmid pALP 2312 according to Figure 15, which is obtainable from pALP 23 (Figure 14a), as a recombinant vector according to claims 17 to 25.

27. A host organism, characterised in that it is transformed with one of the recombinant vectors according to claims 17 to 26.

28. A host organism according to claim 27, characterised in that it is a bacterium, preferably a strain of the *E. coli* or *B.subtilis* type, a microscopically small fungus, preferably of the *Aspergillus oryzae*, *Aspergillus niger* or *Saccharomyces cerevisiae* types, or an animal or human cell.

29. A host organism according to claims 27 and 28, characterised in that it is a strain of the *E. coli* type, particularly of subgroup K12.

30. A host organism according to claims 27 to 29 which is selected from the group comprising the following strains: *E. coli* K12 JM 101 (ATCC 33876), *E. coli* K12 JM 103 (ATCC 39403), *E. coli* K12 JM 105 (DSM 4162) and *E. coli* K12 DH1 (ATCC 33849).

31. A method of synthesising the DNA sequences according to claims 15 or 16, characterised in that, starting from plasmid ptacSDT (DSM 5018) in the known manner, using oligonucleotides D21 to D23a according to Figure 1b, and using oligonucleotides D11 to D13a according to Figure 1a in a further step, plasmid pALP23, which contains the DNA sequence illustrated in Figure 2a, is constructed according to Figure 14a, this plasmid is used, optionally with the use in conjunction of oligonucleotides D24 - D25a according to Figure 1c, for the construction of plasmid pALP 24 according to Figure 14b, which contains the DNA sequence illustrated in Figure 2b, those oligonucleotides are then optionally cloned in pairs into plasmids pALP 23 or pALP 24 thus obtained such that the resulting plasmids contain DNA sequences coding for proteins of formula I, in which X₃ is methionine or leucine and W, X₁, X₂, Y and Z have the same meaning as above, and these are modified, optionally with the aid of further oligonucleotides, so that they comprise DNA sequences coding for a protein of formula I in which X₃ is phenylalanine and W, X₁, X₂, Y and Z have the same meaning as above.

32. A method according to claim 31, characterised in that oligonucleotides M1 or M2, M3 and M4 or M5 and M6 of formulae
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
and
3' - GCTT CCA GTC ACG GAC GAC GAC TTG GGC - 5' (M6)
are cloned in pairs into plasmid pALP 23 which is obtainable according to Figure 14a, or into plasmid pALP 24 which is obtainable according to Figure 14b, and DNA sequences are thus produced which code for compounds of formula I in which X₃ is methionine or leucine and W, X₁, X₂, Y and Z have the same meaning as above.

33. A method according to claims 31 or 32, characterised in that for the synthesis of a DNA sequence which codes for a serine protease inhibitor protein of formula I in which X₃ denotes phenylalanine, those oligonucleotides which effect the expression of phenylalanine at position 96 of formula I are cloned into a DNA sequence obtained according to claim 32.

34. A method according to claims 31 to 33, characterised in that for the production of a DNA sequence which codes for a compound of formula I in which X₁ and X₂ represent leucine and X₃ represents phenylalanine and W, Y and Z have the same meaning as above, oligonucleotides M1 and M2, M3 and M4 or M5 and M6 are cloned in pairs into plasmid pALP 23 which is obtainable according to Figure 14a, the plasmid obtained is cleaved with restriction endonucleases Sac I and Spe I, is subsequently dephosphorylated in the usual manner, and the hybridisation product of (phosphorylated) oligonucleotides E1, E2 and E3 of formulae
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
and
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
is ligated into the fragment thus obtained.

35. A method of synthesising the DNA sequence illustrated in Figure 6, which codes for serine protease inhibitor protein ALP 237, characterised in that plasmid pALP 231, which is obtained by cloning oligonucleotides M1 and M2 into plasmid pALP 23 constructed according to Figure 14a, is cleaved with restriction endonucleases Sac I and Spe I and is then dephosphorylated in the usual manner, and the hybridisation product of (phosphorylated) oligonucleotides E1, E2 and E3 is ligated into the fragment pALP 231/Sac I x Spe I thus obtained.

36. A method of producing a recombinant vector for the expression of a compound of formulae I or Ia, characterised in that a DNA sequence obtained according to claims 31 - 35 is inserted in a vector molecule so that it is functionally bound to an expression control sequence, preferably beginning with a tac promoter and ending with a terminator sequence.

37. A method of producing a recombinant vector for the expression of a compound of formulae I or Ia, characterised in that the expression vector obtained according to claim 36 is cleaved into portions in the known manner with different pairs of restriction nucleases, one of the fragments obtained is optionally dephosphorylated and then those fragments are ligated which result in an expression vector which comprises the DNA sequence coding for the compound of formula I or Ia twice behind the *tac* promoter and separated by two stop codons, a sequence containing about 16 nucleotides, a Shine-Dalgarno sequence and a further sequence with about 7 nucleotides.

38. A method according to claim 37 for producing a recombinant vector for the expression of a compound of formula I or Ia, characterised in that one portion of the recombinant vector obtained according to claim 36 is cleaved with restriction endonucleases Pst I and Spe I and the other portion of this vector is cleaved with restriction endonucleases Pst I and Xba I, and the fragments thereby obtained, which contain the DNA sequence coding for the compound of formula I or Ia, are ligated, optionally after dephosphorylation.

39. A method of producing a serine protease inhibitor protein according to claims 1 to 14, characterised in that a host organism according to any one of claims 27 to 30 is cultivated in a customary culture medium, expression of the desired protein is optionally induced, and the expression product is then isolated from the culture, is subjected to a refolding process by the effect of reducing agents followed by oxidising agents, and finally the biologically active serine protease inhibitor protein of formula I which is obtained is isolated and purified in a known manner.

40. A method of producing a serine protease inhibitor protein according to claims 1 - 7 and 14 and comprising partial regions of the amino acid sequences according to formulae I or Ia, characterised in that the expression product formed by the procedure according to claim 39 is isolated from the culture and is then subjected to a controlled acid hydrolysis in order to cleave the aspartate-proline bond in positions 49 - 50 and/or is subjected to a controlled enzymatic cleavage of all or of only one to five of the amino acids of positions 107 - 102, and the hydrolysis product which is thus obtained is extracted from the hydrolysate.

41. A method of producing a serine protease inhibitor protein according to claims 1-5 and comprising at least the partial regions of positions 53 - 101 of the amino acid sequences according to formulae I or Ia in which X₁ and X₂ represent leucine and X₃ represents leucine or phenylalanine, characterised in that the ATG codon is attached to the 5' end of the DNA sequence which is coding for the inhibitor protein, and that, in the product formed during the expression, which comprises the additional amino acid methionine in front of the N-terminus of the protein to be produced, the bond between the methionine and the first (N-terminal) amino acid of the desired inhibitor protein is then cleaved in the usual manner, preferably with bromo-cyan.

42. Drugs, characterised by a content of one of the serine protease inhibitor proteins according to claims 1 to 14 and customary carrier substances and/or diluents and/or auxiliary materials.

43. The use of a serine protease inhibitor protein according to any one of claims 1 to 14 for the production of a drug for the treatment and/or prophylaxis of coagulation disorders, the invasive growth of tumours, emphysemas, arthritis and nephritis, and particularly of excessive inflammation conditions in polytraumatic or septic shock or for pancreatitis and other symptoms which are accompanied by a disturbed equilibrium between serine proteases and serine protease inhibitors.

## Claims (Claims for the following Contracting State(s): ES)

1. A method for the synthesis of DNA sequences which code for new serine protease inhibitor proteins of formula (I) 
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 or partial regions thereof which comprise at least the sequence of positions 53 - 101,
wherein W represents tyrosine or glutamate, X₁ and X₂ are the same or different and each denotes methionine or leucine, X₃ constitutes methionine, leucine or phenylalanine, Y represents proline or arginine, and Z denotes leucine or, if W represents tyrosine and X₁, X₂ and X₃ represent leucine, also denotes isoleucine,
or for fusion proteins of these new serine protease inhibitor proteins, characterised in that, starting from plasmid ptacSDT (DSM 5018) in the known manner, using oligonucleotides D21 to D23a according to Figure 1b, and using oligonucleotides D11 to D13a according to Figure 1a in a further step, plasmid pALP23, which contains the DNA sequence illustrated in Figure 2a, is constructed according to Figure 14a, this plasmid is used, optionally with the use in conjunction of oligonucleotides D24 - D25a according to Figure 1c, for the construction of plasmid pALP 24 according to Figure 14b, which contains the DNA sequence illustrated in Figure 2b, those oligonucleotides are then optionally cloned in pairs into plasmids pALP 23 or pALP 24 thus obtained such that the resulting plasmids contain DNA sequences coding for proteins of formula I, in which X₃ is methionine or leucine and W, X₁, X₂, Y and Z have the same meaning as above, and these are modified, optionally with the aid of further oligonucleotides, so that they comprise DNA sequences coding for a protein of formula I in which X₃ is phenylalanine and W, X₁, X₂, Y and Z have the same meaning as above.

2. A method according to claim 1, characterised in that oligonucleotides M1 or M2, M3 and M4 or M5 and M6 of formulae
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
and
3' - GCTT CCA GTC ACG GAC GAC GAC TTG GGC - 5' (M6)
are cloned in pairs into plasmid pALP 23 which is obtainable according to Figure 14a, or into plasmid pALP 24 which is obtainable according to Figure 14b, and DNA sequences are thus produced which code for compounds of formula I in which X₃ is methionine or leucine and W, X₁, X₂, Y and Z have the same meaning as above.

3. A method according to claims 1 or 2, characterised in that for the synthesis of a DNA sequence which codes for a serine protease inhibitor protein of formula Ia: 
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3'--Cys-Gly-Lys-Ser-Cys-Val-Ser-Pro-Val-Lys-Ala
 or partial regions thereof which comprise at least the sequence of positions 53 - 101,
wherein X₁, X₂, Y and Z have the same meaning as in claim 1 and X₃' represents methionine or leucine,
oligonucleotides M1 and M2 or M3 and M4 are cloned in pairs into plasmid pALP 23 obtainable according to Figure 14a or into plasmid pALP 24 obtainable according to Figure 14b.

4. A method according to claim 1, characterised in that for the production of a DNA sequence which codes for a compound of formula I in which X₃ denotes phenylalanine, those oligonucleotides which effect the expression of phenylalanine at position 96 of formula I are cloned into a DNA sequence obtained according to claims 2 or 3.

5. A method according to claims 1, 2 and 4, characterised in that for the production of a DNA sequence which codes for a compound of formula I in which X₁ and X₂ represent leucine and X₃ represents phenylalanine and W, Y and Z have the same meaning as above, oligonucleotides M1 and M2, M3 and M4 or M5 and M6 are cloned in pairs into plasmid pALP 23 which is obtainable according to Figure 14a, the plasmid obtained is cleaved with restriction endonucleases Sac I and Spe I, is subsequently dephosphorylated in the usual manner, and the hybridisation product of (phosphorylated) oligonucleotides E1, E2 and E3 of formulae
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
and
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
is ligated into the fragment thus obtained.

6. A method of synthesising the DNA sequence illustrated in Figure 6, which is coding for serine protease inhibitor protein ALP 237, characterised in that plasmid pALP 231, which is obtained by cloning oligonucleotides M1 and M2 into plasmid pALP 23 constructed according to Figure 14a, is cleaved with restriction endonucleases Sac I and Spe I and is then dephosphorylated in the usual manner, and the hybridisation product of (phosphorylated) oligonucleotides E1, E2 and E3 is ligated into the fragment pALP 231/Sac I x Spe I thus obtained.

7. A method of producing a recombinant vector for the expression of a compound of formulae I or Ia, characterised in that a DNA sequence obtained according to claims 1 - 6 is inserted in a vector molecule so that it is functionally bound to an expression control sequence, preferably beginning with a *tac* promoter and ending with a terminator sequence.

8. A method of producing a recombinant vector for the expression of a compound of formulae I or Ia, characterised in that the expression vector obtained according to claim 7 is cleaved into portions in the known manner with different pairs of restriction nucleases, one of the fragments obtained is optionally dephosphorylated and then those fragments are ligated which result in an expression vector which comprises the DNA sequence coding for the compound of formula I or Ia twice behind the tac promoter and separated by two stop codons, a sequence containing about 16 nucleotides, a Shine-Dalgarno sequence and a further sequence with about 7 nucleotides.

9. A method according to claim 8 for producing a recombinant vector for the expression of a compound of formula I or Ia, characterised in that one portion of the recombinant vector obtained according to claim 7 is cleaved with restriction endonucleases Pst I and Spe I and the other portion of this vector is cleaved with restriction endonucleases Pst I and Xba I, and the fragments thereby obtained, which contain the DNA sequence coding for the compound of formula I or Ia, are ligated, optionally after dephosphorylation.

10. A method of producing a serine protease inhibitor protein of formula I or a fusion protein thereof, characterised in that a host organism is transformed with a recombinant vector according to claims 7 - 9 and is then cultivated in a customary culture medium, expression of the desired protein is optionally induced, and the expression product formed is then isolated from the culture, is subjected to a refolding process by the effect of reducing agents followed by oxidising agents, and finally the biologically active serine protease inhibitor protein of formula I which is obtained is isolated and purified in a known manner.

11. A method according to claim 10, characterised in that the host organism is a bacterium, preferably a strain of the *E. coli* or *B. subtilis* type, a microscopically small fungus, preferably of the *Aspergillus oryzae*, *Aspergillus niger* or *Saccharomyces cerevisiae* types, or an animal or human cell.

12. A method according to claims 10 or 11, characterised in that the host organism is a strain of the *E. coli* type, particularly of subgroup K12.

13. A method according to claims 10 to 12, characterised in that the host organism is selected from the group comprising the following strains: *E. coli* K12 JM 101 (ATCC 33876), *E. coli* K12 JM 103 (ATCC 39403), *E. coli* K12 JM 105 (DSM 4162) and *E. coli* K12 DH1 (ATCC 33849).

14. A method according to claim 10 for producing a serine protease inhibitor protein comprising partial regions of the amino acid sequences according to formulae I or Ia, characterised, in that the expression product formed by the procedure according to claim 10 is isolated from the culture and is then subjected to a controlled acid hydrolysis in order to cleave the aspartic acid-proline bond in positions 49 - 50 and/or is subjected to a controlled enzymatic cleavage of all or of only one to five of the amino acids of positions 107 - 102, and the hydrolysis product which is thus obtained is extracted from the hydrolysate.

15. A method according to claim 14, characterised in that the amino acids of positions 1 - 49 are separated from serine protease inhibitor protein ALP 231 according to Figure 3 by acid hydrolysis, preferably with 70 % formic acid, and the new serine protease inhibitor protein which contains the amino acid sequence 
50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-Gly-
Gln-Cys-Lys-Arg-Asp-Leu-Lys-Cys-Cys-Leu-Gly-Leu-Cys-Gly-Lys-Ser-Cys-
Val-Ser-Pro-Val-Lys-Ala
107
 is thus obtained.

16. A method of producing a serine protease inhibitor protein comprising at least the partial regions of positions 53 - 101 of the amino acid sequences according to formulae I or Ia in which X₁ and X₂ represent leucine and X₃ represents leucine or phenylalanine, characterised in that the ATG codon is attached to the 5' end of the DNA sequence which is coding for the inhibitor protein, and that, in the product formed during the expression, which comprises the additional amino acid methionine in front of the N-terminus of the protein to be produced, the bond between the methionine and the N-terminal amino acid of the desired inhibitor protein is then cleaved in the usual manner, preferably with bromo-cyan.

17. A method of producing a drug which contains, as an active ingredient, a serine protease inhibitor protein of formula I or Ia or a partial region thereof which comprises at least the sequence of positions 53 - 101, characterised in that the active ingredient is processed in a manner known in the art with customary carrier materials and/or diluents and/or auxiliary materials to produce a form of drug which can be applied parenterally or locally.

## Claims (Claims for the following Contracting State(s): GR)

1. Proteins of formula (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 or partial regions thereof which comprise at least the sequence of positions 53 - 101,
wherein W represents tyrosine or glutamate, X₁ and X₂ are the same or different and each denotes methionine or leucine, X₃ constitutes methionine, leucine or phenylalanine, Y represents proline or arginine, and Z denotes leucine or, if W represents tyrosine and X₁, X₂ and X₃ represent leucine, also denotes isoleucine,
and fusion proteins of these new proteins.

2. Proteins of formula I or a partial region thereof which comprises at least the sequence of positions 53 - 101, wherein W represents tyrosine.

3. Proteins according to claims 1 or 2 of formula (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2--Gly--X3'--Cys-Gly-Lys-Ser-Cys-Val-Ser-Pro-Val-Lys-Ala
 or partial regions thereof which comprise at least the sequence of positions 53 - 101,
wherein X₁, X₂, Y and Z have the same meaning as in claim 1 and X₃' represents methionine or leucine,
and fusion proteins of these new proteins.

4. Proteins according to claims 1 to 3, which constitute partial regions of formulae I or Ia and comprise the sequence regions of positions 53 - 101 to those of positions 48 - 107.

5. Proteins according to claims 1 to 3, which comprise amino acid positions 1 to 107 or the partial sequences 48 - 107, 49 - 107 or 50 - 107 from formulae I or Ia.

6. Proteins according to claims 1, 4 and 5 of formula 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 wherein W, X₁, X₂, X₃ and Z have the same meaning as in claim 1.

7. Proteins according to claims 1 - 6, in which X₁, X₂, X₃ and Z represent leucine.

8. A protein having the amino acid sequence according to Figure 3.

9. A protein having the amino acid sequence according to Figure 4.

10. A protein having the amino acid sequence according to Figure 5.

11. A protein having the amino acid sequence according to Figure 6.

12. A protein having the amino acid sequence according to Figure 7.

13. A protein having the amino acid sequence according to Figure 8.

14. A protein having the amino acid sequence

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. DNA sequences, characterised in that they code for proteins according to claims 1 - 14.

16. A DNA sequence according to claim 15, characterised in that it comprises one of the nucleotide sequences illustrated in Figures 2a, 2b, or 3 to 8.

17. A recombinant vector, characterised in that comprises a coding DNA sequence according to either one of claims 15 or 16.

18. A recombinant vector according to claim 17, characterised in that it is a derivative of plasmid ptacSDT (DSM 5018).

19. Plasmids pALP 23, obtainable according to Figure 14a, and pALP 24, obtainable according to Figure 14b, as recombinant vectors according to claims 17 and 18.

20. Plasmids pALP 231 as recombinant vectors according to claims 17 to 19, which plasmids are obtainable from pALP 23 (Figure 14a) by the cleavage and dephosphorylation of pALP 23 with restriction endonucleases BstE II and Stu I and cloning the oligonucleotides M1 and M2 into the fragment pALP 23/Bst E II x Stu I, pALP 232, which is obtainable from pALP 23 (Figure 14a) by the cleavage and dephosphorylation of pALP 23 with restriction endonucleases BstE II and Stu I and cloning the oligonucleotides M3 and M4 into the fragment pALP 23/Bst E II x Stu I, pALP 236, which is obtainable from pALP 23 (Figure 14a) by the cleavage and dephosphorylation of pALP 23 with restriction endonucleases BstE II and Stu I and cloning the oligonucleotides M5 and M6 into the fragment pALP 23/Bst E II x Stu I, pALP 237, which is obtainable from pALP 231 by the cleavage and dephosphorylation of pALP 231 with restriction endonucleases Sac I and Spe I and cloning the oligonucleotides E1, E2 and E3 into the fragment pALP 231/Sac I x Spe I, pALP 242, which is obtainable from pALP 24 (Figure 14b) by the cleavage and dephosphorylation of pALP 24 with restriction nucleases BstE II and Stu I and cloning the oligonucleotides M1 and M2 into the fragment pALP 24/Bst E II x Stu I and pALP 246, which is obtainable from pALP 24 (Figure 14b) by the cleavage and dephosphorylation of pALP 24 with restriction endonucleases BstE II and Stu I and cloning the oligonucleotides M5 and M6 into the fragment pALP 24/BstE II x Stu I.

21. Plasmids pALP 231, pALP 232 and pALP 242 as recombinant vectors according to claims 17 to 20.

22. A recombinant vector according to claim 17 or 18, characterised in that in comprises a coding DNA sequence, according to either one of claims 15 or 16, twice.

23. A recombinant vector according to claim 22, characterised in that the two coding DNA sequences (according to either one of claims 15 or 16) comprise, at the 5' end in each case, the same Shine-Dalgarno sequence at a spacing of about 7 nucleotides, wherein, after the two stop codons of the first coding DNA sequence, there follows, at a spacing of about 16 nucleotides, the second Shine-Dalgarno sequence, which is followed by the second coding DNA sequence at a spacing of about 7 nucleotides.

24. The following plasmids as recombinant vectors according to claims 17 to 23: plasmids pALP 2302 according to Figure 15 and having 2 ALP 230 genes with the sequence given in Figure 2a instead of 2 ALP 231 genes, pALP 2312 according to Figure 15, which is obtainable from pALP 23 (Figure 14a), pALP 2322 according to Figure 15 having 2 ALP 232 genes instead of 2 ALP 231 genes, pALP 2362 according to Figure 15 having 2 ALP 236 genes instead of 2 ALP 231 genes, pALP 2372 according to Figure 15 having 2 ALP 237 genes instead of 2 ALP 231 genes, pALP 2402 according to Figure 15 having 2 ALP 240 genes with the sequence according to Figure 2b instead of 2 ALP 231 genes, pALP 2422 according to Figure 15 having 2 ALP 242 genes instead of 2 ALP 231 genes, and pALP 2462 according to Figure 15 having 2 ALP 246 genes instead of 2 ALP 231 genes.

25. Plasmids pALP 2302, pALP 2312, pALP 2322, pALP 2402 and pALP 2422 as recombinant vectors according to claims 17 to 24.

26. Plasmid pALP 2312 according to Figure 15, which is obtainable from pALP 23 (Figure 14a), as a recombinant vector according to claims 17 to 25.

27. A host organism, characterised in that it is transformed with one of the recombinant vectors according to claims 17 to 26.

28. A host organism according to claim 27, characterised in that it is a bacterium, preferably a strain of the *E. coli* or *B.subtilis* type, a microscopically small fungus, preferably of the *Aspergillus oryzae*, *Aspergillus niger* or *Saccharomyces cerevisiae* types, or an animal or human cell.

29. A host organism according to claims 27 and 28, characterised in that it is a strain of the *E. coli* type, particularly of subgroup K12.

30. A host organism according to claims 27 to 29 which is selected from the group comprising the following strains: *E. coli* K12 JM 101 (ATCC 33876), *E. coli* K12 JM 103 (ATCC 39403), *E. coli* K12 JM 105 (DSM 4162) and *E. coli* K12 DH1 (ATCC 33849).

31. A method of synthesising the DNA sequences according to claims 15 or 16, characterised in that, starting from plasmid ptacSDT (DSM 5018) in the known manner, using oligonucleotides D21 to D23a according to Figure 1b, and using oligonucleotides D11 to D13a according to Figure 1a in a further step, plasmid pALP23, which contains the DNA sequence illustrated in Figure 2a, is constructed according to Figure 14a, this plasmid is used, optionally with the use in conjunction of oligonucleotides D24 - D25a according to Figure 1c, for the construction of plasmid pALP 24 according to Figure 14b, which contains the DNA sequence illustrated in Figure 2b, those oligonucleotides are then optionally cloned in pairs into plasmids pALP 23 or pALP 24 thus obtained such that the resulting plasmids contain DNA sequences coding for proteins of formula I, in which X₃ is methionine or leucine and W, X₁, X₂, Y and Z have the same meaning as above, and these are modified, optionally with the aid of further oligonucleotides, so that they comprise DNA sequences coding for a protein of formula I in which X₃ is phenylalanine and W, X₁, X₂, Y and Z have the same meaning as above.

32. A method according to claim 31, characterised in that oligonucleotides M1 or M2, M3 and M4 or M5 and M6 of formulae
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
and
3' - GCTT CCA GTC ACG GAC GAC GAC TTG GGC - 5' (M6)
are cloned in pairs into plasmid pALP 23 which is obtainable according to Figure 14a, or into plasmid pALP 24 which is obtainable according to Figure 14b, and DNA sequences are thus produced which code for compounds of formula I in which X₃ is methionine or leucine and W, X₁, X₂, Y and Z have the same meaning as above.

33. A method according to claims 31 or 32, characterised in that for the synthesis of a DNA sequence which codes for a protein of formula I in which X₃ denotes phenylalanine, those oligonucleotides which effect the expression of phenylalanine at position 96 of formula I are cloned into a DNA sequence obtained according to claim 32.

34. A method according to claims 31 to 33, characterised in that for the production of a DNA sequence which codes for a protein of formula I in which X₁ and X₂ represent leucine and X₃ represents phenylalanine and W, Y and Z have the same meaning as above, oligonucleotides M1 and M2, M3 and M4 or M5 and M6 are cloned in pairs into plasmid pALP 23 which is obtainable according to Figure 14a, the plasmid obtained is cleaved with restriction endonucleases Sac I and Spe I, is subsequently dephosphorylated in the usual manner, and the hybridisation product of (phosphorylated) oligonucleotides E1, E2 and E3 of formulae
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
and
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)
is ligated into the fragment thus obtained.

35. A method of synthesising the DNA sequence illustrated in Figure 6, which is coding for protein ALP 237, characterised in that plasmid pALP 231, which is obtained by cloning oligonucleotides M1 and M2 into plasmid pALP 23 constructed according to Figure 14a, is cleaved with restriction endonucleases Sac I and Spe I and is then dephosphorylated in the usual manner, and the hybridisation product of (phosphorylated) oligonucleotides E1, E2 and E3 is ligated into the fragment pALP 231/Sac I x Spe I thus obtained.

36. A method of producing a recombinant vector for the expression of a protein of formulae I or Ia, characterised in that a DNA sequence obtained according to claims 31 - 35 is inserted in a vector molecule so that it is functionally bound to an expression control sequence, preferably beginning with a *tac* promoter and ending with a terminator sequence.

37. A method of producing a recombinant vector for the expression of a protein of formulae I or Ia, characterised in that the expression vector obtained according to claim 36 is cleaved into portions in the known manner with different pairs of restriction nucleases, one of the fragments obtained is optionally dephosphorylated and then those fragments are ligated which result in an expression vector which comprises the DNA sequence coding for the protein of formula I or Ia twice behind the tac promoter and separated by two stop codons, a sequence containing about 16 nucleotides, a Shine-Dalgarno sequence and a further sequence with about 7 nucleotides.

38. A method according to claim 37 for producing a recombinant vector for the expression of a protein of formula I or Ia, characterised in that one portion of the recombinant vector obtained according to claim 36 is cleaved with restriction endonucleases Pst I and Spe I and the other portion of this vector is cleaved with restriction endonucleases Pst I and Xba I, and the fragments thereby obtained, which contain the DNA sequence coding for the protein of formula I or Ia, are ligated, optionally after dephosphorylation.

39. A method of producing a protein according to claims 1 to 14, characterised in that a host organism according to any one of claims 27 to 30 is cultivated in a customary culture medium, expression of the desired protein is optionally induced, and the expression product is then isolated from the culture, is subjected to a refolding process by the effect of reducing followed by oxidising agents, and finally the biologically active serine protease inhibitor protein of formula I which is obtained is isolated and purified in a known manner.

40. A method of producing a protein according to claims 1 - 7 and 14 and comprising partial regions of the amino acid sequences according to formulae I or Ia, characterised in that the expression product formed by the procedure according to claim 39 is isolated from the culture and is then subjected to a controlled acid hydrolysis in order to cleave the aspartic acid-proline bond in positions 49 - 50 and/or is subjected to a controlled enzymatic cleavage of all or of only one to five of the amino acids of positions 107 - 102, and the hydrolysis product which is thus obtained is extracted from the hydrolysate.

41. A method of producing a protein according to claims 1 - 15 and comprising at least the partial regions of positions 53 - 101 of the amino acid sequences according to formulae I or Ia in which X₁ and X₂ represent leucine and X₃ represents leucine or phenylalanine, characterised in that the ATG codon is attached to the 5' end of the DNA sequence which is coding for the inhibitor protern, and that, in the product formed during the expression, which comprises the additional amino acid methionine in front of the N-terminus of the protein to be produced, the bond between the methionine and the first (N-terminal) amino acid of the desired protein is then cleaved in the usual manner, preferably with bromo-cyan.

42. A method of producing a drug which contains, as an active ingredient, a protein of formula I or Ia or a partial region thereof which comprises at least the sequence of positions 53 - 101, characterised in that the active ingredient is processed in a manner known in the art with customary carrier materials and/or diluents and/or auxiliary materials to produce a form of drug which can be applied parenterally or locally.

## Revendications (Revendications pour l'(les) Etat(s) contractant(s) suivant(s): AT, BE, CH, DE, FR, GB, IT, LI, LU, NL, SE)

1. Protéines inhibitrices de sérine-protéases de formule (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 ou leurs domaines partiels, qui comprennent au moins la séquence des positions 53-101,
où W représente la tyrosine ou l'acide glutamique, X₁ et X₂ sont identiques ou différents et chacun de ces restes désigne la méthionine ou la leucine, X₃ est la méthionine, la leucine ou la phénylalanine, Y est la proline ou l'arginine et Z est la leucine, ou désigne également l'isoleucine lorsque W est la tyrosine et X₁, X₂ et X₃ sont la leucine,
et protéines fusionnées de ces nouvelles protéines inhibitrices de sérine-protéases.

2. Protéines inhibitrices de sérine-protéases de formule I ou domaine partiel de ces protéines comprenant au moins la séquence des positions 53-101, où W désigne la tyrosine.

3. Protéines inhibitrices de sérine-protéases suivant les revendications 1 et 2 de formule Ia 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2--Gly--X'3--Cys-Gly-Lys-Ser-Cys-Val-Ser-Pro-Val-Lys-Ala
 ou domaines partiels de ces protéines, qui comprennent au moins la séquence des positions 53-101,
où X₁, X₂, Y et Z ont la même définition que dans la revendication 1 et X_{'3} est la méthionine ou la leucine,
et protéines fusionnées de ces nouvelles protéines inhibitrices de sérine-protéases.

4. Protéines inhibitrices de sérine-protéases suivant les revendications 1 à 3, qui représentent des domaines partiels des formules I et Ia et qui comprennent les régions de séquence des positions 53-101 jusqu'à celles des positions 48-107.

5. Protéines inhibitrices de sérine-protéases suivant les revendications 1 à 3, qui comprennent les positions d'amino-acides 1 à 107 ou les séquences partielles 48-107, 49-107 ou 50-107 des formules I ou Ia.

6. Protéines inhibitrices de sérine-protéases suivant les revendications 1, 4 et 5, de formule 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 où W, X₁, X₂, X₃, Y et Z ont la même définition que dans la revendication 1.

7. Protéines inhibitrices de sérine-protéases suivant les revendications 1 à 6, dans lesquelles X₁, X₂, X₃ et Z représentent la leucine.

8. Protéine inhibitrice de sérine-protéase ayant la séquence d'amino-acides suivant la figure 3.

9. Protéine inhibitrice de sérine-protéase ayant la séquence d'amino-acides suivant la figure 4.

10. Protéine inhibitrice de sérine-protéase ayant la séquence d'amino-acides suivant la figure 5.

11. Protéine inhibitrice de sérine-protéase ayant la séquence d'amino-acides suivant la figure 6.

12. Protéine inhibitrice de sérine-protéase ayant la séquence d'amino-acides suivant la figure 7.

13. Protéine inhibitrice de sérine-protéase ayant la séquence d'amino-acides suivant la figure 8.

14. Protéine inhibitrice de sérine-protéase ayant la séquence d'amino-acides

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. Séquences d'ADN, caractérisées en ce qu'elles codent pour les protéines inhibitrices de sérine-protéase suivant les revendications 1 à 14.

16. Séquence d'ADN suivant la revendication 15, caractérisée en ce qu'elle présente l'une des séquences de nucléotides représentées sur les figures 2a, 2b ou 3 à 8.

17. Vecteur recombinant, caractérisé en ce qu'il présente une séquence d'ADN codante selon l'une des revendications 15 ou 16.

18. Vecteur recombinant suivant la revendication 17, caractérisé en ce qu'il est un dérivé du plasmide ptacSDT (DMS 5018).

19. A titre de vecteurs recombinants suivant les revendications 17 et 18, le plasmide pALP 23 obtenu conformément à là figure 14a et le plasmide pALP 24 obtenu conformément à la figure 14b.

20. A titre de vecteurs recombinants suivant les revendications 17 à 19, le plasmide pALP 231 obtenu à partir de pALP 23 (figure 14a) par coupure et déphosphorylation de pALP 23 avec les endonucléases de restriction BstE II et Stu I et clonage des oligonucléotides M1 et M2 dans le fragment pALP 23/BstE II x Stu I, le plasmide pALP 232 obtenu à partir de pALP 23 (figure 14a) par coupure et déphosphorylation de pALP 23 avec les endonucléases de restriction BstE II et Stu I et clonage des oligonucléotides M3 et M4 dans le fragment pALP 23/BstE II x Stu I, le plasmide pALP 236 obtenu à partir de pALP 23 (figure 14a) par coupure et déphosphorylation de pALP 23 avec les endonucléases de restriction BstE II et Stu I et clonage des oligonucléotides M5 et M6 en le fragment pALP 23/BstE II x Stu I, le plasmide pALP 237 obtenu à partir de pALP 231 par coupure et déphosphorylation de pALP 231 avec les endonucléases de restriction Sac I et Spe I et clonage des oligonucléotides E1, E2 et E3 dans le fragment pALP 231/Sac I x Spe I, le plasmide pALP 242 obtenu à partir de pALP 24 (figure 14b) par coupure et déphosphorylation de pALP 24 avec les endonucléases de restriction BstE II et Stu I et clonage des oligonucléotides M1 et M2 dans le fragment pALP 224/BstE II x Stu I et le plasmide pALP 246 obtenu à partir de pALP 24 (figure 14b) par coupure et déphosphorylation de pALP 24 avec les endonucléases de restriction BstE II et Stu I et clonage des oligonucléotides M5 et M6 dans le fragment pALP 24/BstE II x Stu I.

21. A titre de vecteurs recombinants suivant les revendications 17 à 20, les plasmides pALP 231, pALP 232 et pALP 242.

22. Vecteur recombinant suivant la revendication 17 ou 18, caractérisé en ce qu'il présente deux fois une séquence codante d'ADN selon l'une des revendications 15 et 16.

23. Vecteur recombinant suivant la revendication 22, caractérisé en ce que les deux séquences d'ADN codantes (selon l'une des revendications 15 et 16) portent chacune à l'extrémité terminale 5' la même séquence de Shine-Dalgarno à distance d'environ 7 nucléotides, les deux codons non-sens de la première séquence d'ADN codante étant suivis à distance d'environ 16 nucléotides de la seconde séquence de Shine-Dalgarno à laquelle se raccorde à distance d'environ 7 nucléotides la seconde séquence codante d'ADN.

24. A titre de vecteurs recombinants suivant les revendications 17 à 23, le plasmide pALP 2302 selon la figure 15 avec deux gènes de ALP 230 ayant la séquence indiquée sur la figure 2a au lieu de deux gènes de ALP 231, le plasmide pALP 2312 suivant la figure 15, obtenu à partir de pALP 23 (figure 14a), le plasmide pALP 2322 selon la figure 15 avec deux gènes de ALP 232 à la place de deux gènes de ALP 231, le plasmide pALP 2362 suivant la figure 15 avec deux gènes de ALP 236 à la place de deux gènes de ALP 231, le plasmide pALP 2372 suivant la figure 15 avec deux gènes de ALP 237 à la place de deux gènes de ALP 231, le plasmide pALP 2402 suivant la figure 15 avec deux gènes de ALP 240 ayant la séquence suivant la figure 2b à la place de deux gènes de ALP 231, le plasmide pALP 2422 suivant la figure 15 avec deux gènes de ALP 242 à la place de deux gènes de ALP 231 et le plasmide pALP 2462 suivant la figure 15 avec deux gènes de ALP 246 à la place de deux gènes de ALP 231.

25. A titre de vecteurs recombinants suivant les revendications 17 à 24, les plasmides pALP 2302, pALP 2312, pALP 2322, pALP 2402 et pALP 2422.

26. A titre de vecteur recombinant suivant les revendications 17 à 25, le plasmide pALP 2312 correspondant à la figure 15, obtenu à partir de pALP 23 (figure 14a).

27. Organisme-hôte caractérisé en ce qu'il est transformé avec l'un des vecteurs recombinants suivant les revendications 17 à 26.

28. Organisme hôte suivant la revendication 27, caractérisé en ce qu'il est une bactérie, de préférence une souche de l'espèce de E. coli ou de l'espèce B. subtilis, un champignon de taille microscopique, appartenant de préférence aux espèces Aspergillus oryzae, Aspergillus niger ou Saccharomyces cerevisiae, ou une cellule animale ou humaine.

29. Organisme hôte suivant les revendications 27 et 28, caractérisé en ce qu'il est une souche de l'espèce E. coli, notamment du sous-groupe K12.

30. Organisme hôte suivant les revendications 27 à 29, qui est choisi dans le groupe comprenant les souches suivantes : E. coli K12 JM 101 (ATCC 33876) ; E. coli K12 JM 103 (ATCC 39403) ; E. coli K12 JM 105 (DSM 4162) et E. coli K12 DH 1 (ATCC 33849).

31. Procédé de synthèse des séquences d'ADN suivant les revendications 15 et 16, caractérisé en ce qu'on construit conformément à la figure 14a à partir du plasmide ptacSDT (DSM 5018), d'une manière connue en utilisant les oligonucléotides D21 à D23a selon la figure 1b et en utilisant dans une autre étape les oligonucléotides D11 à D13a selon la figure 1a, le plasmide pALP 23 contenant la séquence d'ADN représentée sur la figure 2a, on utilise ce plasmide en utilisant aussi le cas échéant les oligonucléotides D24-D25a selon la figure 1c pour la construction du plasmide pALP 24 selon la figure 14b, contenant la séquence d'ADN représentée sur la figure 2b, on clone ensuite le cas échéant par paires dans les plasmides pALP 23 ou pALP 24 ainsi obtenus, des oligonucléotides choisis de manière que les plasmides résultants contiennent des séquences d'ADN codant pour des protéines de formule I dans lesquelles X₃ représente la méthionine ou la leucine et W, X₁, X₂, Y et Z ont la même définition que ci-dessus et on les modifie le cas échéant à l'aide d'autres oligonucléotides de manière qu'ils présentent des séquences d'ADN codant pour une protéine de formule I dans laquelle X₃ est la phénylalanine et W, X₁, X₂, Y et Z ont la même définition que ci-dessus.

32. Procédé suivant la revendication 31, caractérisé en ce qu'on clone dans le plasmide pALP 23 (obtenu conformément à la figure 14a) ou dans le plasmide pALP 24 (obtenu conformément à la figure 14b), par paires, les oligonucléotides M1 et M2, M3 et M4 ou M5 et M6 de formules
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
et
3' - GCTT CCA GTC ACG GAC GAC GAC TTG GGC - 5' (M6)
et on prépare ainsi des séquences d'ADN qui codent pour des composés de formule I dans lesquels X₃ représente la méthionine ou la leucine et W, X₁, X₂, Y et Z ont la même définition que ci-dessus.

33. Procédé suivant les revendications 31 et 32, caractérisé en ce que pour la synthèse d'une séquence d'ADN qui code pour une protéine inhibitrice de sérine-protéase de la formule I dans laquelle X₃ désigne la phénylalanine, on code dans une séquence d'ADN obtenue conformément à la revendication 32, des oligonucléotides qui induisent dans la position 96 de la formule I l'expression de phénylalanine.

34. Procédé suivant les revendications 31 à 33, caractérisé en ce que pour la production d'une séquence d'ADN qui code pour un composé de formule I dans laquelle X₁ et X₂ représentent la leucine et X₃ est la phénylalanine, et W, Y et Z ont la même définition que ci-dessus, on clone par paires dans le plasmide pALP 23 (obtenu conformément à la figure 14a) les oligonucléotides M1 et M2, M3 et M4 ou M5 et M6, on coupe le plasmide obtenu avec les endonucléases de restriction Sac I et Spe I, puis on les déphosphoryle de manière classique et on incorpore par ligation au fragment ainsi obtenu le produit d'hybridation des oligonucléotides (phosphorylés) E1, E2 et E3 des formules
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
et
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)

35. Procédé de synthèse de la séquence d'ADN représentée sur la figure 6, codant pour la protéine inhibitrice de sérine-protéase ALP 237, caractérisé en ce qu'on coupe avec les endonucléases de restriction Sac I et Spe I le plasmide pALP 231 obtenu par clonage des oligonucléotides M1 et M2 dans le plasmide pALP 23 (construit selon la figure 14a) puis on le déphosphoryle d'une manière classique et on soude par ligation dans le fragment pALP 231/Sac I x Spe I ainsi obtenu le produit d'hybridation des oligonucléotides (phosphorylés) E1, E2 et E3.

36. Procédé de production d'un vecteur recombinant pour l'expression d'un composé de formule I ou Ia, caractérisé en ce qu'on insère une séquence d'ADN obtenue selon les revendications 31-35 dans une molécule-vecteur de façon telle qu'elle soit en liaison fonctionnelle avec une séquence de contrôle d'expression, de préférence commençant par un tac-promotteur et se terminant par une séquence de terminateur.

37. Procédé de production d'un vecteur recombinant pour l'expression d'un composé de formule I ou Ia, caractérisé en ce qu'on coupe le vecteur d'expression obtenu selon la revendication 36 d'une manière connue en quantités partielles avec des paires différentes d'endonucléases de restriction, on déphosphoryle éventuellement l'un des fragments obtenus puis on soude par ligation les fragments qui mènent à un vecteur d'expression qui porte la séquence d'ADN codant pour le composé de formule I ou Ia derrière le tac-promoteur, deux fois séparées par deux codons anti-sens, une séquence contenant environ 16 nucléotides, une séquence de Shine-Dalgarno et une autre séquence ayant environ 7 nucléotides.

38. Procédé suivant la revendication 37, pour la production d'un vecteur recombinant pour l'expression d'un composé de formule I ou Ia, caractérisé en ce qu'on coupe une quantité partielle du vecteur d'expression obtenu selon la revendication 36 avec les endonucléases de restriction Pst I et Spe I et l'autre quantité partielle de ce vecteur avec les endonucléases de restriction Pst I et Xba I et on soude par ligation les fragments ainsi obtenus, qui contiennent la séquence d'ADN codant pour le composé de formule I ou Ia, éventuellement après déphosphorylation.

39. Procédé de production d'une protéine inhibitrice de sérine-protéase suivant les revendications 1 à 14, caractérisé en ce qu'on cultive un organisme hôte selon l'une des revendications 27 à 30 dans un milieu nutritif classique, on induit éventuellement l'expression de la protéine souhaitée puis on isole de la culture le produit d'expression, on le soumet à un processus de refolding par l'action d'agents réducteurs puis d'agents oxydants et on isole et purifie enfin d'une manière connue la protéine inhibitrice de sérineprotéase de formule I douée d'activité biologique ainsi obtenue.

40. Procédé de production d'une protéine inhibitrice de sérine-protéase suivant les revendications 1 à 7 et 14, comprenant des domaines partiels des séquences d'aminoacides suivant les formules I ou Ia, caractérisé en ce qu'on isole de la culture le produit d'expression formé dans le processus selon la revendication 39 puis on le soumet à une hydrolyse acide contrôlée pour couper la liaison acide aspartique-proline dans les positions 49-50 et/ou à une coupure enzymatique contrôlée de la totalité ou seulement d'1 à 5 des amino-acides des positions 107-102 et on sépare de l'hydrolysat le produit d'hydrolyse ainsi obtenu.

41. Procédé de production d'une protéine inhibitrice de sérine-protéase suivant les revendications 1 à 5, comprenant au moins les domaines partiels des positions 53-101 des séquences d'amino-acides selon les formules I ou Ia, dans lesquelles X₁ et X₂ représentent la leucine et X₃ est la leucine ou la phénylalanine, caractérisé en ce qu'on adapte le codon ATG par l'extrémité 5' terminale à la séquence d'ADN codant pour la protéine inhibitrice et on coupe ensuite d'une manière classique, de préférence avec le bromure de cyanogène, dans le produit formé lors de l'expression, portant de la méthionine comme amino-acide supplémentaire devant l'extrémité N-terminale de la protéine devant être produite, la liaison entre la méthionine et le premier amino-acide (N-terminal) de la protéine inhibitrice souhaitée.

42. Médicament caractérisé par une teneur en l'une des protéines inhibitrices de sérine-protéase suivant les revendications 1 à 14 et des supports et/ou des diluants et/ou des substances auxiliaires classiques.

43. Utilisation d'une protéine inhibitrice de sérine-protéase selon l'une des revendications 1 à 14 pour la préparation d'un médicament destiné au traitement curatif et/ou prophylactique de troubles de la coagulation, de croissance invasive de tumeurs, d'emphysème, d'arthrite, d'inflammations rénales et notamment d'inflammations excessives en cas de choc polytraumatique ou septique ou dans la pancréatite et d'autres états pathologiques qui créent une perturbation de l'équilibre entre des sérine-protéases et leurs inhibiteurs.

## Revendications (Revendications pour l'(les) Etat(s) contractant(s) suivant(s): ES)

1. Procédé de synthèse de séquences d'ADN qui code pour de nouvelles protéines inhibitrices de sérineprotéases de formule (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 ou leurs domaines partiels, qui comprennent au moins la séquence des positions 53-101,
où W représente la tyrosine ou l'acide glutamique, X₁ et X₂ sont identiques ou différents et chacun de ces restes désigne la méthionine ou la leucine, X₃ est la méthionine, la leucine ou la phénylalanine, Y est la proline ou l'arginine et Z est la leucine, ou désigne également l'isoleucine lorsque W est la tyrosine et X₁, X₂ et X₃ sont la leucine,
ou des protéines fusionnées de ces nouvelles protéines inhibitrices de sérine-protéase, caractérisé en ce qu'on construit conformément à la figure 14a à partir du plasmide ptacSDT (DSM 5018), d'une manière connue en utilisant les oligonucléotides D21 à D23a selon la figure 1b et en utilisant dans une autre étape les oligonucléotides D11 à D13a selon la figure 1a, le plasmide pALP 23 contenant la séquence d'ADN représentée sur la figure 2a, on utilise ce plasmide en utilisant aussi le cas échéant les oligonucléotides D24-D25a selon la figure 1c pour la construction du plasmide pALP 24 selon la figure 14b, contenant la séquence d'ADN représentée sur la figure 2b, on clone ensuite le cas échéant par paires dans les plasmides pALP 23 ou pALP 24 ainsi obtenus, des oligonucléotides choisis de manière que les plasmides résultants contiennent des séquences d'ADN codant pour des protéines de formule I dans lesquelles X₃ représente la méthionine ou la leucine et W, X₁, X₂, Y et Z ont la même définition que ci-dessus et on les modifie le cas échéant à l'aide d'autres oligonucléotides de manière qu'ils présentent des séquences d'ADN codant pour une protéine de formule I dans laquelle X₃ est la phénylalanine et W, X₁, X₂, Y et Z ont la même définition que ci-dessus.

2. Procédé suivant la revendication 1, caractérisé en ce qu'on clone dans le plasmide pALP 23 (obtenu conformément à la figure 14a) ou dans le plasmide pALP 24 (obtenu conformément à la figure 14b), par paires, les oligonucléotides M1 et M2, M3 et M4 ou M5 et M6 de formules
3' - GCC CAA GTC GTC GTC CGT GAC TGG CAT CCA TTG - 5' (M1)
3' - G ATG CCA GTC ACG GAC GAC GAC TTG GGC - 5' (M2) 3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
et
3' - GCTT CCA GTC ACG GAC GAC GAC TTG GGC - 5' (M6)
et on prépare ainsi des séquences d'ADN qui codent pour des composés de formule I dans lesquels X₃ représente la méthionine ou la leucine et W, X₁, X₂, Y et Z ont la même définition que ci-dessus.

3. Procédé suivant les revendications 1 et 2, caractérisé en ce que pour la synthèse d'une séquence d'ADN qui code pour la protéine inhibitrice de sérine-protéase de formule Ia : 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X'3--Cys-Gly-Lys-Ser-Cys-Val-Ser-Pro-Val-Lys-Ala
 ou des domaines partiels de cette séquence qui comprennent au moins la séquences des positions 53-101
où X₁, X₂, Y et Z ont la même définition que dans la revendication 1 et X'₃ représente la méthionine ou la leucine, on clone par paires les oligonucléotides M1 et M2 ou M3 et M4 dans le plasmide pALP 23 (obtenu conformément à la figure 14a) ou dans le plasmide pALP 24 (obtenu conformément à la figure 14b).

4. Procédé suivant la revendication 1, caractérisé en ce que pour la production d'une séquence d'ADN qui code pour un composé de formule I dans laquelle X₃ désigne la phénylalanine, on code dans une séquence d'ADN obtenue conformément à la revendication 2 ou 3 des oligonucléotides qui induisent dans la position 96 de la formule I l'expression de phénylalanine.

5. Procédé suivant les revendications 1, 2 et 4, caractérisé en ce que pour la production d'une séquence d'ADN qui code pour un composé de formule I dans laquelle X₁ et X₂ représentent la leucine et X₃ est la phénylalanine, et W, Y et Z ont la même définition que ci-dessus, on clone par paires dans le plasmide pALP 23 (obtenu conformément à la figure 14a) les oligonucléotides M1 et M2, M3 et M4 ou M5 et M6, on coupe le plasmide obtenu avec les endonucléases de restriction Sac I et Spe I, puis on les déphosphoryle de manière classique et on soude par ligation au fragment ainsi obtenu le produit d'hybridation des oligonucléotides (phosphorylés) E1, E2 et E3 des formules
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
et
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)

6. Procédé de synthèse de la séquence d'ADN représentée sur la figure 6, codant pour la protéine inhibitrice de sérine-protéase ALP 237, caractérisé en ce qu'on coupe avec les endonucléases de restriction Sac I et Spe I le plasmide pALP 231 obtenu par clonage des oligonucléotides M1 et M2 dans le plasmide pALP 23 (construit selon la figure 14a) puis on le déphosphoryle d'une manière classique et on soude par ligation dans le fragment pALP 231/Sac I x Spe I ainsi obtenu le produit d'hybridation des oligonucléotides (phosphorylés) E1, E2 et E3.

7. Procédé de production d'un vecteur recombinant pour l'expression d'un composé de formule I ou Ia, caractérisé en ce qu'on insère une séquence d'ADN obtenue selon les revendications 1-6 dans une molécule-vecteur de façon telle qu'elle soit en liaison fonctionnelle avec une séquence de contrôle d'expression, de préférence commençant par un tac-promotteur et se terminant par une séquence de terminateur.

8. Procédé de production d'un vecteur recombinant pour l'expression d'un composé de formule I ou Ia, caractérisé en ce qu'on coupe le vecteur d'expression obtenu selon la revendication 7 d'une manière connue en quantités partielles avec des paires différentes d'endonucléases de restriction, on déphosphoryle éventuellement l'un des fragments obtenus puis on soude par ligation les fragments qui mènent à un vecteur d'expression qui porte la séquence d'ADN codant pour le composé de formule I ou Ia derrière le tac-promoteur, deux fois séparées par deux codons anti-sens, une séquence contenant environ 16 nucléotides, une séquence de Shine-Dalgarno et une autre séquence ayant environ 7 nucléotides.

9. Procédé suivant la revendication 8, pour la production d'un vecteur recombinant pour l'expression d'un composé de formule I ou Ia, caractérisé en ce qu'on coupe une quantité partielle du vecteur d'expression obtenu selon la revendication 7 avec les endonucléases de restriction Pst I et Spe I et l'autre quantité partielle de ce vecteur avec les endonucléases de restriction Pst I et Xba I et on soude les fragments ainsi obtenus, qui contiennent la séquence d'ADN codant pour le composé de formule I ou Ia, éventuellement après déphosphorylation.

10. Procédé de production d'une protéine inhibitrice de sérine-protéase de formule I ou d'une protéine fusionnée de cette protéine inhibitrice, caractérisé en ce qu'on transforme un organisme hôte avec un vecteur recombinant suivant les revendications 7 à 9 puis on le cultive dans un milieu nutritif classique, on induit éventuellement l'expression de la protéine souhaitée puis on isole de la culture le produit d'expression formé, on le soumet à un processus de refolding par l'action d'agents réducteurs puis d'agents oxydants et on isole et purifie enfin d'une manière connue la protéine inhibitrice de sérine-protéase biologiquement active obtenue de formule I.

11. Procédé suivant la revendication 10, caractérisé en ce que l'organisme hôte est une bactérie, de préférence une souche de l'espèce E. coli ou de l'espèce B. subtilis, un champignon microscopique, appartenant de préférence aux espèces Aspergillus oryzae, Aspergillus niger ou Saccharomyces cerevisiae ou une cellule animale ou humaine.

12. Procédé suivant les revendications 10 et 11, caractérisé en ce que l'organisme hôte est une souche de l'espèce E. coli, notamment du sous-groupe K12.

13. Procédé suivant les revendications 10 à 12, caractérisé en ce que l'organisme hôte est choisi dans le groupe comprenant les souches suivantes : E. coli K12 JM 101 (ATCC 33876) ; E. coli K12 JM 103 (ATCC 39403) ; E. coli K12 JM 105 (DSM 4162) et E. coli K12 DH1 (ATCC 33849).

14. Procédé suivant la revendication 10 pour la production d'une protéine inhibitrice de sérine-protéase comprenant des domaines partiels des séquences d'amino-acides suivant les formules I ou Ia, caractérisé en ce qu'on isole de la culture le produit d'expression formé dans le processus selon la revendication 10 et on le soumet ensuite à une hydrolyse acide contrôlée pour la coupure de la liaison acide aspartique-proline dans les positions 49-50 et/ou à une élimination enzymatique contrôlée de la totalité ou de seulement 1 à 5 des amino-acides des positions 107-102 et on isole de l'hydrolysat le produit d'hydrolyse ainsi obtenu.

15. Procédé suivant la revendication 14, caractérisé en ce qu'on sépare de la protéine inhibitrice de sérine-protéase ALP 231 suivant la figure 3, par hydrolyse acide, de préférence avec de l'acide formique à 70 %, les amino-acides des positions 1-49 et on obtient ainsi la nouvelle protéine inhibitrice de sérine-protéase ayant la séquence d'amino-acides

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

16. Procédé de production d'une protéine inhibitrice de sérine-protéase comprenant au moins les domaines partiels des positions 53-101 des séquences d'amino-acides suivant les formules I ou Ia, dans lesquelles X₁ et X₂ représentent la leucine et X₃ est la leucine ou la phénylalanine, caractérisé en ce qu'on adapte le codon ATG par l'extrémité 5' terminale à la séquence d'ADN codant pour la protéine inhibitrice et on coupe ensuite d'une manière classique, de préférence avec le bromure de cyanogène, dans le produit formé lors de l'expression, portant de la méthionine comme amino-acide supplémentaire devant l'extrémité N-terminale de la protéine devant être produite, la liaison entre la méthionine et le premier amino-acide (N-terminal) de la protéine inhibitrice souhaitée.

17. Procédé de préparation d'un médicament qui contient comme substance active une protéine inhibitrice de sérine-protéase de formule I ou Ia ou d'un domaine partiel comprenant au moins la séquence des positions 53-101, caractérisé en ce que la substance active est mise sous une forme médicamenteuse utilisable par voie parentérale ou localement, d'une manière connue avec des supports et/ou des diluants et/ou des substances auxiliaires classiques.

## Revendications (Revendications pour l'(les) Etat(s) contractant(s) suivant(s): GR)

1. Protéines de formule (I) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr--W--Gly-Gln-Cys--Z--
90
Leu-Leu-Asn--Y--Pro-Asn-Phe-Cys-Glu--X1-Asp-Gly-Gln-Cys-Lys-Arg-Asp-Leu-
107
Lys-Cys-Cys--X2--Gly--X3--Cys-Gly-Lys-Ser-Cys-Val-Ser-Pro-Val-Lys-Ala
 ou domaines partiels de ces protéines, qui comprennent au moins la séquence des positions 53-101,
où W représente la tyrosine ou l'acide glutamique, X₁ et X₂ sont identiques et différents et chacun de ces restes désigne la méthionine ou la leucine, X₃ est la méthionine, la leucine ou la phénylalanine, Y est la proline ou l'arginine et Z est la leucine, ou désigne également l'isoleucine lorsque W est la tyrosine et X₁, X₂ et X₃ sont la leucine,
ou protéines fusionnées de ces nouvelles protéines.

2. Protéines de formule I ou domaine partiel de ces protéines comprenant au moins la séquence des positions 53-101, où W représente la tyrosine.

3. Protéines suivant les revendications 1 et 2, de formule (Ia) 
18
Ser-Gly-Lys-Ser-Phe-Lys-Ala-Gly-Val-Cys-Pro-Pro-Lys-Lys-Ser-Ala-Gln-Cys-
36
Leu-Arg-Tyr-Lys-Lys-Pro-Glu-Cys-Gln-Ser-Asp-Trp-Gln-Cys-Pro-Gly-Lys-Lys-
 
54
Arg-Cys-Cys-Pro-Asp-Thr-Cys-Gly-Ile-Lys-Cys-Leu-Asp-Pro-Val-Asp-Thr-Pro-
72
Asn-Pro-Thr-Arg-Arg-Lys-Pro-Gly-Lys-Cys-Pro-Val-Thr-Tyr-Gly-Gln-Cys--Z--
90
Leu-Leu-Asn-Pro--Y--Asn-Phe-Cys-Glu--X1-Asp-Gly-Gln-Cys-Lys-Arg-Asp-Leu-
107
Lys-Cys-Cys--X2-Gly--X3--Cys-Gly-Lys-Ser-Cys-Val-Ser-Pro-Val-Lys-Ala
 ou domaines partiels de ces protéines, qui comprennent au moins la séquence des positions 53-101,
où X₁, X₂, Y et Z ont la même définition que dans la revendication 1 et X_{'3} est la méthionine ou la leucine,
et protéines fusionnées de ces nouvelles protéines.

4. Protéines suivant les revendications 1 à 3, qui représentent les domaines partiels des formules I et Ia et qui comprennent les domaines de séquence des positions 53-101 jusqu'à ceux des positions 48-107.

5. Protéines suivant les revendications 1 à 3, qui comprennent les positions d'amino-acides 1 à 107 ou les séquences partielles 48-107, 49-107 ou 50-107 des formules I ou Ia.

6. Protéines suivant les revendications 1, 4 et 5, de formule 
50
Pro-Val-Asp-Thr-Pro-Asn-Pro-Thr-Arg-Arg-Lys-Pro-Gly-Lys-Cys-Pro-Val-
Thr-W-Gly-Gln-Cys-Z-Leu-Leu-Asn-Y-Pro-Asn-Phe-Cys-Glu-X1-Asp-Gly-
Gln-Cys-Lys-Arg-Asp-Leu-Lys-Cys-Cys-X2-Gly-X3-Cys-Gly-Lys-Ser-Cys-
Val-Ser-Pro-Val-Lys-Ala
107
 où W, X₁, X₂, X₃, Y et Z ont la même définition que dans la revendication 1.

7. Protéines suivant les revendications 1 à 6, dans lesquelles X₁, X₂, X₃ et Z représentent la leucine.

8. Protéine ayant la séquence d'amino-acides suivant la figure 3.

9. Protéine ayant la séquence d'amino-acides suivant la figure 4.

10. Protéine ayant la séquence d'amino-acides suivant la figure 5.

11. Protéine ayant la séquence d'amino-acides suivant la figure 6.

12. Protéine ayant la séquence d'amino-acides suivant la figure 7.

13. Protéine ayant la séquence d'amino-acides suivant la figure 8.

14. Protéine ayant la séquence d'amino-acides

50
Pro-Val-Asp-Thr-Pro-Asn-Pro-Thr-Arg-Arg-Lys-Pro-Gly-Lys-Cys-Pro-Val-
Thr-Tyr-Gly-Gln-Cys-Leu-Leu-Leu-Asn-Pro-Pro-Asn-Phe-Cys-Glu-Leu-Asp-
Gly-Gln-Cys-Lys-Arg-Asp-Leu-Lys-Cys-Cys-Leu-Gly-Leu-Cys-Gly-Lys-Ser-
Cys-Val-Ser-Pro-Val-Lys-Ala
107

15. Séquences d'ADN, caractérisées en ce qu'elles codent pour des protéines suivant les revendications 1 à 14.

16. Séquence d'ADN suivant la revendication 15, caractérisée en ce qu'elle présente l'une des séquences de nucléotides représentées sur les figures 2a, 2b ou 3 à 8.

17. Vecteur recombinant, caractérisé en ce qu'il présente une séquence d'ADN codante suivant l'une des revendications 15 ou 16.

18. Vecteur recombinant suivant la revendication 17, caractérisé en ce qu'il est un dérivé du plasmide ptacSDT (DMS 5018).

19. A titre de vecteurs recombinants suivant les revendications 17 et 18, le plasmide pALP 23 obtenu conformément à la figure 14a et le plasmide pALP 24 obtenu conformément à la figure 14b.

20. A titre de vecteurs recombinants suivant les revendications 17 à 19, le plasmide pALP 231 obtenu à partir de pALP 23 (figure 14a) par coupure et déphosphorylation de pALP 23 avec les endonucléases de restriction BstE II et Stu I et clonage des oligonucléotides M1 et M2 en le fragment pALP 23/BstE II x Stu I, le plasmide pALP 232 obtenu à partir de pALP 23 (figure 14a) par coupure et déphosphorylation de pALP 23 avec les endonucléases de restriction BstE II et Stu I et clonage des oligonucléotides M3 et M4 dans le fragment pALP 23/BstE II x Stu I, le plasmide pALM 236 obtenu à partir de pALP 23 (figure 14a) par coupure et déphosphorylation de pALP 23 avec les endonucléases de restriction BstE II et Stu I et clonage des oligonucléotides M5 et M6 dans le fragment pALP 23/BstE II x Stu I, le plasmide pALP 237 obtenu à partir de pALP 231 par coupure et déphosphorylation de pALP 231 avec les endonucléases de restriction Sac I et Spe I et clonage des oligonucléotides E1, E2 et E3 dans le fragment pALP 231/Sac I x Spe I, le plasmide pALP 242 obtenu à partir de pALP 24 (figure 14b) par coupure et déphosphorylation de pALP 24 avec les endonucléases de restriction BstE II et Stu I et clonage des oligonucléotides M1 et M2 dans le fragment pALP 224/BstE II x Stu I et le plasmide pALP 246 obtenu à partir de pALP 24 (figure 14b) par coupure et déphosphorylation de pALP 24 avec les endonucléases de restriction BstE II et Stu I et clonage des oligonucléotides M5 et M6 dans le fragment pALP 24/BstE II x Stu I.

21. A titre de vecteurs recombinants suivant les revendications 17 à 20, les plasmides pALP 231, pALP 232 et pALP 242.

22. Vecteur recombinant suivant la revendication 17 ou 18, caractérisé en ce qu'il présente deux fois une séquence codante d'ADN selon l'une des revendications 15 et 16.

23. Vecteur recombinant suivant la revendication 22, caractérisé en ce que les deux séquences d'ADN codantes (selon l'une des revendications 15 et 16) portent chacune à l'extrémité terminale 5' la même séquence de Shine-Dalgarno à distance d'environ 7 nucléotides, les deux codons non-sens de la première séquence d'ADN codante étant suivis à distance d'environ 16 nucléotides de la seconde séquence de Shine-Dalgarno à laquelle se raccorde à distance d'environ 7 nucléotides la seconde séquence codante d'ADN.

24. A titre de vecteurs recombinants suivant les revendications 17 à 23, le plasmide pALP 2302 selon la figure 15 avec deux gènes de ALP 230 ayant la séquence indiquée sur la figure 2a au lieu de deux gènes de ALP 231, le plasmide pALP 2312 suivant la figure 15, obtenu à partir de pALP 23 (figure 14a), le plasmide pALP 2322 selon la figure 15 avec deux gènes de ALP 232 à la place de deux gènes de ALP 231, le plasmide pALP 2362 suivant la figure 15 avec deux gènes de ALP 236 à la place de deux gènes de ALP 231, le plasmide pALP 2372 suivant la figure 15 avec deux gènes de ALP 237 à la place de deux gènes de ALP 231, le plasmide pALP 2402 suivant la figure 15 avec deux gènes de ALP 240 ayant la séquence suivant la figure 2b à la place de deux gènes de ALP 231, le plasmide pALP 2422 suivant la figure 15 avec deux gènes de ALP 242 à la place de deux gènes de ALP 231 et le plasmide pALP 2462 suivant la figure 15 avec deux gènes de ALP 246 à la place de deux gènes de ALP 231.

25. A titre de vecteurs recombinants suivant les revendications 17 à 24, les plasmides pALP 2302, pALP 2312, pALP 2322, pALP 2402 et pALP 2422.

26. A titre de vecteur recombinant suivant les revendications 17 à 25, le plasmide pALP 2312 correspondant à la figure 15, obtenu à partir de pALP 23 (figure 14a).

27. Organisme hôte caractérisé en ce qu'il est transformé avec l'un des vecteurs recombinants suivant les revendications 17 à 26.

28. Organisme hôte suivant la revendication 27, caractérisé en ce qu'il est une bactérie, de préférence une souche de l'espèce de E. coli ou de l'espèce B. subtilis, un champignon de taille microscopique, appartenant de préférence aux espèces Aspergillus oryzae, Aspergillus niger ou Saccharomyces cerevisiae, ou une cellule animale ou humaine.

29. Organisme hôte suivant les revendications 27 et 28, caractérisé en ce qu'il est une souche de l'espèce E. coli, notamment du sous-groupe K12.

30. Organisme hôte suivant les revendications 27 à 29, qui est choisi dans le groupe comprenant les souches suivantes : E. coli K12 JM 101 (ATCC 33876) ; E. coli K12 JM 103 (ATCC 39403) ; E. coli K12 JM 105 (DSM 4162) et E. coli K12 DH 1 (ATCC 33849).

31. Procédé de synthèse des séquences d'ADN suivant les revendications 15 et 16, caractérisé en ce qu'on construit conformément à la figure 14a à partir du plasmide ptacSDT (DSM 5018), d'une manière connue en utilisant les oligonucléotides D21 à D23a selon la figure 1b et en utilisant dans une autre étape les oligonucléotides D11 à D13a selon la figure 1a, le plasmide pALP 23 contenant la séquence d'ADN représentée sur la figure 2a, on utilise ce plasmide en utilisant aussi le cas échéant les oligonucléotides D24-D25a selon la figure 1c pour la construction du plasmide pALP 24 selon la figure 14b, contenant la séquence d'ADN représentée sur la figure 2b, on clone ensuite le cas échéant par paires dans les plasmides pALP 23 ou pALP 24 ainsi obtenus des oligonucléotides choisis de manière que les plasmides résultants contiennent des séquences d'ADN codant pour des protéines de formule I dans lesquelles X₃ représente la méthionine ou la leucine et W, X₁, X₂, Y et Z ont la même définition que ci-dessus et on les modifie le cas échéant à l'aide d'autres oligonucléotides de manière qu'ils présentent des séquences d'ADN codant pour une protéine de formule I dans laquelle X₃ est la phénylalanine et W, X₁, X₂, Y et Z ont la même définition que ci-dessus.

32. Procédé suivant la revendication 31, caractérisé en ce qu'on clone dans le plasmide pALP 23 (obtenu conformément à la figure 14a) ou dans le plasmide pALP 24 (obtenu conformément à la figure 14b), par paires, les oligonucléotides M1 et M2, M3 et M4 ou M5 et M6 de formules
3' - GCC CAA GTC GTC GTC CGT GAC TGG CAT CCA TTG - 5' (M1)
3' - G ATG CCA GTC ACG GAC GAC GAC TTG GGC - 5' (M2)
3' - GCC CAA GTC GTC CTA CGT GAC TGG CAT CCA TTG - 5' (M3)
3' - G ATG CCA GTC ACG TAG GAC GAC TTG GGC - 5' (M4)
3' - GCC CAA GTC GTC GTC CGT GAC TGG AAG CCA TTG - 5' (M5)
et
3' - GCTT CCA GTC ACG GAC GAC GAC TTG GGC - 5' (M6)
et on prépare ainsi des séquences d'ADN qui codent pour des composés de formule I dans lesquels X₃ représente la méthionine ou la leucine et W, X₁, X₂, Y et Z ont la même définition que ci-dessus.

33. Procédé suivant les revendications 31 et 32, caractérisé en ce que pour la synthèse d'une séquence d'ADN qui code pour une protéine inhibitrice de sérine-protéase de la formule I dans laquelle X₃ désigne la phénylalanine, on code dans une séquence d'ADN obtenue conformément à la revendication 32, des oligonucléotides qui induisent dans la position 96 de la formule I l'expression de phénylalanine.

34. Procédé suivant les revendications 31 à 33, caractérisé en ce que pour la production d'une séquence d'ADN qui code pour un composé de formule I dans laquelle X₁ et X₂ représentent la leucine et X₃ est la phénylalanine, et W, Y et Z ont la même définition que ci-dessus, on clone par paires dans le plasmide pALP 23 (obtenu conformément à la figure 14a) les oligonucléotides M1 et M2, M3 et M4 ou M5 et M6, on coupe le plasmide obtenu avec les endonucléases de restriction Sac I et Spe I, puis on les déphosphoryle de manière classique et on incorpore par ligation au fragment ainsi obtenu le produit d'hybridation des oligonucléotides (phosphorylés) E1, E2 et E3 des formules
3' - A GAA CGC AAT CGG GCC AAT TCC GAA CTA TCC TAG TTG ATC - 5' (E1)
3' - TC GAG CTA CCG GTC ACG TTT GCT CTA GAC TTT ACG ACG GAC CCA AAG ACG CCA TTT - 5' (E2)
et
3' - A ACT AGG ATA GTT CGG AAT TGG CCC GAT TGC GTT CTA AAT GGC GTC TTT GGG TCC GTC GTA AAG TCT AGA GCA AAC GTG ACC GGT AGC - 5' (E3)

35. Procédé de synthèse de la séquence d'ADN représentée sur la figure 6, codant pour la protéine inhibitrice de sérine-protéase ALP 237, caractérisé en ce qu'on coupe avec les endonucléases de restriction Sac I et Spe I le plasmide pALP 231 obtenu par clonage des oligonucléotides M1 et M2 dans le plasmide pALP 23 (construit selon la figure 14a) puis on le déphosphoryle d'une manière classique et on soude par ligation dans le fragment pALP 231/Sac I x Spe I ainsi obtenu le produit d'hybridation des oligonucléotides (phosphorylés) E1, E2 et E3.

36. Procédé de production d'un vecteur recombinant pour l'expression d'un composé de formule I ou Ia, caractérisé en ce qu'on insère une séquence d'ADN obtenue selon les revendications 31-35 dans une molécule-vecteur de façon telle qu'elle soit en liaison fonctionnelle avec une séquence de contrôle d'expression, de préférence commençant par un tac-promotteur et se terminant par une séquence de terminateur.

37. Procédé de production d'un vecteur recombinant pour l'expression d'un composé de formule I ou Ia, caractérisé en ce qu'on coupe le vecteur d'expression obtenu selon la revendication 36 d'une manière connue en quantités partielles avec des paires différentes d'endonucléases de restriction, on déphosphoryle éventuellement l'un des fragments obtenus puis on soude par ligation les fragments qui mènent à un vecteur d'expression qui porte la séquence d'ADN codant pour le composé de formule I ou Ia derrière le tac-promoteur, deux fois séparées par deux codons anti-sens, une séquence contenant environ 16 nucléotides, une séquence de Shine-Dalgarno et une autre séquence ayant environ 7 nucléotides.

38. Procédé suivant la revendication 37, pour la production d'un vecteur recombinant pour l'expression d'un composé de formule I ou Ia, caractérisé en ce qu'on coupe une quantité partielle du vecteur d'expression obtenu selon la revendication 36 avec les endonucléases de restriction Pst I et Spe I et l'autre quantité partielle de ce vecteur avec les endonucléases de restriction Pst I et Xba I et on soude par ligation les fragments ainsi obtenus, qui contiennent la séquence d'ADN codant pour le composé de formule I ou Ia, éventuellement après déphosphorylation.

39. Procédé de production d'une protéine inhibitrice de sérine-protéase suivant les revendications 1 à 14, caractérisé en ce qu'on cultive un organisme hôte selon l'une des revendications 27 à 30 dans un milieu nutritif classique, on induit éventuellement l'expression de la protéine souhaitée puis on isole de la culture le produit d'expression, on le soumet à un processus de refolding par l'action d'agents réducteurs puis d'agents oxydants et on isole et purifie enfin d'une manière connue la protéine inhibitrice de sérineprotéase de formule I douée d'activité biologique ainsi obtenue.

40. Procédé de production d'une protéine inhibitrice de sérine-protéase suivant les revendications 1 à 7 et 14, comprenant des domaines partiels des séquences d'aminoacides suivant les formules I ou Ia, caractérisé en ce qu'on isole de la culture le produit d'expression formé dans le processus selon la revendication 39 puis on le soumet à une hydrolyse acide contrôlée pour couper la liaison acide aspartique-proline dans les positions 49-50 et/ou à une coupure enzymatique contrôlée de la totalité ou seulement d'1 à 5 des amino-acides des positions 107-102 et on sépare de l'hydrolysat le produit d'hydrolyse ainsi obtenu.

41. Procédé de production d'une protéine inhibitrice de sérine-protéase suivant les revendications 1 à 5, comprenant au moins les domaines partiels des positions 53-101 des séquences d'amino-acides selon les formules I ou Ia, dans lesquelles X₁ et X₂ représentent la leucine et X₃ est la leucine ou la phénylalanine, caractérisé en ce qu'on adapte le codon ATG par l'extrémité 5' terminale à la séquence d'ADN codant pour la protéine inhibitrice et on coupe ensuite d'une manière classique, de préférence avec le bromure de cyanogène, dans le produit formé lors de l'expression, portant de la méthionine comme amino-acide supplémentaire devant l'extrémité N-terminale de la protéine devant être produite, la liaison entre la méthionine et le premier amino-acide (N-terminal) de la protéine inhibitrice souhaitée.

42. Procédé de préparation d'un médicament qui contient comme substance active une protéine des formules I ou Ia ou un domaine partiel de cette protéine comprenant au moins la séquence des positions 53-101, caractérisé en ce qu'on fait prendre à la substance active une forme médicamenteuse utilisable par voie parentérale ou localement, d'une manière connue avec des supports et/ou des diluants et/ou des substances auxiliaires classiques.
